(19) **Europäisches Patentamt / European Patent Office / Office européen des brevets**

(11) **EP 4 738 372 A1**

(12) **EUROPEAN PATENT APPLICATION**
published in accordance with Art. 153(4) EPC

(43) Date of publication:
06.05.2026  Bulletin 2026/19

(21) Application number: 24895855.5

(22) Date of filing: 04.09.2024

(51) International Patent Classification (IPC):
$G16C\ 60/00^{(2019.01)}$    $G16C\ 20/70^{(2019.01)}$

(52) Cooperative Patent Classification (CPC):
G16C 20/70; G16C 60/00

(86) International application number:
PCT/CN2024/116845

(87) International publication number:
WO 2025/112756 (05.06.2025 Gazette 2025/23)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority:  27.11.2023  CN 202311608044

(71) Applicants:
• Contemporary Amperex Technology Co., Limited
Ningde, Fujian 352100 (CN)
• Shanghai Jiao Tong University
Shanghai 200240 (CN)

(72) Inventors:
• JIA, Jun
Ningde, Fujian 352100 (CN)
• WANG, Qing
Ningde, Fujian 352100 (CN)
• WANG, Xiang
Ningde, Fujian 352100 (CN)
• LIN, Zhiquan
Ningde, Fujian 352100 (CN)
• FENG, Kai
Ningde, Fujian 352100 (CN)
• DU, Shangzhe
Ningde, Fujian 352100 (CN)

(74) Representative: Ziebig Hengelhaupt Intellectual Property Attorneys
Patentanwaltskanzlei PartGmbB
Leipziger Straße 49
10117 Berlin (DE)

(54) **CONSTRUCTION METHOD FOR CORROSION-RESISTANCE DEGREE-MECHANICAL PROPERTY ANALYSIS MODEL OF METAL MATERIAL, AND USE THEREOF**

(57)    This application relates to a method for constructing a corrosion resistance-mechanical property analysis model for a metallic material and an application thereof. The method for constructing the analysis model includes: using a metallic material as a test object, acquiring a corrosion performance test experimental dataset at different equivalent corrosion time points under a corrosion test condition, and a mechanical property test experimental dataset at different corrosion degrees corresponding to the different equivalent corrosion time points; and establishing empirical relational expressions describing variations of a corrosion parameter and a mechanical parameter with equivalent corrosion time.

FIG. 1

**Description**

**RELATED APPLICATIONS**

This application claims priority to Chinese Patent Application No. CN2023116080448, filed on November 27, 2023 and entitled "METHOD FOR CONSTRUCTING CORROSION RESISTANCE-MECHANICAL PROPERTY ANALYSIS MODEL FOR METALLIC MATERIAL AND APPLICATION THEREOF", which is incorporated herein by reference in its entirety.

**TECHNICAL FIELD**

This application relates to the technical field of corrosion resistance testing and analysis of metallic materials and the technical field of battery technology, further relates to a method for constructing a corrosion resistance-mechanical property analysis model for a metallic material and an application thereof, and still further, relates to a method for constructing a corrosion resistance-mechanical property analysis model for metallic materials, a method and an apparatus for analyzing a service life of a metallic material, a method and an apparatus for analyzing a mechanical property of a metallic material, a computer device, a computer-readable storage medium, and an electric apparatus.

**BACKGROUND**

The statements herein merely provide background information related to this application and do not necessarily constitute prior art.

The design and development of battery casing materials require high structural strength and corrosion resistance, which are critical to the safe operation of batteries and electric apparatuses as a whole. Taking fuel cell casing materials as an example, current research on the corrosion behavior and mechanisms of fuel cell casing materials usually focuses only on the electrochemical corrosion behavior of fuel cell casing materials or corrosion rates in different corrosive media, making it difficult to provide effective guidance for the design and development of fuel cell casing materials.

**SUMMARY**

According to various implementations and embodiments of this application, this application provides a method for constructing a corrosion resistance-mechanical property analysis model for a metallic material, a method and an apparatus for analyzing service life of a metallic material, a method and an apparatus for analyzing a mechanical property of a metallic material, a computer device, a computer-readable storage medium, and an electric apparatus. The corrosion resistance-mechanical property analysis model for a metallic material can be effectively used for predicting mechanical properties and evaluating service life of the metallic material. The metallic material involved may include, but is not limited to, an aluminum alloy material, and may also include, but is not limited to, a battery casing material.

According to a first aspect, this application provides a method for constructing a corrosion resistance-mechanical property analysis model for a metallic material.

In some embodiments, the method for constructing the analysis model includes the following steps: using a metallic material as a test object, acquiring a corrosion performance test experimental dataset at different equivalent corrosion time points under a corrosion test condition, and a mechanical property test experimental dataset at different corrosion degrees corresponding to the different equivalent corrosion time points; and establishing empirical relational expressions describing variations of a corrosion parameter and a mechanical parameter with equivalent corrosion time. Further, the corrosion test condition may be used to simulate a target service environment.

In some embodiments, a method for constructing a corrosion resistance-mechanical property analysis model for a metallic material is provided, including the following steps:

using a metallic material as a test object, acquiring test values of a corrosion parameter at different equivalent corrosion time points under a corrosion test condition to obtain a corrosion performance test experimental dataset, and acquiring test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate; and

establishing, based on the corrosion performance test experimental dataset, a first set of empirical relational expressions describing variation of the corrosion parameter with equivalent corrosion time, and establishing, based on the mechanical property test experimental dataset, a second set of empirical relational expressions describing variation of the mechanical parameter with equivalent corrosion time.

Using the metallic material as the test object, the target service environment of the metallic material is simulated using the corrosion test condition, to obtain the corrosion performance test experimental dataset at different equivalent corrosion time points and the mechanical property test experimental dataset at different corrosion degrees corresponding to the different equivalent corrosion time points; and based on the equivalent corrosion time under the corrosion test condition and the mechanical properties at different corrosion degrees, the first set of empirical relational expressions describing variation of at least one corrosion parameter of the corrosion degree and the corrosion rate with the equivalent corrosion time are established, and the second set of empirical relational expressions describing variation of at least one mechanical parameter with the equivalent corrosion time are established, thereby constructing the model for analyzing the corrosion resistance degrees and mechanical properties of the metallic material. This model can help understand the corrosion mechanism of the metallic material and can be used to predict and analyze the mechanical properties of the metallic material under a target service condition based on the corrosion behavior of the metallic material, thereby evaluating the service life of metallic material structural components in the target service environment.

Based on any suitable embodiment of this application, further, in some embodiments, the mechanical parameter includes at least one of a tensile strength at break and an elongation at break.

When the mechanical parameter in the method includes at least one of the tensile strength at break and the elongation at break, the constructed corrosion resistance-mechanical property analysis model for the metallic material can be applied to metallic material structural components that are prone to failure due to tensile stress.

Based on any suitable embodiment of this application, further, in some embodiments, the mechanical parameter includes a yield strength.

Appropriate parameters are selected for fitting functional relational expressions, which is conducive to obtaining the second set of empirical relational expressions with better fitting results, thereby helping to obtain a more effective corrosion resistance-mechanical property analysis model.

Based on any suitable embodiment of this application, further, in some embodiments, the method for constructing the analysis model meets at least one of the following characteristics:

the metallic material is an aluminum alloy material; or
the metallic material is a metallic structural component, optionally an aluminum alloy structural component.

Based on any suitable embodiment of this application, further, in some embodiments, the method for constructing the analysis model meets at least one of the following characteristics:

the metallic material is any one of the battery casing material; optionally, the battery casing material includes a fuel cell casing material; optionally, the battery casing material includes a lithium battery casing material; or
the metallic material includes at least a portion of a structural component of a battery casing; optionally, the battery casing structural component includes at least a portion of a structural component of a fuel cell casing; optionally, the battery casing structural component includes at least a portion of a structural component of a lithium battery casing.

The metallic material may be an aluminum alloy material or a metallic structural component, and further may be an aluminum alloy structural component. Due to the advantages such as low density, high specific strength, excellent thermal stability, good machinability, and low cost, die-cast aluminum alloys can be used to prepare various automotive components, including, but not limited to, engine blocks, generator housings, fuel cell casings, and various engine brackets. The aluminum alloy material can be applied in the field of battery technology and can be used as a main body material (including a constituent material) for battery casings or aluminum alloy structural components in battery casings. Therefore, the metallic material may be a battery casing material, and the battery casing may include, but is not limited to, a fuel cell casing or a lithium battery casing; and correspondingly, the metallic material may include at least a portion of a structural component of a battery casing. When the corrosion parameter and the mechanical parameter are obtained based on the aluminum alloy material, the constructed corrosion resistance-mechanical property analysis model for the metallic material can be applied to mechanical property prediction and service life evaluation of the aluminum alloy material, and also facilitates understanding of the white rust corrosion mechanism of the aluminum alloy material. When the aluminum alloy material is used as the main body material or constituent material for battery casings or structural components in battery casings, the corrosion resistance-mechanical property analysis model for the metallic material can be applied to mechanical property prediction and service life evaluation of battery casings or structural components in battery casings.

Taking a fuel cell casing as an example, the model also facilitates understanding of the white rust corrosion mechanism of fuel cell casing materials. Using fuel cell casing materials as the test objects, corrosion behaviors of the fuel cell casing materials can be analyzed, corrosion rates of different types of fuel cell casing materials can be determined, and relational expressions between the corrosion degrees and mechanical properties of fuel cell casing materials can be established, providing reference for the safe operation of fuel cells and electric apparatuses including fuel cells.

Based on any suitable embodiment of this application, further, in some embodiments, the corrosion performance test experimental dataset includes at least a corrosion performance test experimental dataset under a salt spray corrosion test condition;

> optionally, the salt spray corrosion test condition includes at least one of the following salt spray conditions: one or more of NaCl aqueous solution salt spray condition, acetic acid salt spray condition, copper-accelerated acetic acid salt spray condition, and alternating salt spray corrosion condition;
> further optionally, the salt spray corrosion test condition includes a NaCl aqueous solution salt spray condition.

Based on any suitable embodiment of this application, further, in some embodiments, the NaCl aqueous solution salt spray condition includes the following parameter: a simulated salt spray condition with a 3wt% to 6wt% NaCl aqueous solution at 34°C-36°C.

When the corrosion performance test experimental dataset includes at least a corrosion performance test experimental dataset under the salt spray corrosion test condition, the constructed corrosion resistance-mechanical property analysis model for the metallic material can be applied to metallic materials and structural components or products thereof in service environments such as road transportation, computer technology, electronic communication, and electrical appliances, as well as structural components or products thereof in related service environments such as electroplating, coating, packaging boxes, and transportation equipment. The road transportation field may involve, but is not limited to, electronic and electrical equipment for road vehicles, equipment and devices for rail transit locomotives, automotive components, and other equipment or metallic structural components thereof. The computer technology field may involve, but is not limited to, computers, displays, hosts, computer components, precision instruments such as medical equipment, and other equipment, products or metallic structural components thereof. The electronic communication field may involve, but is not limited to, mobile phones, radio frequency devices, electronic communication components, printed circuit boards (PCB), printed circuit board assemblies (PCBA), other equipment, products, or metallic structural components thereof. The electrical appliances may involve, but are not limited to, household electrical equipment such as household appliances, lighting fixtures, and transformers, instruments and meters, medical devices, and other devices, products, or metallic structural components thereof.

Without limitation, a model for obtaining corrosion performance test experimental datasets based on the NaCl aqueous solution salt spray condition can be applied to assess the quality and uniformity of protective coatings and to compare differences in salt spray corrosion resistance of samples with similar structures, but is not limited thereto. Without limitation, a model for obtaining corrosion performance test experimental datasets based on the acetic acid salt spray condition can be applied to harsh salt spray environments in coastal cities of southern regions, and the like. Without limitation, a model for obtaining corrosion performance test experimental datasets based on the copper-accelerated acetic acid salt spray condition can be applied to harsh salt spray environments. Without limitation, a model for obtaining corrosion performance test experimental datasets based on the alternating salt spray corrosion condition can be applied to high-temperature and high-humidity environments.

For the NaCl aqueous solution salt spray condition, the above test conditions are conducive to assessing the corrosion resistance of battery casings, automotive components, and the like.

Based on any suitable embodiment of this application, further, in some embodiments, the establishing, based on the corrosion performance test experimental dataset, a first set of empirical relational expressions describing variation of the corrosion parameter with equivalent corrosion time includes:

for each type of corrosion parameter in the corrosion parameter, performing segmented fitting based on the corresponding corrosion performance test experimental dataset, and for each fitting interval, with the corresponding corrosion parameter as a dependent variable and the equivalent corrosion time as an independent variable, performing fitting in the form of a power function or a linear function to construct an empirical relational expression between each type of corrosion parameter and equivalent corrosion time for each fitting interval, thereby obtaining the first set of empirical relational expressions.

The first set of empirical relational expressions describing variation of the corrosion parameter with equivalent corrosion time can be obtained for each type of corrosion parameter through segmented fitting. The fitting for each segment can be performed using a power function or a linear function, resulting in a higher degree of fit between the fitting curves and the experimental test datasets, making the model more effective. However, the function types are not limited to those described above.

Based on any suitable embodiment of this application, further, in some embodiments, in the first set of empirical relational expressions, a fitting form of the power function is $y1 = A \cdot x^B$, and a fitting form of the linear function is $y1 = a + b \cdot x$; where x is the equivalent corrosion time, y1 is the corrosion parameter, A is a positive number, B is a negative number, b is a positive number, and a is a real number;

optionally, a is a negative number.

Based on any suitable embodiment of this application, further, in some embodiments, A is a real number selected from 0.01 to 1.00, B is a real number selected from -0.3 to -0.8, b is a real number selected from 0.001 to 0.05, and a is a real number selected from 0.02 to -0.8;

optionally, a is a real number selected from -0.001 to -0.8, further optionally, a is a real number selected from -0.001 to -0.5;
optionally, b is a real number selected from 0.001 to 0.02, further optionally, b is a real number selected from 0.001 to 0.01.

Appropriate fitting function types are selected, which is conducive to obtaining a first set of empirical relational expressions with better fitting results, thereby obtaining a more effective corrosion resistance-mechanical property analysis model.

Based on any suitable embodiment of this application, further, in some embodiments, the corrosion performance test experimental dataset includes at least one of a corrosion performance test experimental dataset under an electrochemical corrosion test condition and a corrosion performance test experimental dataset under an immersion corrosion test condition;

optionally, the corrosion parameter in the corrosion performance test experimental dataset under the electrochemical corrosion test condition includes at least one of a self-corrosion potential or a self-corrosion current;
optionally, the corrosion parameter in the corrosion performance test experimental dataset under the immersion corrosion test condition includes at least one of a corrosion degree and a corrosion rate.

When the corrosion performance test experimental dataset includes a corrosion performance test experimental dataset under the electrochemical corrosion test condition, the corrosion resistance-mechanical property analysis model for the metallic material can be applied to the performance prediction and service life evaluation of the metallic material in an electrochemical environment, for example, but not limited to, the performance prediction and service life evaluation of battery casings, further including, but not limited to, the performance prediction and service life evaluation of fuel cell casings.

When the corrosion performance test experimental dataset includes a corrosion performance test experimental dataset under the immersion corrosion test condition, the corrosion resistance-mechanical property analysis model for the metallic material can be applied to the performance prediction and service life evaluation of the metallic material in an environment exposed to corrosive liquids.

When the corrosion performance test experimental dataset includes both a corrosion performance test experimental dataset under the electrochemical corrosion test condition and a corrosion performance test experimental dataset under the immersion corrosion test condition, the corrosion resistance-mechanical property analysis model for the metallic material can be applied to the performance prediction and service life evaluation of battery casings containing electrolytes, further including, but not limited to, the performance prediction and service life evaluation of fuel cell casings.

Based on any suitable embodiment of this application, further, in some embodiments, the establishing, based on the mechanical property test experimental dataset, a second set of empirical relational expressions describing variation of the mechanical parameter with equivalent corrosion time includes:

for each type of mechanical parameter in the mechanical parameter, performing segmented fitting based on the corresponding mechanical property test experimental dataset, and for each fitting interval, with the corresponding mechanical parameter as a dependent variable and the equivalent corrosion time as an independent variable, performing fitting in the form of a power function or a linear function to construct an empirical relational expression between each type of mechanical parameter and the equivalent corrosion time, thereby obtaining the second set of empirical relational expressions.

The second set of empirical relational expressions describing variation of the mechanical parameter with equivalent corrosion time can be obtained for each type of mechanical parameter through segmented fitting. The fitting for each segment can be performed using a power function or a linear function, resulting in a higher degree of fit between the fitting curves and the experimental test datasets, making the model more effective. However, the function types are not limited to those described above.

Based on any suitable embodiment of this application, further, in some embodiments, in the second set of empirical relational expressions, a fitting form of the power function is $y2 = M \cdot x^N$, and a fitting form of the linear function is $y2 = m + n \cdot x$; where x is the equivalent corrosion time, y2 is the mechanical parameter, M is a positive number, N is a negative number, n is a negative number, and m is a positive number.

Based on any suitable embodiment of this application, further, in some embodiments, M is a real number selected from 1 to 250, N is a real number selected from -0.01 to -1, n is a real number selected from -0.1 to -5, and m is a real number selected from 1 to 300;

optionally, N is a real number selected from -0.01 to -0.5, further optionally, N is a real number selected from -0.01 to -0.2;

optionally, n is a real number selected from -1 to -3; optionally, n is a real number selected from -0.5 to -1.5; optionally, n is a real number selected from -0.05 to -0.5.

Based on any suitable embodiment of this application, further, in some embodiments, the method for constructing a corrosion resistance-mechanical property analysis model for a metallic material further includes the following step: establishing a third set of empirical relational expressions between equivalent corrosion time under the corrosion test condition and service time in the target service environment.

The empirical relational expressions (which may be referred to as the third set of empirical relational expressions) between the equivalent corrosion time under the corrosion test condition and the service time in the target service environment are established, to convert the equivalent corrosion time into the service time in the target service environment, thereby implementing more direct prediction of the service life of the metallic material and the structural components or products thereof.

Based on any suitable embodiment of this application, further, in some embodiments, the method for constructing a corrosion resistance-mechanical property analysis model for a metallic material includes the following step: establishing a fourth set of empirical relational expressions between a corrosion rate and the equivalent corrosion time under the corrosion test condition, where the first set of empirical relational expressions includes the fourth set of empirical relational expressions.

The empirical relational expressions (which may be referred to as the fourth set of empirical relational expressions) between the corrosion rate and the equivalent corrosion time under the corrosion test condition are established, to dynamically analyze a correlation between the corrosion behavior and mechanical property of the metallic material in the target service environment.

According to a second aspect of this application, a method for analyzing service life of a metallic material is provided, where the metallic material is as defined in the first aspect of this application.

In some embodiments, the method for analyzing service life of a metallic material includes the following steps:

determining a mechanical parameter related to a failure behavior of the metallic material based on a target service environment of the metallic material, determining a corrosion test condition capable of simulating the target service environment, and acquiring a test value of a corrosion degree of the metallic material under the corrosion test condition; and

obtaining a service life parameter of the metallic material under the corrosion test condition based on the test value of the corrosion degree, an effective state threshold of the mechanical parameter, and a corrosion resistance-mechanical property analysis model for the metallic material; where the corrosion resistance-mechanical property analysis model for the metallic material includes at least the following relational expressions: a first set of empirical relational expressions describing variation of a corrosion parameter characterizing the corrosion degree with equivalent corrosion time, and a second set of empirical relational expressions describing variation of the mechanical parameter with equivalent corrosion time.

According to the method for analyzing service life of a metallic material, the mechanical parameter related to the failure behavior of the metallic material is selected based on the target service environment of the metallic material, the corrosion test condition capable of simulating the target service environment is selected, and then the test value of the corrosion degree of the metallic material under the selected corrosion test condition is obtained, thereby obtaining the service life parameter of the metallic material under the selected corrosion test condition based on the test value of the corrosion degree, the effective state threshold of the selected mechanical parameter, and the corrosion resistance-mechanical property analysis model for the metallic material. Using the equivalent corrosion time and the mechanical properties at different corrosion degrees as a link, the corrosion resistance-mechanical property analysis model for the metallic material includes at least the following two relational expressions: a first set of empirical relational expressions describing variation of at least one corrosion parameter of the corrosion degree and the corrosion rate with equivalent corrosion time, and a second set of empirical relational expressions describing variation of the mechanical parameter with equivalent corrosion time. This analysis method can effectively and accurately analyze the service life of the metallic material in the target service environment.

Based on any suitable embodiment of this application, further, in some embodiments, the service life parameter includes at least the equivalent corrosion time.

According to the analysis method, at least the equivalent corrosion time parameter of the metallic material can be obtained, reflecting the service life status of the metallic material.

Based on any suitable embodiment of this application, further, in some embodiments, the obtaining a service life parameter of the metallic material under the corrosion test condition based on the test value of the corrosion degree, an effective state threshold of the mechanical parameter, and a corrosion resistance-mechanical property analysis model for the metallic material includes:

obtaining remaining service time of the metallic material based on the test value of the corrosion degree, the effective state threshold of the mechanical parameter, the corrosion resistance-mechanical property analysis model for the metallic material, and a third set of empirical relational expressions between the equivalent corrosion time under the corrosion test condition and the service time in the target service environment.

The remaining service time of the metallic material can be obtained based on the test value of the corrosion degree of the metallic material, the effective state threshold of the mechanical parameter related to the failure behavior of the metallic material, the corrosion resistance-mechanical property analysis model for the metallic material, and the empirical relational expression between the equivalent corrosion time under the selected corrosion test condition and the service time in the target service environment, implementing predictive analysis of the remaining service life of the metallic material.

Based on any suitable embodiment of this application, further, in some embodiments, the mechanical parameter includes at least two types, and the mechanical parameters are sorted in descending order by degrees of response to failure of the metallic material, and the corrosion resistance-mechanical property analysis model for the metallic material is obtained based on the mechanical parameter ranked first.

The mechanical parameters are sorted in descending order by degrees of response to the failure of the metallic material, and the corrosion resistance-mechanical property analysis model for the metallic material is obtained based on the mechanical parameter ranked first, more effectively reflecting the correlation between the corrosion behavior and mechanical property failure of the metallic material in the target service environment.

Based on any suitable embodiment of this application, further, in some embodiments, the corrosion resistance-mechanical property analysis model for the metallic material is constructed according to the method for constructing a corrosion resistance-mechanical property analysis model for a metallic material provided in the first aspect of this application.

The corrosion resistance-mechanical property analysis model involved in the method for analyzing service life of a metallic material can be constructed using the construction method described in the first aspect of this application, thereby establishing the relational expression between the corrosion degree of the metallic material and the mechanical property including at least one mechanical parameter of the tensile strength at break and the elongation at break.

According to a third aspect of this application, an apparatus for analyzing service life of a metallic material is provided, where the metallic material is as defined in the first aspect of this application.

In some embodiments, the apparatus for analyzing service life of a metallic material includes:

a corrosion performance data acquisition module configured to determine a mechanical parameter related to a failure behavior of the metallic material based on a target service environment of the metallic material, determine a corrosion test condition capable of simulating the target service environment, and acquire a test value of a corrosion degree of the metallic material under the corrosion test condition; and

a corrosion performance data processing module configured to obtain a service life parameter of the metallic material under the corrosion test condition based on the test value of the corrosion degree, an effective state threshold of the mechanical parameter, and a corrosion resistance-mechanical property analysis model for the metallic material; where the corrosion resistance-mechanical property analysis model for the metallic material is as defined in the first or second aspect of this application.

The apparatus for analyzing service life of a metallic material provided in the third aspect of this application can be used to implement the method for analyzing service life of a metallic material provided in the second aspect of this application.

According to a fourth aspect of this application, a method for analyzing a mechanical property of a metallic material is provided, where the metallic material is as defined in the first aspect of this application.

In some embodiments, the method for analyzing a mechanical property of a metallic material includes the following steps:

determining a mechanical parameter related to a failure behavior of the metallic material based on a target service environment of the metallic material, determining a corrosion test condition capable of simulating the target service environment, and acquiring a test value of a corrosion degree of the metallic material under the corrosion test condition;

obtaining, based on the test value of the corrosion degree, a target service time of the metallic material, a third set of empirical relational expressions between equivalent corrosion time under the corrosion test condition and service time in the target service environment, and a corrosion resistance-mechanical property analysis model for the metallic material, a preliminary predicted value of the mechanical parameter of the metallic material having been used for the target service time under the corrosion test condition; where the corrosion resistance-mechanical property analysis model for the metallic material is as defined in the first or second aspect of this application; and

comparing the preliminary predicted value of the mechanical parameter with an effective state threshold of the mechanical parameter to obtain a predicted result of the mechanical parameter of the metallic material having been used for the target service time under the corrosion test condition; where under a condition that the preliminary predicted value of the mechanical parameter is greater than or equal to the effective state threshold of the mechanical parameter, the preliminary predicted value is output as an effective predicted value of the mechanical parameter of the metallic material having been used for the target service time under the corrosion test condition; or under a condition that the preliminary predicted value of the mechanical parameter is less than the effective state threshold of the mechanical parameter, a prediction result indicating that the metallic material fails before the target service time is reached is output.

According to the method for analyzing a mechanical property of a metallic material, the mechanical parameter related to the failure behavior of the metallic material is selected based on the target service environment of the metallic material, the corrosion test condition capable of simulating the target service environment is selected, and then test value of the corrosion degree of the metallic material under the selected corrosion test condition is acquired, thereby obtaining, based on the test value of the corrosion degree, the target service time of the metallic material, the empirical relational expression between the equivalent corrosion time under the selected corrosion test condition and the service time in the target service environment, and the corrosion resistance-mechanical property analysis model for the metallic material, the preliminary predicted value of the mechanical parameter of the metallic material having been used for the target service time under the selected corrosion test condition. The preliminary predicted value of the mechanical parameter is compared with the effective state threshold of the mechanical parameter, to obtain the predicted result of the mechanical parameter of the metallic material having been used for the target service time under the selected corrosion test condition. Under a condition that the preliminary predicted value of the mechanical parameter is greater than or equal to the effective state threshold of the mechanical parameter, the effective predicted value of the mechanical parameter of the metallic material having been used for the target service time under the selected corrosion test condition is equal to the preliminary predicted value. Under a condition that the preliminary predicted value of the mechanical parameter is less than the effective state threshold of the mechanical parameter, it indicates that the metallic material fails before the target service is reached.

According to a fifth aspect of this application, an apparatus for analyzing a mechanical property of a metallic material is provided, where the metallic material is as defined in the first aspect of this application.

In some embodiments, the apparatus for analyzing a mechanical property of a metallic material includes:

a test data acquisition module configured to determine a mechanical parameter related to a failure behavior of the metallic material based on a target service environment of the metallic material, determine a corrosion test condition capable of simulating the target service environment, and acquire a test value of a corrosion degree of the metallic material under the corrosion test condition;

a test data processing module configured to obtain, based on the test value of the corrosion degree, a target service time of the metallic material, a third set of empirical relational expressions between equivalent corrosion time under the corrosion test condition and service time in the target service environment, and a corrosion resistance-mechanical property analysis model for the metallic material, a preliminary predicted value of the mechanical parameter of the metallic material having been used for the target service time under the corrosion test condition; where the corrosion resistance-mechanical property analysis model for the metallic material is as defined in the first or second aspect of this application; and

a mechanical property classification and identification module configured to compare the preliminary predicted value of the mechanical parameter with an effective state threshold of the mechanical parameter to obtain a predicted result of the mechanical parameter of the metallic material having been used for the target service time under the corrosion test condition; where under a condition that the preliminary predicted value of the mechanical parameter is greater than or equal to the effective state threshold of the mechanical parameter, an effective predicted value of the mechanical parameter of the metallic material having been used for the target service time under the corrosion test condition is equal to the preliminary predicted value.

The apparatus for analyzing a mechanical property of a metallic material provided in the fifth aspect of this application can be used to implement the method for analyzing a mechanical property of a metallic material provided in the fourth aspect of this application.

According to a sixth aspect of this application, a computer device is provided, including a memory and a processor, where the memory stores a computer program, and when the processor executes the computer program, the steps of the method for constructing a corrosion resistance-mechanical property analysis model for a metallic material provided in the first aspect of this application, or the method for analyzing service life of a metallic material provided in the second aspect, or the method for analyzing a mechanical property of a metallic material provided in the fourth aspect are implemented.

According to a seventh aspect of this application, a computer-readable storage medium is provided, where a computer program is stored thereon, and when the computer program is executed by a processor, the steps of the method for constructing a corrosion resistance-mechanical property analysis model for a metallic material provided in the first aspect of this application, or the method for analyzing service life of a metallic material provided in the second aspect, or the method for analyzing a mechanical property of a metallic material provided in the fourth aspect are implemented.

According to another aspect of this application, a computer program product is provided, including a computer program, where when the computer program is executed by a processor, the steps of the method for constructing a corrosion resistance-mechanical property analysis model for a metallic material provided in the first aspect of this application, or the method for analyzing service life of a metallic material provided in the second aspect, or the method for analyzing a mechanical property of a metallic material provided in the fourth aspect are implemented.

According to an eighth aspect of this application, an electric apparatus is provided, including:

a battery system, including a battery casing and a battery cell located inside the battery casing, where the battery casing includes the metallic material according to the first aspect of this application; and

at least one of the apparatus for analyzing service life of a metallic material according to the third aspect of this application, the apparatus for analyzing a mechanical property of a metallic material according to the fifth aspect, the computer device according to the sixth aspect, and the computer-readable storage medium according to the seventh aspect.

In some embodiments, the battery cell includes a fuel cell.

In some embodiments, the battery cell includes a lithium battery cell.

At least one of the apparatus for analyzing service life of a metallic material, the apparatus for analyzing a mechanical property of a metallic material, the computer device, the computer-readable storage medium, and the computer program product can be provided in the electric apparatus to implement the method for constructing the analysis model, the method for analyzing service life of a metallic material, or the method for analyzing a mechanical property of a metallic material, facilitating better management and maintenance of the electric apparatus. This not only facilitates the safety maintenance of the battery casing in the electric apparatus, but also facilitates the design of battery casings with longer service time.

The details of one or more embodiments of this application are set forth in the accompanying drawings and description below. Other features, objects, and advantages of this application become apparent from the specification, accompanying drawings, and claims.

## BRIEF DESCRIPTION OF DRAWINGS

To better describe and illustrate the embodiments or examples provided in this application, reference may be made to one or more drawings. The additional details or examples used to describe the drawings should not be considered as limiting the scope of the disclosed application, the currently described embodiments or examples, or the best mode of these applications as currently understood. In all drawings, the same reference signs denote the same components. It should also be noted that the drawings are drawn in a simplified form and are only used to conveniently and clearly assist in describing this application. The dimensions of each component shown in the drawings are arbitrarily illustrated, may be accurate, or may not be drawn to actual scale. For example, to make the illustrations clearer, the dimensions of some components in the drawings are appropriately exaggerated. Unless otherwise specified, the components in the drawings are not drawn to scale. This application does not limit the dimensions of each component.

In the drawings:

FIG. 1 is a flowchart of a method for constructing a corrosion resistance-mechanical property analysis model for a metallic material according to an embodiment of this application.

FIG. 2 is a flowchart of a method for constructing a corrosion resistance-mechanical property analysis model for a metallic material according to an embodiment of this application.

FIG. 3 is a flowchart of a method for constructing a corrosion resistance-mechanical property analysis model for a metallic material according to an embodiment of this application.

FIG. 4 is a flowchart of a method for constructing a corrosion resistance-mechanical property analysis model for a metallic material according to an embodiment of this application.

FIG. 5 is a flowchart of a method for constructing a corrosion resistance-mechanical property analysis model for a metallic material according to an embodiment of this application.

FIG. 6 is a flowchart of a method for analyzing service life of a metallic material according to an embodiment of this application.

FIG. 7 is a schematic diagram of an apparatus for analyzing service life of a metallic material according to an embodiment of this application.

FIG. 8 is a flowchart of a method for analyzing a mechanical property of a metallic material according to an embodiment of this application.

FIG. 9 is a flowchart of a method for analyzing a mechanical property of a metallic material according to an embodiment of this application.

FIG. 10 is a schematic diagram of an apparatus for analyzing a mechanical property of a metallic material according to an embodiment of this application.

FIG. 11 is an internal structure diagram of a computer device according to an embodiment of this application.

FIG. 12 is a schematic diagram of an electric apparatus according to an embodiment of this application.

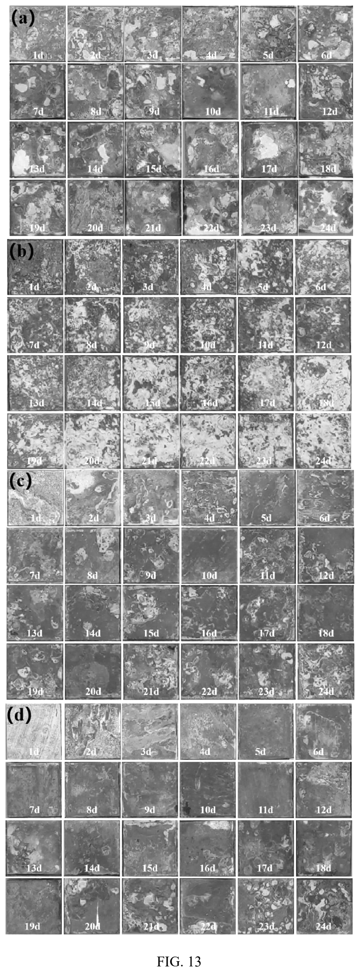

FIG. 13 shows results of a salt spray test in some embodiments of this application, illustrating the macroscopic morphology of four aluminum alloy materials from day 1 to day 24 at different equivalent corrosion time points: (a) A356.2; (b) A380; (c) AlSi10MgMn; and (d) AlSi9MnMoZr; where samples of each aluminum alloy material were taken daily, with the first row from left to right corresponding to day 1 (1d) to day 6 (6d), the second row from left to right corresponding to day 7 (7d) to day 12 (12d), the third row from left to right corresponding to day 13 (13d) to day 18 (18d), and the fourth row from left to right corresponding to day 19 (19d) to day 24 (24d).

FIG. 14 shows the results of a salt spray test in some embodiments of this application, illustrating the mass loss and corrosion rate of four aluminum alloy materials at different equivalent corrosion time points, where the four aluminum alloy materials are A356.2 (square, ■), A380 (circle, •), AlSi10MgMn (upward triangle, ▲), and AlSi9MnMoZr (downward triangle, ▼).

FIG. 15 shows fitting results of corrosion rate versus equivalent corrosion time for A356.2 aluminum alloy in a salt spray corrosion test according to an embodiment of this application.

FIG. 16 shows fitting results of corrosion rate versus equivalent corrosion time for A380 aluminum alloy in a salt spray corrosion test according to an embodiment of this application.

FIG. 17 shows fitting results of corrosion rate versus equivalent corrosion time for AlSi10MgMn aluminum alloy in a salt spray corrosion test according to an embodiment of this application.

FIG. 18 shows fitting results of corrosion rate versus equivalent corrosion time for AlSi9MnMoZr aluminum alloy in a salt spray corrosion test according to an embodiment of this application.

FIG. 19 shows experiment results and fitting results of mechanical parameters versus equivalent corrosion time for A356.2 aluminum alloy in a salt spray corrosion test at different equivalent corrosion time points in some embodiments of this application; where the mechanical parameters include a tensile strength at break (tensile strength, abbreviated as UTS), an elongation at break (elongation, abbreviated as El), and a yield strength (yield strength, abbreviated as YS); where fit corresponds to a fitting curve.

FIG. 20 shows experiment results and fitting results of mechanical parameters versus equivalent corrosion time for A380 aluminum alloy in a salt spray corrosion test at different equivalent corrosion time points in some embodiments of this application.

FIG. 21 shows experiment results and fitting results of mechanical parameters versus equivalent corrosion time for AlSi10MgMn aluminum alloy in a salt spray corrosion test at different equivalent corrosion time points in some embodiments of this application.

FIG. 22 shows experiment results and fitting results of mechanical parameters versus equivalent corrosion time for AlSi9MnMoZr aluminum alloy in a salt spray corrosion test at different equivalent corrosion time points in some embodiments of this application.

FIG. 23 is a schematic diagram of sample dimensions for a tensile test according to an embodiment of this application, in units of millimeters (mm), where R2.5 indicates a radius of 2.5 millimeters.

FIG. 24 is a schematic structural diagram of a three-electrode system in electrochemical corrosion test equipment used in an embodiment of this application, where R, mA, and V represent a resistance meter, an ammeter, and a voltmeter, respectively, and their combination can be understood as an electrochemical workstation which can collect sample signals as measurement data for corrosion parameters by adjusting given voltage and current.

FIG. 25 shows variation curves of open circuit potential (OCP, chemical formula, unit: V) with time for four aluminum alloy materials using a 3.5wt% NaCl aqueous solution, where the four aluminum alloy materials are A356.2 (square, ■), A380 (circle, •), AlSi10MgMn (upward triangle, ▲), and AlSi9MnMoZr (downward triangle, ▼).

FIG. 26 shows electrochemical corrosion test and analysis results in some embodiments of this application, where (a) is an EIS Bode plot (electrochemical impedance spectroscopy Bode plot); (b) is a phase plot (phase plot), with the horizontal axis corresponding to frequency (unit: Hz (Hertz)) and the vertical axis corresponding to phase angle (unit: ° (degree)); (c) is a Nyquist plot (Nyquist plot); and (d) is an equivalent circuit diagram.

FIG. 27 shows potentiodynamic polarization curves of an electrochemical corrosion test in some embodiments of this application, with the horizontal axis representing chemical formula (unit: V) and the vertical axis representing current density (unit: $A/cm^2$).

FIG. 28 shows macroscopic morphologies of sample surfaces at different immersion corrosion times in immersion corrosion tests according to some embodiments of this application, from left to right corresponding to four aluminum alloy materials: (a) A356.2; (b) A380; (c) AlSi10MgMn; and (d) AlSi9MnMoZr; and from top to bottom corresponding to the initial morphology before corrosion (0d, scale bar 50 mm), and the macroscopic morphology at 10 days (10d), 20 days (20d), and 30 days (30d).

FIG. 29 shows a summary of corrosion rates of four die-cast aluminum alloy materials at different immersion corrosion times in the immersion corrosion test shown in FIG. 28, where the four aluminum alloy materials are A356.2, A380, AlSi10MgMn, and AlSi9MnMoZr.

FIG. 30 shows a relationship between corrosion rates and corrosion times in salt spray corrosion tests for different aluminum alloy materials N1 and A380 according to an embodiment of this application, where corrosion rates I, II, and III at different corrosion times represent the initial, middle, and late stages of corrosion, respectively.

FIG. 31 shows a relationship describing variation of mechanical parameters with corrosion time in a tensile test for aluminum alloy material N1 according to an embodiment of this application, where (a) represents stress-strain curves, including stress-strain curves corresponding to an initial state and corrosion time of 5 days (5d), 15 days (15d), and 25 days (25d); and (b) represents fitting results of mechanical properties, including results and fitting curves for tensile strength at break (tensile strength), elongation at break (elongation), and yield strength (yield strength).

Description of reference signs:

310. corrosion performance data acquisition module; 320. corrosion performance data processing module; 510. test data acquisition module; 520. test data processing module; 530. mechanical property classification and identification module; 6. electric apparatus; 131. auxiliary electrode; 132. working electrode; and 133. reference electrode.

## DESCRIPTION OF EMBODIMENTS

Hereinafter, some implementations and embodiments of the method for constructing a corrosion resistance-mechanical property analysis model for a metallic material and an application thereof are described in detail with appropriate reference to the drawings. However, unnecessary detailed descriptions may be omitted. For example, detailed descriptions of well-known matters or repetitive descriptions of substantially identical structures may be omitted. This is to avoid unnecessarily lengthy descriptions and facilitate understanding by those skilled in the art. In addition, the drawings and the following descriptions are provided for those skilled in the art to fully understand this application and are not intended to limit the subject matter described in the claims.

The "ranges" disclosed in this application may be defined in the form of lower and upper limits, where a given range is defined by selecting a lower limit and an upper limit, and the selected lower and upper limits define the boundaries of a specific range. Ranges defined in this manner may include or exclude the endpoints, and any endpoint may be independently included or excluded, and may be arbitrarily combined, i.e., any lower limit may be combined with any upper limit to form a range. For example, if ranges of 60-120 and 80-110 are listed for a specific parameter, it is understood that ranges of 60-110 and 80-120 are also contemplated. In addition, if minimum range values of 1 and 2 are listed, and maximum range values of 3, 4, and 5 are listed, the following ranges are all contemplated: 1-3, 1-4, 1-5, 2-3, 2-4, and 2-5. In this application, unless otherwise specified, the numerical range "a-b" represents a shorthand notation for any combination of real numbers between a and b, where a and b are both real numbers. For example, the numerical range "0-5" means that all real numbers between "0-5" have been listed herein, and "0-5" is merely a shorthand notation for these numerical combinations. Additionally, when a parameter is expressed as an integer greater than or equal to 2, it is equivalent to disclosing that the parameter is, for example, the integer 2, 3, 4, 5, 6, 7, 8, 9, 10, 11, 12, or the like. For example, when a parameter is expressed as an integer selected from "2-10", it is equivalent to listing the integers 2, 3, 4, 5, 6, 7, 8, 9, and 10.

In this application, terms such as "multiple", "various", or "several", unless specifically limited, refer to a quantity greater than 2 or equal to 2. For example, "one or more" means one or two or more. It is understood that "any number" of items means a combination of any suitable number of items, that is, a combination of "any number" of items in a manner that does not conflict and can implement this application.

Unless otherwise specified, all embodiments and optional embodiments of this application can be combined with each other to form new technical solutions.

Reference to an "embodiment" herein means that a particular feature, structure, or characteristic described in connection with the embodiment may be included in at least one embodiment of this application. The appearance of this phrase in various places in the specification does not necessarily refer to the same embodiment, nor is it an independent or alternative embodiment mutually exclusive with other embodiments. Those skilled in the art explicitly and implicitly understand that the embodiments described herein may be combined with other embodiments. The term "embodiment" has a similar understanding.

Those skilled in the art can understand that in the methods of various embodiments, the order of writing the steps does not imply a strict execution order that imposes any limitation on the implementation process, and the specific execution order of the steps should be determined by their function and possible internal logic. Unless otherwise specified, all steps of this application can be performed sequentially or randomly, preferably sequentially. For example, a method M includes steps (a) and (b), indicating that method M may include steps (a) and (b) performed sequentially, or steps (b) and (a) performed sequentially. For another example, method M may also include step (c), indicating that step (c) can be added to method M in any order, for example, method M may include steps (a), (b), and (c), or steps (a), (c), and (b), or steps (c), (a), and (b), or the like.

In this application, unless otherwise specified, open-ended technical features or solutions described with terms such as "contain", "include", or "comprise" do not exclude additional members beyond those listed and can be considered to provide both closed-ended features or solutions consisting of the listed members and open-ended features or solutions including additional members beyond the listed members. For example, A includes a1, a2, and a3, unless otherwise specified, may also include other members or may not include additional members, and may be regarded as providing both features or solutions where "A consists of a1, a2, and a3" or "A is selected from a1, a2, and a3", and features or solutions where "A includes not only a1, a2, and a3 but also other members".

In this application, unless otherwise specified, A (for example, B) indicates that B is a non-limiting example of A, and may be understood as A being not limited to B.

In this application, unless otherwise specified, "optionally", "optional", or "option" means that it may or may not be included, that is, it refers to either of the two parallel options of "included" or "not included". If multiple "optional" instances appear in a technical solution without contradictions or mutual constraints, unless otherwise specified, each "optional" instance is independent. Unless otherwise specified, descriptions such as "optionally include" or "optionally comprise" in this application, taking "optionally include" as an example, mean "may include or may not include".

In this application, unless otherwise specified, "and/or" corresponding features or solutions include any single item among two or more related listed items, as well as any and all combinations of the related listed items, where "any and all combinations" include any two related listed items, any more related listed items, or all related listed items. For example, "A and/or B" refers to the group consisting of A, B, and "a combination of A and B". "Including A and/or B" can mean "including A, including B, and including A and B", or "containing A, containing B, or containing A and B", and can be appropriately understood based on the context of the statement.

The terms "combinations thereof", "any combinations thereof", or "any combination manners thereof" used herein include all suitable combinations of any two or more of the listed items.

The term "suitable" in phrases such as "suitable combinations", "suitable manners", or "any suitable manners" refers to combinations that enable the implementation of the technical solutions of this application.

The terms "preferably", "better", "more preferably", "suitably", or "more suitably" used herein merely describe embodiments or examples with better effects and should be understood as not limiting the scope of protection of this application. If multiple "preferably" instances appear in a technical solution without contradictions or mutual constraints, unless otherwise specified, each "preferably" instance is independent.

In this application, terms such as "further", "furthermore", "particularly", "for example", "such as", "example", or "by way of example" are used for descriptive purposes to indicate differences in content but should not be understood as limiting the scope of protection of this application.

In this application, terms such as "first aspect", "second aspect", "third aspect", and "fourth aspect" are used for descriptive purposes only and should not be understood as indicating or implying relative importance or quantity, nor as implying the importance or quantity of the indicated technical features. Moreover, the terms "first", "second", "third", "fourth", and the like serve only as non-exhaustive enumerative descriptions and should not be understood as imposing a closed limitation on quantity.

In this application, the term "room temperature" generally refers to 4°C-35°C, and may refer to 20°C $\pm$ 5°C. In some embodiments of this application, room temperature refers to 20°C-30°C.

In this application, for units of data ranges, if a unit is specified only after the right endpoint, it indicates that the units of the left and right endpoints are the same. For example, 3-5 h or 3-5 h both indicate that the units of the left endpoint "3" and the right endpoint "5" are h (hours), and both have the same meaning as 3h-5h. Other parameters such as temperature and dimensions are understood in the same manner.

The weight of related components mentioned in the embodiments or examples of this application may refer not only to the content of each component but also to the proportional relationship of weights between components. Therefore, scaling up or down the content of related components in accordance with the embodiments or examples of this application is within the scope described by this application. Further, the weight involved in the embodiments or examples of this application may be in units of mass commonly known in the chemical field, such as $\mu g$, mg, g, and kg. Unless otherwise specified, the mass ratio is numerically equal to the corresponding weight ratio. For example, if the mass of substance A is m1 and its weight is W1, and the mass of substance B is m2 and its weight is W2, the mass ratio m1/m2 is numerically equal to the corresponding weight ratio W1/W2.

In this application, unless otherwise specified, wt% refers to the weight percentage by weight, which is numerically equal to the corresponding mass percentage by mass.

In this application, unless otherwise specified, "greater than/equal to" and "greater than or equal to" can both be represented as "$\geq$", "less than/equal to" and "less than or equal to" can both be represented as "$\leq$", "greater than" can be equivalently represented as ">", and "less than" can be equivalently represented as "<." In this application, unless otherwise specified, "greater than or equal to" and "$\geq$" can be considered to include both "greater than" and "equal to" options. In this application, unless otherwise specified, "less than or equal to" and "$\leq$" can be considered to include both "less than" and "equal to" options.

In this application, exemplary descriptions such as "in some embodiments (or examples) " or "in one embodiment (or example) " may include, but are not limited to, the following meanings: these solutions can be combined with other solutions in a suitable manner to form new technical solutions.

Current research on the corrosion behaviors and mechanisms of fuel cell casing materials usually focuses only on the electrochemical corrosion behaviors or corrosion rates of fuel cell casing materials in different corrosive media, making it difficult to provide effective guidance for the design and development of fuel cell casing materials. The lack of systematic and in-depth research on the corrosion behaviors of fuel cell casing materials are becoming increasingly apparent, such as the unclear white rust corrosion mechanism of fuel cell casing materials, the need to clarify the impact of metallic materials (such as alloy materials) on the corrosion behaviors of fuel cell casing materials, and the lack of correlated studies linking fuel cell casing materials to their mechanical properties and service life.

According to various implementations and embodiments of this application, this application provides a method for constructing a corrosion resistance-mechanical property analysis model for a metallic material and an application thereof. The application relates to an electric apparatus including a battery system.

According to various implementations and embodiments of this application, this application provides a method for constructing a corrosion resistance-mechanical property analysis model for a metallic material, a method and an apparatus for analyzing service life of a metallic material, a method and an apparatus for analyzing a mechanical property of a metallic material, a computer device, a computer-readable storage medium, and an electric apparatus. The corrosion resistance-mechanical property analysis model for a metallic material can be effectively used for predicting mechanical properties and evaluating service life of the metallic material. The metallic material involved may include, but is not limited to, an aluminum alloy material, and may also include, but is not limited to, a battery casing material (such as a fuel cell casing material).

According to a first aspect of this application, a method for constructing a corrosion resistance-mechanical property analysis model for a metallic material is provided. This construction method can be used to construct an analysis model for corrosion degrees and mechanical properties of the metallic material under a corrosion test condition simulating a target service environment, and can be used for predicting mechanical properties of the metallic material. When the mechanical properties are mechanical properties related to the failure behavior of the metallic material, this construction method can obtain an analysis model for corrosion degrees and failure-related mechanical properties of the metallic material under a corrosion test condition simulating a target service environment, and can be used for predicting and analyzing the mechanical properties and evaluating the service life of the metallic material.

In some embodiments, this application provides a method for constructing a corrosion resistance-mechanical property analysis model for a metallic material.

In some embodiments, the method for constructing the analysis model includes the following steps: using a metallic material as a test object, acquiring a corrosion performance test experimental dataset at different equivalent corrosion time points under a corrosion test condition, and a mechanical property test experimental dataset at different corrosion degrees corresponding to the different equivalent corrosion time points; and establishing empirical relational expressions describing variations of a corrosion parameter and a mechanical parameter with equivalent corrosion time. Further, the corrosion test condition may be used to simulate a target service environment.

A first set of empirical relational expressions describing variation of the corrosion parameter with equivalent corrosion time can be established based on the corrosion performance test experimental dataset.

A second set of empirical relational expressions describing variation of the mechanical parameter with equivalent corrosion time can be established based on the mechanical property test experimental dataset.

In some embodiments, a method for constructing a corrosion resistance-mechanical property analysis model for a metallic material is provided, including the following steps:

using a metallic material as a test object, acquiring a corrosion performance test experimental dataset at different equivalent corrosion time points under a corrosion test condition, and a mechanical property test experimental dataset at different corrosion degrees corresponding to the different equivalent corrosion time points; where the corrosion test condition is used to simulate a target service environment; and

establishing, based on the corrosion performance test experimental dataset, a first set of empirical relational expressions describing variation of a corrosion parameter with equivalent corrosion time, and establishing, based on the mechanical property test experimental dataset, a second set of empirical relational expressions describing variation of a mechanical parameter with equivalent corrosion time.

This analysis model can be effectively used for predicting the mechanical properties and evaluating the service life of the metallic material. The metallic material may include an aluminum alloy material, and further may include a battery casing material.

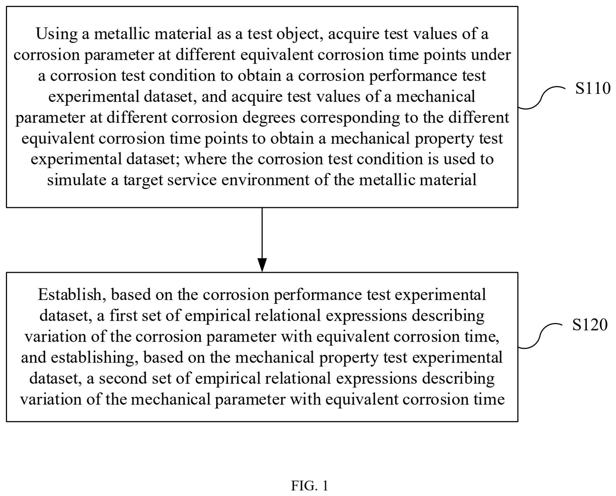

In some embodiments, a method for constructing a corrosion resistance-mechanical property analysis model for a metallic material is provided, including the following steps (as shown in FIG. 1):

S110: using a metallic material as a test object, acquiring test values of a corrosion parameter at different equivalent corrosion time points under a corrosion test condition to obtain a corrosion performance test experimental dataset, and acquiring test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate; and

S120: establishing, based on the corrosion performance test experimental dataset, a first set of empirical relational expressions describing variation of the corrosion parameter with equivalent corrosion time, and establishing, based on the mechanical property test experimental dataset, a second set of empirical relational expressions describing variation of the mechanical parameter with equivalent corrosion time.

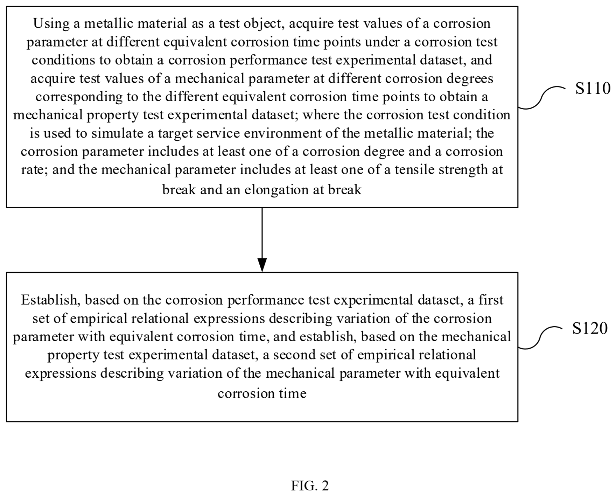

In some embodiments, a method for constructing a corrosion resistance-mechanical property analysis model for a metallic material is provided, including the following steps (as shown in FIG. 2):

S110: using a metallic material as a test object, acquiring test values of a corrosion parameter at different equivalent corrosion time points under a corrosion test condition to obtain a corrosion performance test experimental dataset, and acquiring test values of a mechanical parameter at different corrosion degrees corresponding to the different equivalent corrosion time points to obtain a mechanical property test experimental dataset; where the corrosion test condition is used to simulate a target service environment of the metallic material; the corrosion parameter includes at least one of a corrosion degree and a corrosion rate; and the mechanical parameter includes at least one of a tensile strength at break and an elongation at break; and

S120: establishing, based on the corrosion performance test experimental dataset, a first set of empirical relational expressions describing variation of the corrosion parameter with equivalent corrosion time, and establishing, based on the performance test experimental dataset, a second set of empirical relational expressions describing variation of the mechanical parameter with equivalent corrosion time.

In this application, unless otherwise specified, the "metallic material" refers to a solid material with specific macroscopic dimensions, for example, but not limited to, plates or blocks.

In this application, unless otherwise specified, the "service environment" refers to an environment in which a metallic material is used in actual application scenarios.

In this application, unless otherwise specified, the "corrosion degree" refers to a degree of structural degradation of the metallic material compared to its uncorroded state, which can be characterized by, but is not limited to, the "mass loss relative to the uncorroded state". A greater mass loss means a higher corrosion degree.

In this application, unless otherwise specified, the "equivalent corrosion time" means that under a corrosion test condition capable of simulating a target service environment, there is a mapping relationship between corrosion test time of a metallic material and service time in the target service environment, allowing the service time to be indirectly reflected through the corrosion test time; therefore, this corrosion test time is also referred to as "equivalent corrosion time".

In this application, unless otherwise specified, the "corrosion rate" refers to an amount of corrosion loss per unit time. The amount of corrosion loss can be expressed in ways including, but not limited to, mass, volume, or linear length (such as corrosion depth). The corrosion rate can be obtained by dividing total loss over a period by duration of that period.

In this application, unless otherwise specified, the "tensile strength at break" and the "elongation at break" have well-known meanings in the art. The "tensile strength at break", also known as tensile strength or ultimate tensile strength, refers to a maximum tensile stress that a sample can withstand in a tensile test, numerically equal to the tensile force borne by the sample at the point of fracture. The "elongation at break" refers to a percentage of a deformed length at fracture to an original length, where the original length is a length of the sample before application of tensile force. For a sample with a length $L_0$, when a tensile force is applied in a length direction, a ratio of the tensile force $F_{max}$ borne by the sample at fracture to a cross-sectional area A is equal to the "tensile strength at break", where the "cross-section" refers to a section perpendicular to the length direction of the sample; and "elongation at break" $\delta = (L - L_0) \times 100\%$, where L is a length of the sample at fracture under tensile force.

Using a metallic material as a test object, a target service environment of the metallic material is simulated through a corrosion test condition, to obtain a corrosion performance test experimental dataset at different equivalent corrosion time points and a mechanical property test experimental dataset at different corrosion degrees corresponding to the different equivalent corrosion time points; and using equivalent corrosion time under the corrosion test condition and mechanical properties at different corrosion degrees as a link, a first set of empirical relational expressions describing variation of at least one corrosion parameter of a corrosion degree and a corrosion rate with equivalent corrosion time is established, and a second set of empirical relational expressions describing variation of at least one mechanical parameter with equivalent corrosion time, thereby constructing a corrosion resistance-mechanical property analysis model for the metallic material. This model can help understand the corrosion mechanism of the metallic material and can be used to predict and analyze the mechanical properties of the metallic material under a target service condition and evaluate the service life of metallic material structural components in the target service environment.

Based on any suitable embodiment of this application, further, in some embodiments, the mechanical parameter includes at least one of a tensile strength at break and an elongation at break.

When the mechanical parameter in the method includes at least one of the tensile strength at break and the elongation at break, the constructed corrosion resistance-mechanical property analysis model for the metallic material can be applied to metallic material structural components that are prone to failure due to tensile stress.

Based on any suitable embodiment of this application, further, in some embodiments, the mechanical parameter in the mechanical property test experimental dataset includes at least one of a tensile strength at break and an elongation at break, and further optionally includes a yield strength.

Based on any suitable embodiment of this application, further, in some embodiments, the mechanical parameter in the mechanical property test experimental dataset includes at least a tensile strength at break and an elongation at break, and further optionally includes a yield strength.

Based on any suitable embodiment of this application, further, in some embodiments, the mechanical parameter includes a yield strength.

Appropriate parameters are selected for fitting functional relational expressions, which is conducive to obtaining the second set of empirical relational expressions with better fitting results, thereby helping to obtain a more effective corrosion resistance-mechanical property analysis model.

In this application, unless otherwise specified, tensile tests can be conducted on an electronic tensile testing machine, for example, on a Z20 TEW electronic tensile testing machine. In this application, unless otherwise specified, the loading rate for tensile testing may be 0.5 mm/min to 1.5 mm/min.

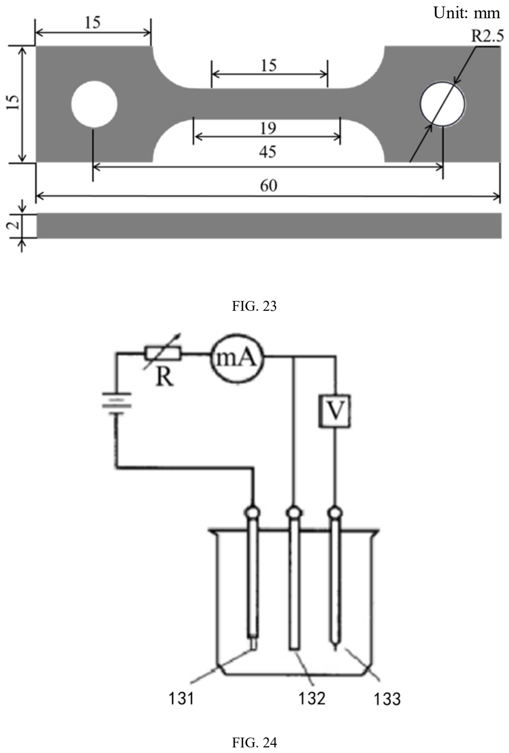

Without limitation, tensile tests can be conducted using samples with the dimensions shown in FIG. 23. The sample in FIG. 23 is a plate-shaped tensile specimen with a length of 60 millimeters (mm) and a thickness of 2 mm.

Without limitation, during tensile tests, the tensile sample to be tested can be wrapped with a blue film, leaving only the test surface exposed. The total test duration can be set to 600 hours (h). During the test, a batch of samples can be taken every 24 hours for tensile property testing, with at least three parallel samples for each test.

Salt spray corrosion test experiments can be conducted using the salt spray corrosion test equipment commonly used in the art. Without limitation, an LP/YWX-250 model salt spray corrosion test equipment can be used. During the test, samples can be placed on a V-shaped bracket to allow salt spray to fall slowly and uniformly onto the sample surface.

In this application, unless otherwise specified, the average corrosion depth per unit time can be used to characterize the corrosion rate. Further, the corrosion rate can be calculated as follows:

$$\text{Corrosion rate} = (K \times W)/(A \times T \times D) \text{ mm/y} \qquad (I)$$

In formula (I): $K = 8.64 \times 10^4$, and K is a time constant;

W is a mass difference before and after the test, in mg;
A is a test surface area, in $cm^2$;
T is test duration, in h;
$D = 2.7 \text{ g/cm}^3$, and D is a density of the test sample.

In formula (I), the corrosion rate is in the unit of millimeters per year, denoted as "mm/y".

Based on any suitable embodiment of this application, further, in some embodiments, the metallic material is an aluminum alloy material. The metallic material may alternatively be a metallic structural component, and further may be an aluminum alloy structural component.

Based on any suitable embodiment of this application, further, in some embodiments, the metallic material is a battery casing material, which can be any one of battery casing materials. In some embodiments, the battery casing material may include a fuel cell casing material. In some embodiments, the battery casing material includes a lithium battery casing material.

In some embodiments, the metallic material includes at least a portion of a structural component of a battery casing. In some embodiments, the battery casing structural component may include at least a portion of a structural component of a fuel cell casing. In some embodiments, the battery casing structural component may include at least a portion of a structural component of a lithium battery casing.

In this application, unless otherwise specified, the "structural component" may be a standalone object or a part of a standalone object. The "metallic structural component" is a structural component made of a metallic material.

In this application, unless otherwise specified, the "battery casing material" refers to a main material constituting the battery casing, which is a main material of at least a portion of a structure of the battery casing, A mass proportion of the battery casing material in that portion of the structure may exceed 80%, further may exceed 90%, and even further may be close to 100% or 100%. In some embodiments, the battery casing material may refer to a constituent material of the battery casing, meaning that at least a portion of the structural component of the battery casing is made of this "battery casing material".

In this application, unless otherwise specified, the "fuel cell casing" refers to a battery casing containing a fuel cell; the "fuel cell casing material" refers to a main material constituting the fuel cell casing, which is a main material of at least a portion of a structure of the fuel cell casing. Unless otherwise specified, a mass proportion of the fuel cell casing material in that portion of the structure may exceed 80%, further may exceed 90%, and even further may be close to 100% or 100%. In some embodiments, the fuel cell casing material may refer to a constituent material of the fuel cell casing, meaning that at least a portion of a structural component of the fuel cell casing is made of this "fuel cell casing material".

In this application, unless otherwise specified, the "fuel cell" has the well-known meaning in the art, and refers to a chemical apparatus that directly converts the chemical energy of fuel into electrical energy. The service environment of a fuel cell casing is prone to contact with various corrosive media, including moisture, rainwater, salt spray, various organic and inorganic liquids used during vehicle operation or cleaning, and the like.

Due to the advantages such as low density, high specific strength, excellent thermal stability, good machinability, and low cost, die-cast aluminum alloys can be used to prepare various automotive components, including, but not limited to, engine blocks, generator housings, fuel cell casings, and various engine brackets.

The metallic material may be an aluminum alloy material or a metallic structural component, and further may be an aluminum alloy structural component. Due to the advantages such as low density, high specific strength, excellent thermal stability, good machinability, and low cost, die-cast aluminum alloys can be used to prepare various automotive components, including, but not limited to, engine blocks, generator housings, fuel cell casings, and various engine brackets. The aluminum alloy material can be applied in the field of battery technology and can be used as a main body material (including a constituent material) for battery casings or aluminum alloy structural components in battery casings. Therefore, the metallic material may be a battery casing material, and the battery casing may include, but is not limited to, a fuel cell casing or a lithium battery casing; and correspondingly, the metallic material may include at least a portion of a structural component of a battery casing. When the corrosion parameter and the mechanical parameter are obtained based on the aluminum alloy materials, the constructed corrosion resistance-mechanical property analysis model for the metallic material can be applied to mechanical property prediction and service life evaluation of the aluminum alloy material, and also facilitates understanding of the white rust corrosion mechanism of the aluminum alloy material. When the aluminum alloy material is used as the main body material or constituent material for battery casings or structural components in battery casings, the corrosion resistance-mechanical property analysis model for the metallic material can be applied to mechanical property prediction and service life evaluation of battery casings or structural components in battery casings.

Taking a fuel cell casing as an example, the model also facilitates understanding of the white rust corrosion mechanism of fuel cell casing materials. Using fuel cell casing materials as the test objects, corrosion behaviors of the fuel cell casing materials can be analyzed, corrosion rates of different types of fuel cell casing materials can be determined, and relational expressions between the corrosion degrees and mechanical properties of fuel cell casing materials can be established, providing reference for the safe operation of fuel cells and electric apparatuses including fuel cells.

Based on any suitable embodiment of this application, further, in some embodiments, the corrosion performance test experimental dataset under the corrosion test condition includes at least a corrosion performance test experimental dataset under a salt spray corrosion test condition. Without limitation, the salt spray corrosion test condition may include at least one of the following salt spray conditions: one or more of NaCl aqueous solution salt spray condition, acetic acid salt spray condition, copper-accelerated acetic acid salt spray condition, and alternating salt spray corrosion condition. In some embodiments, the salt spray corrosion test condition includes a NaCl aqueous solution salt spray condition.

When the corrosion performance test experimental dataset includes at least a corrosion performance test experimental dataset under the salt spray corrosion test condition, the constructed corrosion resistance-mechanical property analysis model for the metallic material can be applied to metallic materials and structural components or products thereof in service environments such as road transportation, computer technology, electronic communication, and electrical appliances, as well as structural components or products thereof in related service environments such as electroplating, coating, packaging boxes, and transportation equipment. The road transportation field may involve, but is not limited to, electronic and electrical equipment for road vehicles, equipment and devices for rail transit locomotives, automotive components, and other equipment or metallic structural components thereof. The computer technology field may involve, but is not limited to, computers, displays, hosts, computer components, precision instruments such as medical equipment, and other equipment, products or metallic structural components thereof. The electronic communication field may involve, but is not limited to, mobile phones, radio frequency devices, electronic communication components, printed circuit boards (PCB), printed circuit board assemblies (PCBA), other equipment, products, or metallic structural components thereof. The electrical appliances may involve, but are not limited to, household electrical equipment such as household appliances, lighting fixtures, and transformers, instruments and meters, medical devices, and other devices, products, or metallic structural components thereof.

Without limitation, a model for obtaining corrosion performance test experimental datasets based on the NaCl aqueous solution salt spray condition can be applied to assess the quality and uniformity of protective coatings and to compare differences in salt spray corrosion resistance of samples with similar structures, but is not limited thereto. Without limitation, a model for obtaining corrosion performance test experimental datasets based on the acetic acid salt spray condition can be applied to harsh salt spray environments in coastal cities of southern regions, and the like. Without limitation, a model for obtaining corrosion performance test experimental datasets based on the copper-accelerated acetic acid salt spray condition can be applied to harsh salt spray environments. Without limitation, a model for obtaining corrosion performance test experimental datasets based on the alternating salt spray corrosion condition can be applied to high-temperature and high-humidity environments.

Based on any suitable embodiment of this application, further, in some embodiments, the NaCl aqueous solution salt spray condition includes the following parameter: a simulated salt spray condition with a 3wt% to 6wt% NaCl aqueous solution at 34°C-36°C. In some embodiments, the NaCl aqueous solution salt spray condition includes the following parameter: a simulated salt spray condition with a 5wt% NaCl aqueous solution at 35°C. In some embodiments, the NaCl aqueous solution salt spray condition includes the following parameter: a simulated salt spray condition with a 3.5wt% NaCl aqueous solution at 35°C.

For the NaCl aqueous solution salt spray condition, the above test conditions are conducive to assessing the corrosion resistance of battery casings, automotive components, and the like.

Without limitation, starting time when the sample is placed under the corrosion test condition can be used as a first sampling time point.

Without limitation, during corrosion test experiments, the sampling method for different equivalent corrosion time points may be as follows: total test duration (that is, duration of the preset period) is greater than or equal to 30 h, and an time interval between adjacent sampling points may be greater than or equal to 1 day. Without limitation, the following sampling method may be used: total test duration is 600 h, and sampling is performed once every 24 h; further, the first sampling point may be starting time when the sample is placed under the corrosion test condition. The following sampling method may also be used: total test duration is 30 days, and sampling is performed once every 10 days; further, the first sampling point may be starting time when the sample is placed under the corrosion test condition.

It can be understood that during corrosion test experiments, sampling at any time point may further optionally include, but not limited to, the following test analysis on the sample: one or more of macroscopic morphology observation, microscopic morphology observation, corrosion rate analysis, and the like. For example, one or more of the above test analyses can be conducted every 1 to 10 days. In some embodiments, macroscopic morphology observation, microscopic morphology observation, and corrosion rate analysis are conducted every 10 days.

Without limitation, the "macroscopic morphology observation" can be conducted using means such as a stereo microscope or shadowless lamp observation.

Without limitation, the "microscopic morphology observation" includes local morphology of the sample at different magnifications (for example, 200X-10000X times) and can be conducted using means such as a metallographic microscope or a scanning electron microscope (SEM). Without limitation, a CX40M metallographic microscope, a field emission scanning electron microscope FEI NOVA NanoSEM 230, or the like can be used.

Without limitation, for salt spray corrosion test experiments, sampling can be performed every 12 h to 36 h (for example, every 24 h) to obtain the corrosion parameter and the corresponding mechanical parameter, thereby obtaining the corrosion performance test experimental dataset and the mechanical property test experimental dataset. The test duration may be greater than or equal to 24 days, further greater than or equal to 25 days, and even further greater than or equal to 30 days.

Without limitation, the sample for salt spray corrosion tests may be a block of 12 mm × 12 mm × 6 mm. Without limitation, non-test surfaces are wrapped with a blue film. Before the salt spray corrosion test, the following pretreatment steps can be performed: grinding and polishing the test surface, cleaning with ethanol, and drying. Before the test, the sample is weighed, and the initial weight WO is recorded. Unless otherwise specified, the following test parameters can be used: total test duration is 600 h, and during the test, samples are taken and analyzed every 24 h. Macroscopic morphology observation and microscopic morphology observation can be performed first. Then corrosion products are washed from the sample surface, and the sample is weighed again. The weight obtained at the i-th sampling is recorded as Wi, and the test duration of the i-th sampling is recorded as Ti. The corrosion rate for the i-th sampling can be calculated by substituting W = Wi - WO and T = Ti into the formula (I). Unless otherwise specified, at least three parallel samples are set for each test. Without limitation, corrosion products on the sample surface can be washed using a chromic acid cleaner (20 g/L $Cr_2O_3$ + 50 mL/L $H_3PO_4$).

Based on any suitable embodiment of this application, further, in some embodiments, the establishing, based on the corrosion performance test experimental dataset, a first set of empirical relational expressions describing variation of the corrosion parameter with equivalent corrosion time includes:

for each type of corrosion parameter in the corrosion parameter, performing segmented fitting based on the corresponding corrosion performance test experimental dataset, and for each fitting interval, with the corresponding corrosion parameter as a dependent variable and the equivalent corrosion time as an independent variable, performing fitting in the form of a power function or a linear function to construct an empirical relational expression between each type of corrosion parameter and equivalent corrosion time for each fitting interval, thereby obtaining the first set of empirical relational expressions.

The first set of empirical relational expressions describing variation of the corrosion parameter with equivalent corrosion time can be obtained for each type of corrosion parameter through segmented fitting. The fitting for each segment can be performed using a power function or a linear function, resulting in a higher degree of fit between the fitting curves and the experimental test datasets, making the model more effective. However, the function types are not limited to those described above.

Based on any suitable embodiment of this application, further, in some embodiments, in the first set of empirical relational expressions, a fitting form of the power function is $yl = A \cdot x^B$, and a fitting form of the linear function is $yl = a + b \cdot x$; where x is the equivalent corrosion time, y1 is the corrosion parameter, A is a positive number, B is a negative number, b is a positive number, and a is a negative number.

Based on any suitable embodiment of this application, further, in some embodiments, A is a real number selected from 0.01 to 1.00, B is a real number selected from -0.3 to -0.8, b is a real number selected from 0.001 to 0.05, and a is a real number selected from 0.02 to -0.8. Further, non-limiting examples of A include 0.145, 0.755, 0.756, 0.125, 0.126, 0.134, and the like. Non-limiting examples of B include -0.533, -0.531, -0.532, -0.681, -0.680, -0.688, and the like. Alternatively, a may be a real number selected from the following ranges: -0.001 to -0.8, -0.001 to -0.7, -0.001 to -0.5, and the like. Non-limiting examples of a include -0.124, -0.0132, -0.00171, -0.366, -0.668, and the like. Alternatively, b may further be a real number selected from 0.001 to 0.02, and further may be a real number selected from 0.001 to 0.01. Non-limiting examples of b include 0.00713, 0.00334, 0.0197, 0.0311, 0.00222, and the like. Further, in some embodiments, fitting is performed for the corrosion rate, and further, the corrosion rate may be expressed by the corrosion depth per unit time.

Appropriate fitting function types are selected, which is conducive to obtaining a first set of empirical relational expressions with better fitting results, thereby obtaining a more effective corrosion resistance-mechanical property analysis model.

Depending on the corrosion parameters used for fitting, the factors such as A, B, a, and b in the above functions can be selected from different ranges. Any one of these factors can be selected from a range defined by any two example point values described in this application, provided that the range is suitable.

Based on any suitable embodiment of this application, further, in some embodiments, the corrosion parameter in the corrosion performance test experimental dataset includes at least the corrosion rate.

Based on any suitable embodiment of this application, further, in some embodiments, the corrosion performance test experimental dataset includes at least one of a corrosion performance test experimental dataset under an electrochemical corrosion test condition and a corrosion performance test experimental dataset under an immersion corrosion test condition. Without limitation, the corrosion parameter in the corrosion performance test experimental dataset under the electrochemical corrosion test condition may include at least one of a self-corrosion potential or a self-corrosion current. Without limitation, the corrosion parameter in the corrosion performance test experimental dataset under the immersion corrosion test condition may include at least one of a corrosion degree and a corrosion rate.

When the corrosion performance test experimental dataset includes a corrosion performance test experimental dataset under the electrochemical corrosion test condition, the corrosion resistance-mechanical property analysis model for the metallic material can be applied to the performance prediction and service life evaluation of the metallic material in an electrochemical environment, for example, but not limited to, the performance prediction and service life evaluation of battery casings, further including, but not limited to, the performance prediction and service life evaluation of fuel cell casings.

When the corrosion performance test experimental dataset includes a corrosion performance test experimental dataset under the immersion corrosion test condition, the corrosion resistance-mechanical property analysis model for the metallic material can be applied to the performance prediction and service life evaluation of the metallic material in an environment exposed to corrosive liquids.

When the corrosion performance test experimental dataset includes both a corrosion performance test experimental dataset under the electrochemical corrosion test condition and a corrosion performance test experimental dataset under the immersion corrosion test condition, the corrosion resistance-mechanical property analysis model for the metallic material can be applied to the performance prediction and service life evaluation of battery casings containing electrolytes, further including, but not limited to, the performance prediction and service life evaluation of fuel cell casings.

In some embodiments, the corrosion performance test experimental dataset under the corrosion test condition includes a corrosion performance test experimental dataset under an electrochemical corrosion test condition; further, the electrochemical corrosion test may include one or more of open circuit potential testing, alternating current impedance testing, potentiodynamic polarization testing, and the like; correspondingly, the corrosion parameter in the corrosion performance test experimental dataset may include one or more of open circuit potential, impedance, and the like.

The test equipment used for electrochemical corrosion testing may include an electrochemical workstation and a three-electrode system. A structure of the three-electrode system is shown in FIG. 24. Without limitation, different excitation signals can be applied to the three-electrode system through the electrochemical workstation, and corresponding response signals can be recorded to establish an electrochemical corrosion behavior of the test sample. In some non-limiting embodiments, in the three-electrode system, the test sample serves as a working electrode, a silver-silver chloride (Ag-AgCl) electrode serves as a reference electrode, and a platinum (Pt) electrode serves as an auxiliary electrode; an electrolyte solution used may be a 3.5wt% NaCl aqueous solution; and during the test, the three-electrode system is placed in a Faraday shield box.

Without limitation, the sample for electrochemical corrosion testing may be a plate-shaped sample of 10 mm $\times$ 10 mm $\times$ 2 mm. The test surface area may be 1 cm$^2$. Before testing, the sample is subjected to the following pretreatment steps: cold mounting with epoxy resin, grinding using abrasive paper, polishing, cleaning with ethanol, and drying.

Without limitation, the electrochemical corrosion test may include open circuit potential (OCP) measurement and alternating current impedance (EIS) testing performed after the OCP becomes stable for a period (for example, after the OCP becomes stable for 60 minutes). Further, the electrochemical impedance spectra of the sample at different alternating current frequencies are measured. During the test, a 10 mV alternating-current sine wave can be used as the excitation voltage, and the test frequency can be controlled within 0.01 Hz to 105 Hz. Typically, the experimental results of EIS can be fitted using an equivalent circuit, thereby analyzing parameters of the components in the equivalent circuit diagram.

Without limitation, in the electrochemical corrosion test steps, potentiodynamic polarization testing can be performed after the alternating current impedance testing. Further, the following test parameters may be used: starting from an OCP of 300 mV, scanning is performed at a scanning speed of 0.167 mV/s until the current exceeds 1 mA. Tafel fitting can be performed on the polarization characteristic data of the sample using ZSimpWin v3.40 software.

In some embodiments, the corrosion performance test experimental dataset under the corrosion test condition includes a corrosion performance test experimental dataset under an immersion corrosion test condition. Without limitation, a corrosion parameter in the corrosion performance test experimental dataset under the immersion corrosion test condition may include at least one of a corrosion degree and a corrosion rate.

Without limitation, during immersion corrosion testing, the following sampling method may be used: total test duration is 30 days, and sampling is performed once every 10 days; further, the first sampling point may be starting time when the sample is placed under the corrosion test condition. At each sampling point, the sample may be optionally subjected to, but not limited to, the following test analysis: one or more of macroscopic morphology observation, microscopic morphology observation, and corrosion rate analysis.

In some embodiments, the immersion corrosion test includes the following steps: with total test duration of 30 days, samples are taken and tested every 10 days, to analyze the macroscopic morphology, microscopic morphology, and corrosion rate of the samples.

Without limitation, the sample for immersion corrosion testing may be a block of 15 mm $\times$ 15 mm $\times$ 2 mm, or a block of 50 mm $\times$ 25 mm $\times$ 2 mm, or samples of other shapes required for testing.

Without limitation, composition of a corrosion solution used for immersing the sample can be determined based on the corrosion environment of the test sample. A 500 mL corrosion solution can be used. In some embodiments, the corrosion solution is a 3wt% to 6wt% NaCl aqueous solution, such as a 3wt%, 3.5wt%, 4wt%, 4.5wt%, 5wt%, 5.5wt%, or 6wt% NaCl aqueous solution.

Without limitation, during the immersion corrosion test, a ratio of the corrosion solution to the sample test area can be maintained at greater than 0.2 mL/mm$^2$.

Without limitation, before the immersion corrosion test, the following pretreatment steps can be performed: grinding and polishing the test surface, rinsing sequentially with deionized water and ethanol, drying, and weighing. After weighing, the sample can be stored in a desiccator for later use.

Without limitation, the immersion corrosion test includes: placing the sample into a container, adding 500 mL of corrosion solution; after sealing, placing the entire container into a constant-temperature water bath tank, with the temperature set to 25°C; taking samples every 10 days (that is, test durations are 10 days, 20 days, and 30 days), and observing the macroscopic morphology and microscopic morphology of the corroded sample surface; and then, removing corrosion products from the sample surface, and comparing the sample masses before and after corrosion to calculate the immersion corrosion rate according to formula (I). At least three parallel samples are set for each test.

In this application, unless otherwise specified, as a time unit, 1d refers to 1 day and 1h refers to 1 hour.

Based on any suitable embodiment of this application, further, in some embodiments, the establishing, based on the mechanical property test experimental dataset, a second set of empirical relational expressions describing variation of the mechanical parameter with equivalent corrosion time includes:

for each type of mechanical parameter in the mechanical parameter, performing segmented fitting based on the corresponding mechanical property test experimental dataset, and for each fitting interval, with the corresponding mechanical parameter as a dependent variable and the equivalent corrosion time as an independent variable, performing fitting in the form of a power function or a linear function to construct an empirical relational expression between each type of mechanical parameter and the equivalent corrosion time, thereby obtaining the second set of empirical relational expressions.

The second set of empirical relational expressions describing variation of the mechanical parameter with equivalent corrosion time can be obtained for each type of mechanical parameter through segmented fitting. The fitting for each segment can be performed using a power function or a linear function, resulting in a higher degree of fit between the fitting curves and the experimental test datasets, making the model more effective. However, the function types are not limited to those described above.

Based on any suitable embodiment of this application, further, in some embodiments, in the second set of empirical relational expressions, a fitting form of the power function is $y2 = M \cdot x^N$, and a fitting form of the linear function is $y2 = m + n \cdot x$; where x is the equivalent corrosion time, y2 is the mechanical parameter, M is a positive number, N is a negative number, n is a negative number, and m is a positive number.

Based on any suitable embodiment of this application, further, in some embodiments, M is a real number selected from 1 to 250; N is a real number selected from -0.01 to -1; n is a real number selected from -0.05 to -5, and further may be a real number selected from -0.1 to -1.5; and m is a real number selected from 1 to 300. Non-limiting examples of M include 233.54, 152.16, 1.60, and the like. N may alternatively be a real number selected from -0.01 to -0.5, and further may be a real time selected from -0.01 to -0.2. Non-limiting examples of N include -0.11, -0.07, -0.16, and the like. Non-limiting examples of m include 269.59, 203.21, 9.69, 248.48, 134.55, 3.95, 248.21, 131.83, 8.31, and the like. Non-limiting examples of n include -1.44, -1.06, -0.15, -1.91, -0.40, -0.08, -1.61, -0.73, -0.19, and the like. Alternatively, n may be a real number selected from ranges such as -0.05 to -4, -0.05 to -3, -0.05 to -2, -1 to -2, -0.5 to -1.5, and -0.05 to -0.5.

Appropriate fitting function types are selected, which is conducive to obtaining the second set of empirical relational expressions with better fitting results, thereby helping to obtain a more effective corrosion resistance-mechanical property analysis model.

Depending on the mechanical parameters used for fitting, the factors such as M, N, m, and n in the above functions can be selected from different ranges. Any one of these factors can be selected from a range defined by any two example point values described in this application, provided that the range is suitable. For example, during fitting for tensile strength at break (UTS), n may be selected from any suitable range: -0.05 to -5, -0.1 to -5, -0.2 to -5, -0.05 to -4, -0.1 to -4, -0.2 to -4, -0.05 to -3, -0.1 to -3, -0.2 to -3, -0.3 to -3, and the like; and m may be selected from any suitable range: 60-300, 80-300, 60-250, 80-250, 100-300, 100-250, 120-240, and the like. For example, during fitting for elongation at break (EI), n may be selected from any suitable range: 0.05 to -0.5, -0.05 to -0.4, -0.05 to -0.3, -0.05 to -0.2, -0.06 to -0.2, and the like; m may be selected from any suitable range: 0.1-50, 0.1-30, 0.1-20, 0.1-10, 0.5-50, 0.5-30, 0.5-20, 0.5-10, 1-50, 1-30, 1-20, 1-10, and the like. For example, during fitting for yield strength (YS), n may be selected from any suitable range: -0.05 to -5, -0.1 to -5, -0.2 to -5, -0.5 to -5, -0.05 to -4, -0.1 to -4, -0.2 to -4, -0.5 to -4, -0.05 to -3, -0.1 to -3, -0.2 to -3, -0.3 to -3, -0.5 to -3, -0.8 to -2.5, -0.8 to -2.4, -0.8 to -2.0, and the like; and m may be selected from any suitable range: 50-300, 80-300, 50-250, 80-250, 100-300, 100-250, 120-250, and the like.

Based on any suitable embodiment of this application, further, in some embodiments, the method for constructing a corrosion resistance-mechanical property analysis model for a metallic material further includes the following step: establishing a third set of empirical relational expressions between equivalent corrosion time under the corrosion test condition and service time in the target service environment.

The empirical relational expressions (which may be referred to as the third set of empirical relational expressions) between the equivalent corrosion time under the corrosion test condition and the service time in the target service environment are established, to convert the equivalent corrosion time into the service time in the target service environment, thereby implementing more direct prediction of the service life of the metallic material and the structural components or products thereof.

Without limitation, the third set of empirical relational expressions between the equivalent corrosion time under the selected corrosion test condition and the service time in the target service environment can be established based on existing standards, specifications, or conventional experience in the art. For example, 1 day (d) of the die-cast aluminum alloy parts in a salt spray corrosion environment is equivalent to more than one year (that is, greater than or equal to 1 year) of corrosion in the actual service environment.

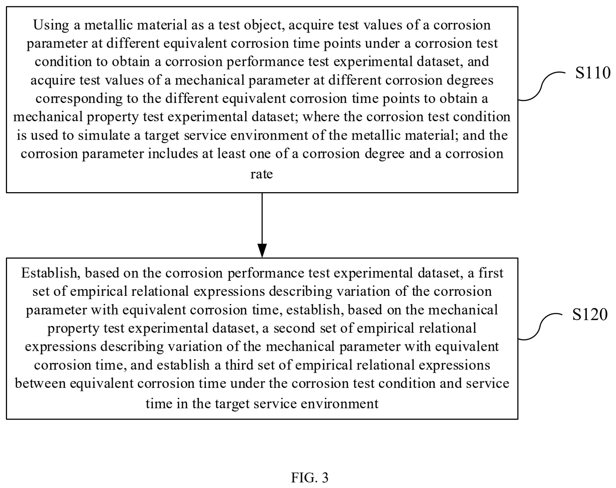

In some embodiments, a method for constructing a corrosion resistance-mechanical property analysis model for a metallic material is provided, including the following steps (as shown in FIG. 3):

S110: using a metallic material as a test object, acquiring test values of a corrosion parameter at different equivalent corrosion time points under a corrosion test condition to obtain a corrosion performance test experimental dataset, and acquiring test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate; and

S120: establishing, based on the corrosion performance test experimental dataset, a first set of empirical relational expressions describing variation of the corrosion parameter with equivalent corrosion time, establishing, based on the mechanical property test experimental dataset, a second set of empirical relational expressions describing variation of the mechanical parameter with equivalent corrosion time, and establishing a third set of empirical relational expressions between equivalent corrosion time under the corrosion test condition and service time in the target service environment.

Based on any suitable embodiment of this application, further, in some embodiments, the method for constructing a corrosion resistance-mechanical property analysis model for a metallic material includes the following step: establishing a fourth set of empirical relational expressions between a corrosion rate under the corrosion test condition and equivalent corrosion time. In this case, the first set of empirical relational expressions includes the fourth set of empirical relational expressions. In this case, the corrosion parameter in the corrosion performance test experimental dataset includes the corrosion rate.

The empirical relational expressions (which may be referred to as the fourth set of empirical relational expressions) between the corrosion rate and the equivalent corrosion time under the corrosion test condition are established, to dynamically analyze a correlation between the corrosion behavior and mechanical property of the metallic material in the target service environment.

The corrosion rate and corresponding equivalent corrosion time data points obtained from test analysis can be subjected to fitting analysis.

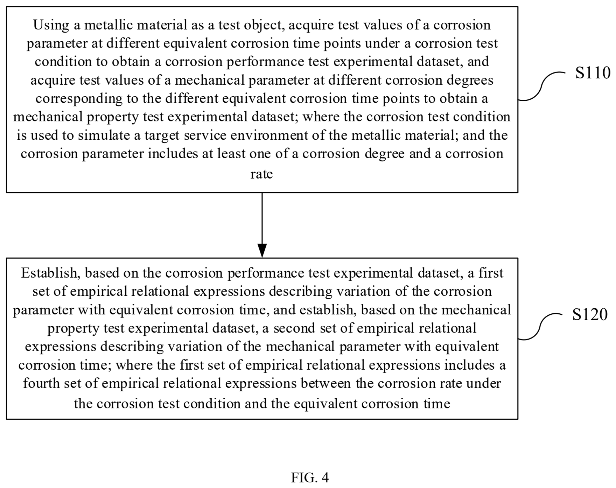

In some embodiments, a method for constructing a corrosion resistance-mechanical property analysis model for a metallic material is provided, including the following steps (as shown in FIG. 4):

S110: using a metallic material as a test object, acquiring test values of a corrosion parameter at different equivalent corrosion time points under a corrosion test condition to obtain a corrosion performance test experimental dataset, and acquiring test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate; and

S120: establishing, based on the corrosion performance test experimental dataset, a first set of empirical relational expressions describing variation of the corrosion parameter with equivalent corrosion time, and establishing, based on the mechanical property test experimental dataset, a second set of empirical relational expressions describing variation of the mechanical parameter with equivalent corrosion time; where the first set of empirical relational expressions includes a fourth set of empirical relational expressions between the corrosion rate under the corrosion test condition and the equivalent corrosion time.

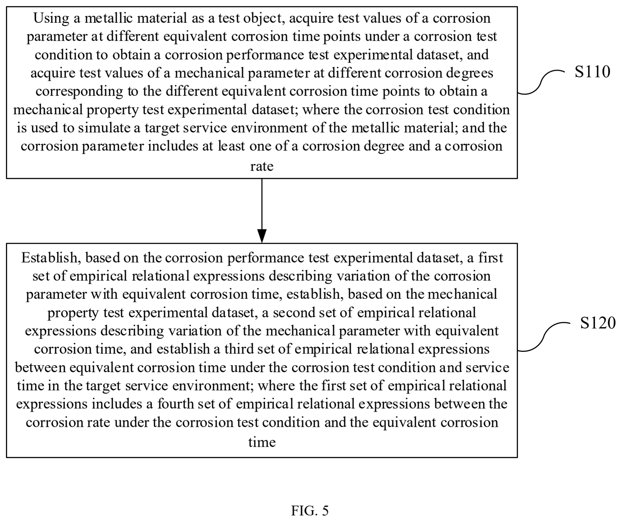

In some embodiments, a method for constructing a corrosion resistance-mechanical property analysis model for a metallic material is provided, including the following steps (as shown in FIG. 5):

S110: using a metallic material as a test object, acquiring test values of a corrosion parameter at different equivalent corrosion time points under a corrosion test condition to obtain a corrosion performance test experimental dataset, and acquiring test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate; and

S120: establishing, based on the corrosion performance test experimental dataset, a first set of empirical relational expressions describing variation of the corrosion parameter with equivalent corrosion time, establishing, based on the mechanical property test experimental dataset, a second set of empirical relational expressions describing variation of the mechanical parameter with equivalent corrosion time, and establishing a third set of empirical relational expressions between equivalent corrosion time under the corrosion test condition and service time in the target service environment; where the first set of empirical relational expressions includes a fourth set of empirical relational expressions between the corrosion rate under the corrosion test condition and the equivalent corrosion time.

In the embodiments shown in FIG. 3, FIG. 4, and FIG. 5, independently, for the mechanical parameter in the mechanical property test experimental dataset, reference may be made to the definitions above. Further, the mechanical parameter may include at least one of a tensile strength at break and an elongation at break. In some of these embodiments, the mechanical parameter includes a tensile strength at break and an elongation at break. The mechanical parameter may include a yield strength. In some of these embodiments, the mechanical parameter includes a yield strength. In some embodiments, the mechanical parameter includes a tensile strength at break, an elongation at break, and a yield strength.

According to a second aspect of this application, a method for analyzing service life of a metallic material is provided, which analyzes the service life of the metallic material using a corrosion resistance-mechanical property analysis model for the metallic material.

The metallic material may be as defined in the first aspect of this application.

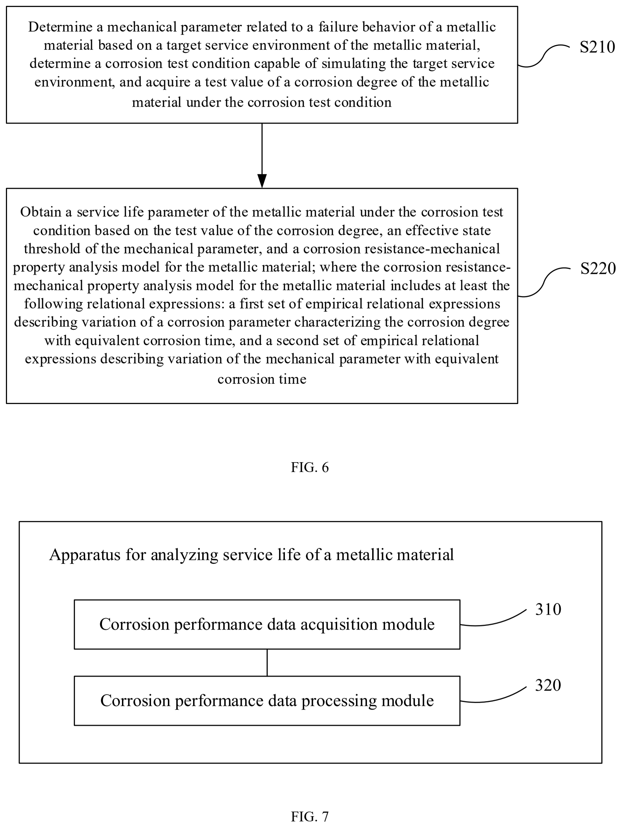

In some embodiments, a method for analyzing service life of a metallic material is provided, including the following steps (as shown in FIG. 6):

S210: determining a mechanical parameter related to a failure behavior of the metallic material based on a target service environment of the metallic material, determining a corrosion test condition capable of simulating the target service environment, and acquiring a test value of a corrosion degree of the metallic material under the corrosion test condition; and

S220: obtaining a service life parameter of the metallic material under the corrosion test condition based on the test value of the corrosion degree, an effective state threshold of the mechanical parameter, and a corrosion resistance-mechanical property analysis model for the metallic material; where the corrosion resistance-mechanical property analysis model for the metallic material includes at least the following relational expressions: a first set of empirical relational expressions describing variation of a corrosion parameter characterizing the corrosion degree with equivalent corrosion time, and a second set of empirical relational expressions describing variation of the mechanical parameter with equivalent corrosion time.

According to the method for analyzing service life of a metallic material, the mechanical parameter related to the failure behavior of the metallic material is selected based on the target service environment of the metallic material, the corrosion test condition capable of simulating the target service environment is selected, and then the test value of the corrosion degree of the metallic material under the selected corrosion test condition is obtained, thereby obtaining the service life parameter of the metallic material under the selected corrosion test condition based on the test value of the corrosion degree, the effective state threshold of the selected mechanical parameter, and the corrosion resistance-mechanical property analysis model for the metallic material. Using the equivalent corrosion time and the mechanical properties at different corrosion degrees as a link, the corrosion resistance-mechanical property analysis model for the metallic material includes at least the following two relational expressions: a first set of empirical relational expressions describing variation of at least one corrosion parameter of the corrosion degree and the corrosion rate with equivalent corrosion time, and a second set of empirical relational expressions describing variation of the mechanical parameter with equivalent corrosion time. This analysis method can effectively and accurately analyze the service life of the metallic material in the target service environment.

Based on any suitable embodiment of this application, further, in some embodiments, the service life parameter includes at least the equivalent corrosion time.

According to the analysis method, at least the equivalent corrosion time parameter of the metallic material can be obtained, reflecting the service life status of the metallic material.

Based on any suitable embodiment of this application, further, in some embodiments, the obtaining a service life parameter of the metallic material under the corrosion test condition based on the test value of the corrosion degree, an effective state threshold of the mechanical parameter, and a corrosion resistance-mechanical property analysis model for the metallic material includes:

obtaining remaining service time of the metallic material based on the test value of the corrosion degree, the effective state threshold of the mechanical parameter, the corrosion resistance-mechanical property analysis model for the metallic material, and a third set of empirical relational expressions between the equivalent corrosion time under the corrosion test condition and the service time in the target service environment.

The remaining service time of the metallic material can be obtained based on the test value of the corrosion degree of the metallic material, the effective state threshold of the mechanical parameter related to the failure behavior of the metallic material, the corrosion resistance-mechanical property analysis model for the metallic material, and the empirical relational expression between the equivalent corrosion time under the selected corrosion test condition and the service time in the target service environment, implementing predictive analysis of the remaining service life of the metallic material.

Based on any suitable embodiment of this application, further, in some embodiments, the mechanical parameters include at least two types. Without limitation, the mechanical parameters can be sorted in descending order by degrees of response to failure of the metallic material, and the corrosion resistance-mechanical property analysis model for the metallic material is obtained based on the mechanical parameter ranked first.

The mechanical parameters are sorted in descending order by degrees of response to the failure of the metallic material, and the corrosion resistance-mechanical property analysis model for the metallic material is obtained based on the mechanical parameter ranked first, more effectively reflecting the correlation between the corrosion behavior and mechanical property failure of the metallic material in the target service environment.

Based on any suitable embodiment of this application, further, in some embodiments, the corrosion resistance-mechanical property analysis model for the metallic material is constructed according to the method for constructing a corrosion resistance-mechanical property analysis model for a metallic material provided in the first aspect of this application.

The corrosion resistance-mechanical property analysis model involved in the method for analyzing service life of a metallic material can be constructed using the construction method described in the first aspect of this application, thereby establishing the relational expression between the corrosion degree of the metallic material and the mechanical property including at least one mechanical parameter of the tensile strength at break and the elongation at break.

According to a third aspect of this application, an apparatus for analyzing service life of a metallic material is provided, including: a corrosion performance data acquisition module 310 and a corrosion performance data processing module 320, as shown in FIG. 7.

The metallic material may be as defined in the first aspect of this application.

In some embodiments, an apparatus for analyzing service life of a metallic material is provided, including:

a corrosion performance data acquisition module 310 configured to determine a mechanical parameter related to a failure behavior of the metallic material based on a target service environment of the metallic material, determine a corrosion test condition capable of simulating the target service environment, and acquire a test value of a corrosion degree of the metallic material under a corrosion test condition; and

a corrosion performance data processing module 320 configured to obtain a service life parameter of the metallic material under the corrosion test condition based on the test value of the corrosion degree, an effective state threshold of the mechanical parameter, and a corrosion resistance-mechanical property analysis model for the metallic material; where the corrosion resistance-mechanical property analysis model for the metallic material is as defined in the first or second aspect of this application.

The apparatus for analyzing service life of a metallic material provided in the third aspect of this application can be used to implement the method for analyzing service life of a metallic material provided in the second aspect of this application.

According to a fourth aspect of this application, a method for analyzing a mechanical property of a metallic material is provided, to analyze mechanical properties of the metallic material using the corrosion resistance-mechanical property analysis model for the metallic material.

The metallic material may be as defined in the first aspect of this application.

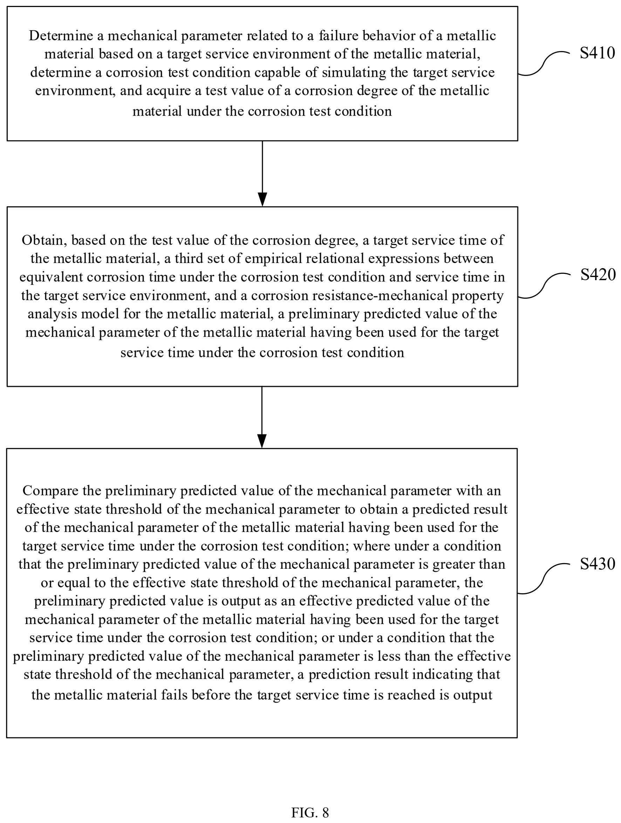

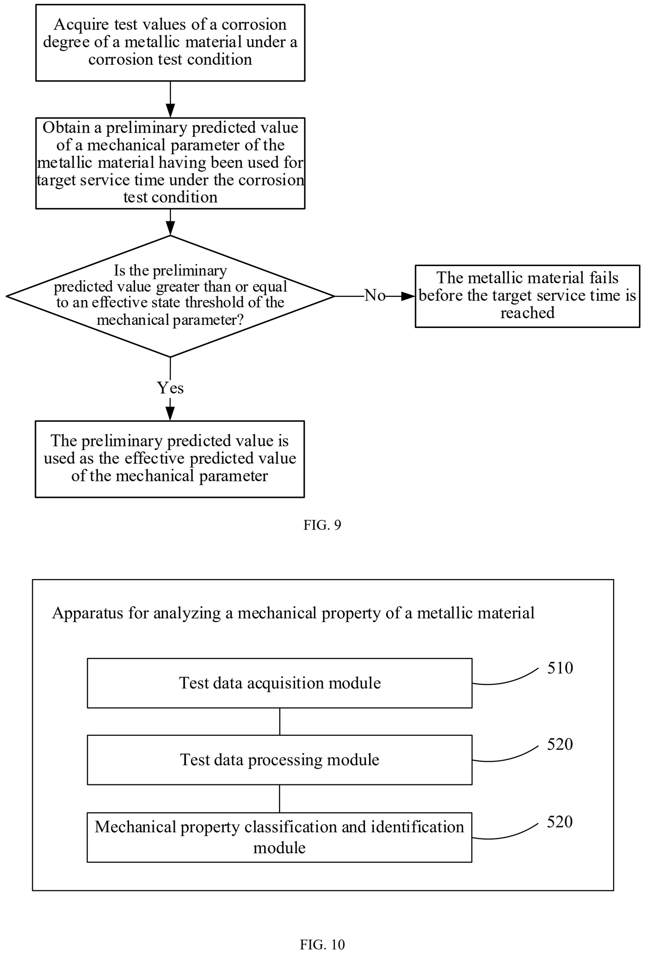

In some embodiments, a method for analyzing a mechanical property of a metallic material is provided, including the following steps (as shown in FIG. 8 and FIG. 9):

determining mechanical parameters related to a failure behavior of the metallic material based on a target service environment of the metallic material, determining a corrosion test condition capable of simulating the target service environment, and acquiring a test value of a corrosion degree of the metallic material under the corrosion test condition;

obtaining, based on the test value of the corrosion degree, a target service time of the metallic material, a third set of empirical relational expressions between equivalent corrosion time under the corrosion test condition and service time in the target service environment, and a corrosion resistance-mechanical property analysis model for the metallic material, a preliminary predicted value of the mechanical parameter of the metallic material having been used for the target service time under the corrosion test condition; where the corrosion resistance-mechanical property analysis model for the metallic material is as defined in the first or second aspect of this application; and

comparing the preliminary predicted value of the mechanical parameter with an effective state threshold of the mechanical parameter to obtain a predicted result of the mechanical parameter of the metallic material having been used for the target service time under the corrosion test condition; where under a condition that the preliminary predicted value of the mechanical parameter is greater than or equal to the effective state threshold of the mechanical parameter, the preliminary predicted value is output as an effective predicted value of the mechanical parameter of the metallic material having been used for the target service time under the corrosion test condition; or under a condition that the preliminary predicted value of the mechanical parameter is less than the effective state threshold of the mechanical parameter, a prediction result indicating that the metallic material fails before the target service time is reached is output.

According to the method for analyzing a mechanical property of a metallic material, the mechanical parameter related to the failure behavior of the metallic material is selected based on the target service environment of the metallic material, the corrosion test condition capable of simulating the target service environment is selected, and then test value of the corrosion degree of the metallic material under the selected corrosion test condition is acquired, thereby obtaining, based on the test value of the corrosion degree, the target service time of the metallic material, the empirical relational expression between the equivalent corrosion time under the selected corrosion test condition and the service time in the target service environment, and the corrosion resistance-mechanical property analysis model for the metallic material, the preliminary predicted value of the mechanical parameter of the metallic material having been used for the target service time under the selected corrosion test condition. The preliminary predicted value of the mechanical parameter is compared with the effective state threshold of the mechanical parameter, to obtain the predicted result of the mechanical parameter of the metallic material having been used for the target service time under the selected corrosion test condition. Under a condition that the preliminary predicted value of the mechanical parameter is greater than or equal to the effective state threshold of the mechanical parameter, the effective predicted value of the mechanical parameter of the metallic material having been used for the target service time under the selected corrosion test condition is equal to the preliminary predicted value. Under a condition that the preliminary predicted value of the mechanical parameter is less than the effective state threshold of the mechanical parameter, it indicates that the metallic material fails before the target service is reached.

According to a fifth aspect of this application, an apparatus for analyzing a mechanical property of a metallic material is provided (as shown in FIG. 10), including: a test data acquisition module 510, a test data processing module 520, and a mechanical property classification and identification module 530.

The metallic material may be as defined in the first aspect of this application.

In some embodiments, an apparatus for analyzing a mechanical property of a metallic material is provided, including:

a test data acquisition module 510 configured to determine a mechanical parameter related to a failure behavior of the metallic material based on a target service environment of the metallic material, determine a corrosion test condition capable of simulating the target service environment, and acquire a test value of a corrosion degree of the metallic material under the corrosion test condition;

a test data processing module 520 configured to obtain, based on the test value of the corrosion degree, a target service time of the metallic material, a third set of empirical relational expressions between equivalent corrosion time under the corrosion test condition and service time in the target service environment, and a corrosion resistance-mechanical property analysis model for the metallic material, a preliminary predicted value of the mechanical parameter of the metallic material having been used for the target service time under the corrosion test condition; where the corrosion resistance-mechanical property analysis model for the metallic material is as defined in the first or second aspect of this application; and

a mechanical property classification and identification module 530 configured to compare the preliminary predicted value of the mechanical parameter with an effective state threshold of the mechanical parameter to obtain a predicted result of the mechanical parameter of the metallic material having been used for the target service time under the corrosion test condition; where under a condition that the preliminary predicted value of the mechanical parameter is greater than or equal to the effective state threshold of the mechanical parameter, an effective predicted value of the mechanical parameter of the metallic material having been used for the target service time under the corrosion test condition is equal to the preliminary predicted value.

The apparatus for analyzing a mechanical property of a metallic material provided in the fifth aspect of this application can be used to implement the method for analyzing a mechanical property of a metallic material provided in the fourth aspect of this application.

According to a sixth aspect of this application, a computer device is provided.

In some embodiments, a computer device is provided, including a memory and a processor, where the memory stores a computer program, and when the processor executes the computer program, the steps of the method for constructing a corrosion resistance-mechanical property analysis model for a metallic material provided in the first aspect of this application, or the method for analyzing service life of a metallic material provided in the second aspect, or the method for analyzing a mechanical property of a metallic material provided in the fourth aspect are implemented.

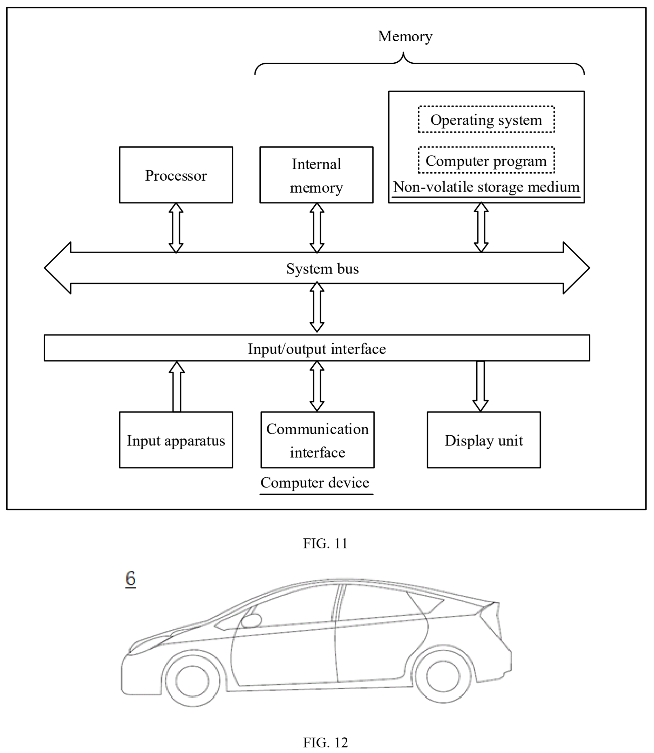

The computer device may be a terminal, and its internal structure is shown in FIG. 11. The computer device includes a processor, a memory, a communication interface, a display screen, and an input device connected via a system bus. The processor of the computer device is configured to provide computing and control capabilities. The memory of the computer device includes a non-volatile storage medium and an internal memory. The non-volatile storage medium stores an operating system and a computer program. The internal memory provides an environment for the operation of the operating system and the computer program in the non-volatile storage medium. The communication interface of the computer device is configured for wired or wireless communication with an external terminal, where wireless communication can be achieved through Wi-Fi, mobile cellular networks, NFC (near field communication), or other technologies. The computer program, when executed by the processor, implements a method for determining battery performance in an energy storage system. The display screen of the computer device may be a liquid crystal display or an electronic ink display, and the input device of the computer device may be a touch layer covering the display screen, or may be a button, a trackball, or a touchpad provided on the computer device housing, or may be an external keyboard, a touchpad, a mouse, or the like.

Those skilled in the art can understand that the structure shown in FIG. 11 is merely a block diagram of a portion of the structure related to the solution of this application and does not constitute a limitation on the computer device to which the solution of this application is applied. A specific computer device may include more or fewer components than shown in the figure, or combine certain components, or have a different arrangement of components.

According to a seventh aspect of this application, a computer-readable storage medium is provided, where a computer program is stored thereon, and when the computer program is executed by a processor, the steps of the method for constructing a corrosion resistance-mechanical property analysis model for a metallic material provided in the first aspect of this application, or the method for analyzing service life of a metallic material provided in the second aspect, or the method for analyzing a mechanical property of a metallic material provided in the fourth aspect are implemented.

According to another aspect of this application, a computer program product is provided, including a computer program, characterized in that when the computer program is executed by a processor, the steps of the method for constructing a corrosion resistance-mechanical property analysis model for a metallic material provided in the first aspect of this application, or the method for analyzing service life of a metallic material provided in the second aspect, or the method for analyzing a mechanical property of a metallic material provided in the fourth aspect are implemented.

Those skilled in the art can understand that all or part of the processes in the above method embodiments can be implemented by instructing relevant hardware through a computer program, which can be stored in a non-volatile computer-readable storage medium. When executed, the computer program may include the processes of the embodiments of the methods described above. Any reference to memory, databases, or other media used in the embodiments provided in this application may include at least one of non-volatile and volatile memory. The non-volatile memory may include a read-only memory (Read-Only Memory, ROM), a magnetic tape, a floppy disk, a flash memory, an optical memory, a high-density embedded non-volatile memory, a resistive random access memory (ReRAM), a magnetoresistive random access memory (Magnetoresistive Random Access Memory, MRAM), a ferroelectric random access memory (Ferroelectric Random Access Memory, FRAM), a phase change memory (Phase Change Memory, PCM), a graphene memory, and the like. The volatile memory may include a random access memory (RAM), an external cache memory, or the like. By way of illustration other than limitation, the RAM may be of various forms, such as a static random access memory (Static Random Access Memory, SRAM) or a dynamic random access memory (Dynamic Random Access Memory, DRAM). The database involved in the embodiments provided in this application may include at least one of a relational database or a non-relational database. The non-relational databases may include a blockchain-based distributed database, and the like, but is not limited thereto. The processor involved in the embodiments provided in this application may be a general-purpose processor, a central processing unit, a graphics processing unit, a digital signal processor, a programmable logic device, a quantum computing-based data processing logic device, or the like, but is not limited thereto.

According to an eighth aspect of this application, an electric apparatus including a battery system is provided.

In some embodiments, the electric apparatus includes:

a battery system, including a battery casing and a battery cell located inside the battery casing, where the battery casing includes the metallic material according to the first aspect of this application; and

at least one of the apparatus for analyzing service life of a metallic material according to the third aspect of this application, the apparatus for analyzing a mechanical property of a metallic material according to the fifth aspect, the computer device according to the sixth aspect, and the computer-readable storage medium according to the seventh aspect.

In this application, unless otherwise specified, the "battery cell" refers to a basic unit capable of implementing mutual conversion between chemical energy and electrical energy.

In some embodiments, the battery cell includes a fuel cell. In this case, the corresponding battery system may be a fuel cell system, and the corresponding battery casing may be a fuel cell casing.

In this application, unless otherwise specified, the "fuel cell" has the well-known meaning in the art, and refers to a chemical device that directly converts the chemical energy of fuel into electrical energy, and the "fuel cell unit" refers to a battery cell that converts the chemical energy of fuel into electrical energy. In this application, unless otherwise specified, the "fuel cell casing" refers to a battery casing containing fuel cell-type battery cells.

In some embodiments, the electric apparatus is a fuel-powered electric apparatus, including: a fuel cell system including a fuel cell casing and a fuel cell unit located inside the fuel cell casing, where the fuel cell casing includes the metallic material as defined in the first aspect of this application.

In some embodiments, the battery cell includes a lithium battery cell. In this case, the corresponding battery system may be a lithium battery system, and the corresponding battery casing may be a lithium battery casing.

In this application, unless otherwise specified, the "lithium battery" has the well-known meaning in the art, and refers to a type of battery where active ions include lithium ions, and the "lithium battery cell" refers to a battery cell where active ions include lithium ions. The lithium battery may be a lithium-ion secondary battery. In this application, unless otherwise specified, the "lithium battery casing" refers to a battery casing containing lithium battery cells.

In some embodiments, the electric apparatus includes: a lithium battery system, including a lithium battery casing and a lithium battery cell located inside the lithium battery casing, where the lithium battery casing includes the metallic material as defined in the first aspect of this application.

The electric apparatus may include a mobile device (such as a mobile phone or a laptop computer), an electric vehicle (such as a battery electric vehicle, a hybrid electric vehicle, a plug-in hybrid electric vehicle, an electric bicycle, an electric scooter, an electric golf cart, or an electric truck), an electric train, a ship, a satellite, an energy storage system, or the like, but is not limited thereto.

For the electric apparatus, a battery can be selected based on its usage requirements.

FIG. 12 is an example of an electric apparatus. The electric apparatus is a battery electric vehicle, a hybrid electric vehicle, a plug-in hybrid electric vehicle, or the like.

Another example of the apparatus may be a mobile phone, a tablet computer, a laptop computer, or the like. The apparatus typically requires to be light and thin and may use a secondary battery as a power source.

At least one of the apparatus for analyzing service life of a metallic material, the apparatus for analyzing a mechanical property of a metallic material, the computer device, the computer-readable storage medium, and the computer program product can be provided in the electric apparatus to implement the method for constructing the analysis model, the method for analyzing service life of a metallic material, or the method for analyzing a mechanical property of a metallic material, facilitating better management and maintenance of the electric apparatus. This not only facilitates safety maintenance of the battery casing in the electric apparatus, but also facilitates design of battery casings with longer service time.

Hereinafter, some examples of this application are described. The examples described below are exemplary and are used only to explain this application and should not be construed as limiting this application. For examples where technical solutions or conditions are not specified, they are carried out in accordance with the descriptions above, or in accordance with the technical solutions or conditions described in the literature in the art, or in accordance with product specifications. Reagents or instruments used without specifying the manufacturer are conventional products that can be obtained commercially or prepared in a conventional manner from commercially available products.

In the following examples, room temperature refers to 20°C to 30°C.

I. Test samples:

Fuel cell casings made of four aluminum alloy materials, A356.2, A380, AlSi10MgMn, and AlSi9MnMoZr, were cut into specific dimensions and used as test samples for corrosion and tensile tests. The elemental composition of the four aluminum alloy materials are shown in Table 1.

Each type of aluminum alloy can be prepared according to the elemental composition shown in Table 1 using the following method: In a smelting furnace, pure aluminum ingots were first added, then other constituent elements were added in batches based on the differences in their melting points, with subsequent raw materials added after the previously added raw materials had melted. After melting, the melt was left standing and slag was removed, a refining agent was added, and after further slag removal, some constituent elements were added, followed by casting in a mold to obtain an ingot of the target dimensions. The ingot was held at a constant temperature and then water-cooled to room temperature to obtain the aluminum alloy material of the target dimensions for battery casings, also referred to as die-cast aluminum alloy samples.

**Table 1. Elemental composition of four aluminum alloy materials (unit: weight percentage wt%)**

| Alloy type | Si | Cu | Mg | Fe | Mn | Zn | Ti | Other elements | Al |
|---|---|---|---|---|---|---|---|---|---|
| A356.2 | 6.5-7.5 | ≤0.1 | 0.30-0.45 | ≤0.12 | ≤0.05 | ≤0.05 | ≤0.2 | - | Bal. |
| A380 | 7.5-9.5 | 3.0-4.0 | ≤0.1 | ≤2.0 | ≤0.5 | ≤3.0 | - | Sn: ≤0.35 | Bal. |
| AlSi10MgMn | 8.5-10.5 | <0.05 | 0.17-0.90 | 0.2-0.3 | 0.20-0.50 | - | - | Cr: 0.1-0.2 | Bal. |
| AlSi9MnMoZr | 8.5-10.5 | 0.05 | 0.06 | ≤0.6 | 0.35-0.60 | ≤0.07 | ≤0.15 | Zr: ≤0.3 | Bal. |

In Table 1, "-" indicates not detected, and based on conventional compositions, it can be considered as not present; "Bal." indicates the base element.

II. Construction of a corrosion resistance-mechanical property analysis model based on salt spray corrosion test

1. Salt spray corrosion test method

A 3.5wt% NaCl aqueous solution was used, tested at 35°C.

In the total test duration of 600 hours, the macroscopic morphology, microscopic morphology, corrosion mass loss, and corrosion rate of the battery casing material were analyzed every 24 hours.

Before the test, the fuel cell casings of known alloy types were cut into blocks of 15 mm × 15 mm × 2 mm, with non-test surfaces wrapped with a blue film. The test surface was ground, polished, and dried, and then the sample was weighed, with the weight recorded as WO. The total test duration was 600 hours, and during the test, a batch of samples was taken every 24 hours for observation of the macroscopic morphology and microscopic morphology of the sample surface. After observation, corrosion products on the sample surface were removed using a chromic acid cleaner (20 g/L $Cr_2O_3$ + 50 mL/L $H_3PO_4$), and the samples were weighed again to calculate the corrosion rate and perform morphology observation. The test duration of the i-th sampling was recorded as Ti, and the weight of the i-th sampling was recorded as Wi. At least three parallel samples were set for each test.

2. Test methods

(1) Macroscopic morphology test method

Samples: Sampling time points are as described above.

Instrument: Shadowless lamp.

(2) Microscopic morphology test method

Samples: Sampling time points are as described above.

Instrument: NOVA NanoSEM 230 low-vacuum ultra-high-resolution field emission electron microscope.

Method: Probe type (det) is ETD. For example, in FIG. 13, the accelerating voltage (HV) is 5 kV, and the working distance is 30 mm.

(3) Corrosion mass loss and corrosion rate analysis method

The mass loss of the i-th sampling is W = WO - Wi.

The corrosion rate Ri of the i-th sampling can be calculated using formula (I):

$$\text{Corrosion rate} = (K \times W) / (A \times Ti \times D) \text{ mm/y} \qquad (I)$$

In formula (I): K = 8.64 × 10$^4$, and K is a time constant;

W is a mass difference before and after the test, equal to WO - Wi, in mg;
A is a test surface area, in cm$^2$, which is 1.5 cm × 1.5 cm = 2.25 cm$^2$ herein;
Ti is test duration of the i-th sampling, in h; and
D = 2.7 g/cm$^3$, D is a density.
"mm/y" indicates millimeters per year.

(4) Establishment the first set of empirical relational expressions describing variation of the corrosion parameter with the equivalent corrosion time

The total test duration was divided into three time periods, and fitting was performed using the following functions, to select the function with better fitting results.

$$\text{Optional function 1: } y1 = A \cdot x^B;$$

$$\text{Optional function 2: } y1 = a + b \cdot x;$$

where x is the equivalent corrosion time, y1 is the corrosion parameter, A is a positive number, B is a negative number, b is a positive number, and a is a real number.

The fitting effect can be evaluated using at least one of Reduced Chi-Sqr, $R^2$, and the adjusted $R^2$. Reduced Chi-Sqr is equivalent to the residual mean square RSS/dof in Anova, where a value closer to 1 indicates a better fitting effect, mainly used in nonlinear fitting. $R^2$ is a coefficient of determination (COD), where a value closer to 1 indicates a better fitting result. The "adjusted $R^2$" is obtained from SPSSAU linear regression, where a value closer to 1 indicates a better fitting result.

3. Analysis of salt spray corrosion test results

FIG. 13 shows the macroscopic morphology (first row) and microscopic morphology of four aluminum alloy materials at different equivalent corrosion time points in the salt spray test: (a) A356.2; (b) A380; (c) AlSi10MgMn; and (d) AlSi9MnMoZr. Based on the surface macroscopic morphology of the four die-cast aluminum alloy samples at different corrosion times, it can be found that among the four aluminum alloy materials, A380 alloy exhibits the most severe corrosion, with a significant accumulation of white corrosion products, while the other three die-cast aluminum alloys corrode relatively slowly with fewer surface corrosion products, consistent with the electrochemical test results. For A356.2 and A380 alloys, at shorter corrosion time (1d to 6d), the alloy surface is quickly covered with corrosion products, presumably due to uniform corrosion. The stacking area and thickness of surface corrosion products increase with prolonged corrosion time, and the sample surface transitions from an initially corroded dark color to being covered with a large amount of white corrosion products. For AlSi10MgMn and AlSi9MnMoZr alloy materials, in the early corrosion stage (1d to 6d), the sample surface mainly shows localized pitting corrosion, with corrosion products accumulating more near the pitting sites, presumably due to the protection of a passivation film. In the middle corrosion stage (7d to 18d), corrosion further expands, and the passivation film is destroyed, gradually showing uniform corrosion. In the late corrosion stage (19d to 24d), more and more areas of the sample surface are covered with corrosion products until the entire surface is covered.

The microscopic morphology of the four aluminum alloy materials (A356.2, A380, AlSi10MgMn, and AlSi9Mn-MoZr) at different equivalent corrosion time points in the salt spray test includes the microscopic structure diagrams of the four die-cast aluminum alloy samples in the early and late stages of the salt spray corrosion test (not shown). In the early corrosion stage (1d), A356.2 and A380 alloys exhibit uniform corrosion morphology, with A380 alloy showing significantly more severe corrosion and more corrosion products. AlSi10MgMn and AlSi9MnMoZr alloys mainly exhibit pitting corrosion, with AlSi10MgMn alloy showing more obvious pitting, consistent with the electrochemical corrosion results. In the late corrosion stage (25d), all four alloys exhibit uniform corrosion on the surface, with corrosion products essentially covering the sample surface and accumulating to some extent. A380 alloy has the most and thickest corrosion products on the surface, followed by AlSi10MgMn alloy, while the states of A356.2 and AlSi9MnMoZr alloys are quite similar.

The microscopic morphology of the four aluminum alloy materials (A356.2, A380, AlSil0MgMn, and AlSi9Mn-MoZr) after 10 days of salt spray corrosion test with corrosion products removed includes the microscopic structure diagrams of the die-cast aluminum alloys after corrosion product removal (not shown). The $\alpha$-Al matrix of all four alloy materials exhibits varying degrees of corrosion. Due to the presence of numerous dispersed Si particles in A356.2 alloy, although Si particles cannot directly participate in corrosion reactions, they accelerate matrix corrosion. Due to the presence of Si particles, it is difficult to form a dense oxide film on the alloy surface, leading to more obvious corrosion of the matrix $\alpha$-Al around Si particles. Moreover, with the occurrence and expansion of corrosion, Si particles may even peel off. In A380 alloy, numerous corrosion pits are distributed around the Al-Si eutectic structure due to the presence of the $Al_2Cu$ phase near the Al-Si eutectic structure, further exacerbating the intergranular corrosion tendency of the Al-Si eutectic structure. Additionally, a small amount of Cu dissolved in the matrix increases the potential difference between grains and inside grains, and the $Al_2Cu$ phase has a relatively positive potential, while the matrix phase with a relatively high Cu content also has a relatively positive potential. In this case, the matrix phase with a relatively low Cu content acts as the cathode phase, forming an electrochemical microcell locally with the nearby Cu-rich matrix phase and $Al_2Cu$ phase,

causing continuous corrosion of the matrix phase with Cu-lean solid solution, leading to the disappearance of grain boundaries. The $Al_{15}(FeMn)_3Si_2$ phase in AlSil0MgMn alloy and the $Al_{12}Mn_3Si_2$ phase in AlSi9MnMoZr alloy have potentials similar to that of the matrix, so after the passivation film is destroyed, corrosion at the grain boundaries and Al-Si eutectic phase boundaries is more severe in AlSi10MgMn and AlSi9MnMoZr alloys. Because the passive film of the AlSi9MnMoZr alloy is more stable and harder to be destroyed, the characteristic of corrosion degree at the grain boundaries and the phase boundaries of the Al-Si eutectic structure in the AlSi10MgMn alloy is more obvious.

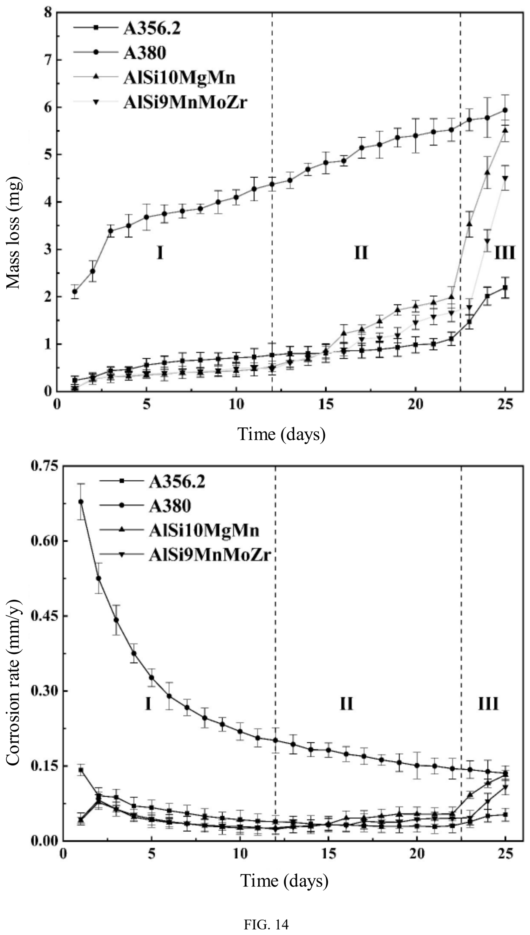

FIG. 14 shows experimental results of mass loss and corrosion rate of the four aluminum alloy materials at different equivalent corrosion time points in the salt spray test. The corrosion weight loss and corrosion rate of A380 alloy are significantly higher than those of the other three alloys, further confirming its poorer corrosion resistance. In stage I (0d to 13d), the corrosion rate of A356.2 alloy is higher than that of AlSil0MgMn and AlSi9MnMoZr alloys. In stage II (14d to 22d), with the rupture of the passivation film and the intensification of localized corrosion behavior, the corrosion rates of AlSi10MgMn and AlSi9MnMoZr alloys gradually increase, while the corrosion rate of A356.2 alloy remains around 0.05 mm/y. In stage III (23d to 25d), the oxide film formed by self-passivation in AlSil0MgMn and AlSi9MnMoZr alloys is almost totally destroyed, and galvanic corrosion caused by the second phase intensifies, resulting in a significant increase in the corrosion rates of AlSi10MgMn and AlSi9MnMoZr alloys.

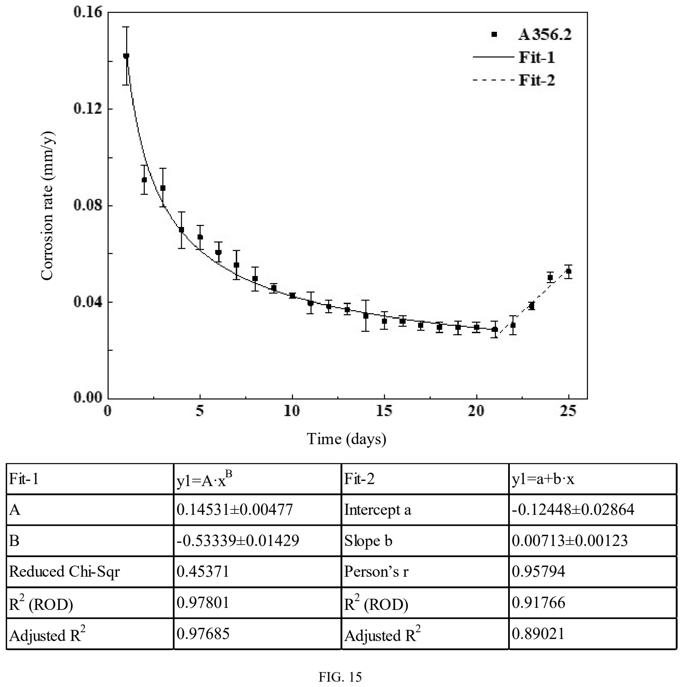

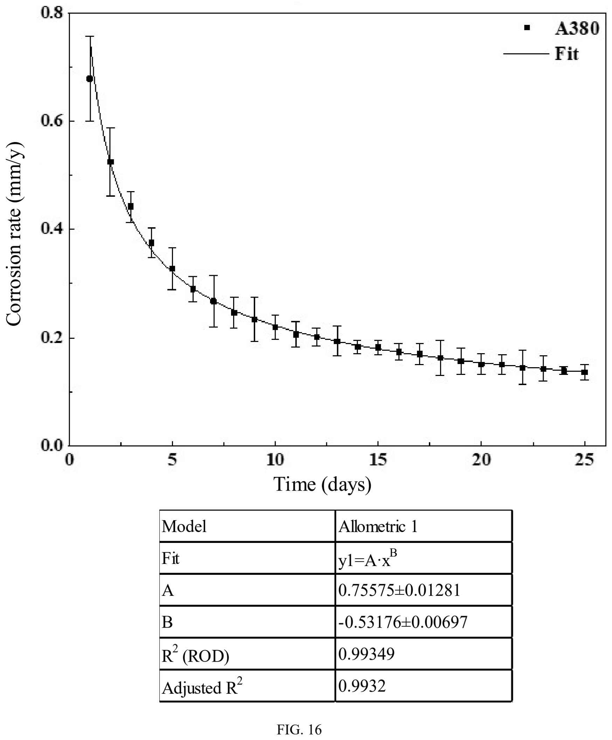

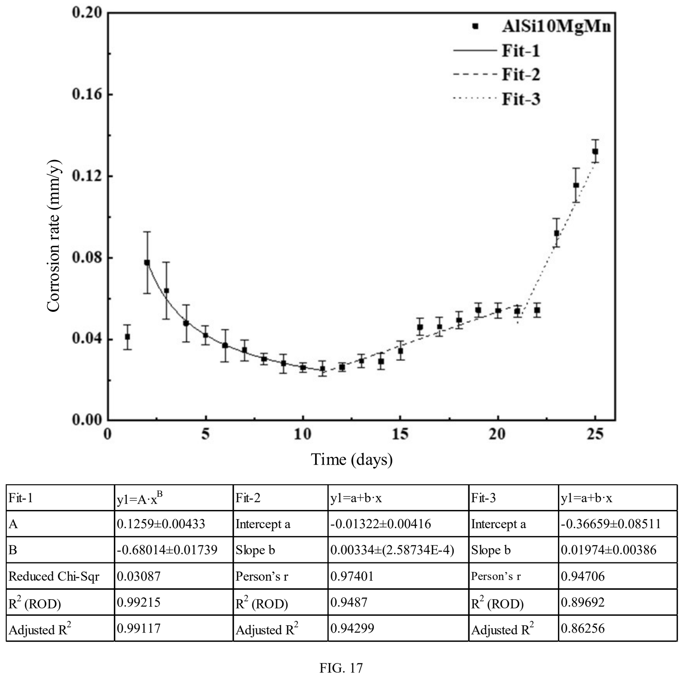

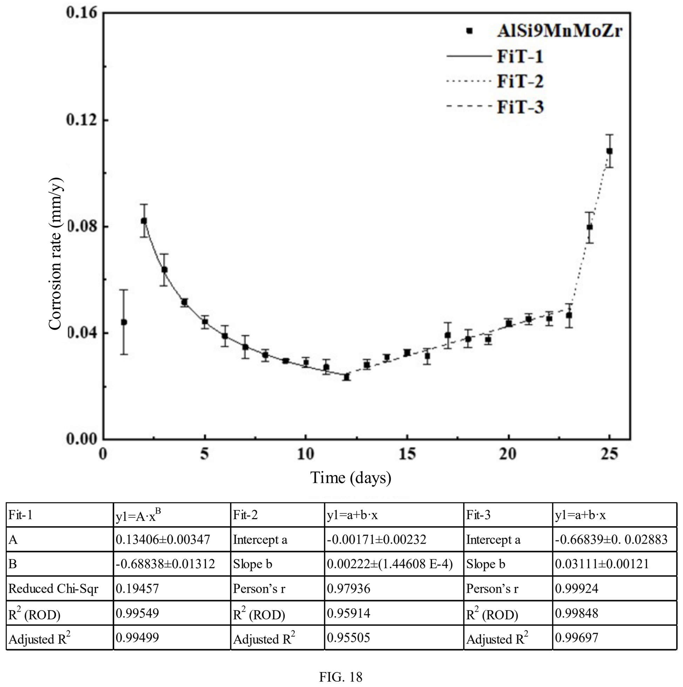

FIG. 15 to FIG. 18 show fitting results of the corrosion rate versus equivalent corrosion time for the four aluminum alloy materials in the salt spray corrosion test. After segmented fitting of the corrosion rates of the four die-cast aluminum alloys, the fitting curves are essentially consistent with the test results. Empirical relational expressions (the first set of empirical relational expressions, also the fourth set of empirical relational expressions) between corrosion rate and equivalent corrosion time are established. The fitting results for the four samples are shown in Table 2.

**Table 2. Parameters related to fitting results of empirical relational expressions between corrosion rate and equivalent corrosion time for four aluminum alloy materials in FIG. 15 to FIG. 18**

| Function fitting result | Value range of equivalent corrosion time (days) | Formula number | Reduced Chi-Sqr (reduced Chi-Sqr) | $R^2$ | Adjusted $R^2$ |
|---|---|---|---|---|---|
| $Y_{A356.2} = 0.145x^{-0.533}$ | $x \in (0, 21)$ | (3-1) | 0.4537 | 0.9780 | 0.9769 |
| $Y_{A356.2} = 0.00713x-0.124$ | $x \in (21, 25)$ | (3-2) | 0.9579 | 0.9177 | 0.8902 |
| $Y_{A380} = 0.755x^{-0.532}$ | $x \in (0, 25)$ | (3-3) | | 0.9935 | 0.9932 |
| $Y_{AlSi10MgMn} = 0.126x^{-0.680}$ | $x \in (1, 11)$ | (3-4) | 0.0309 | 0.9922 | 0.9912 |
| $Y_{AlSi10MgMn} = 0.00334x-0.0132$ | $x \in (11, 21)$ | (3-5) | 0.9740 | 0.9487 | 0.9430 |
| $Y_{AlSi10MgMn} = 0.0197x0.366$ | $x \in (21, 25)$ | (3-6) | 0.9471 | 0.8969 | 0.8626 |
| $Y_{AlSi9MnMoZr} = 0.134x^{-0.688}$ | $x \in (1, 12)$ | (3-7) | 0.1955 | 0.9955 | 0.9950 |
| $Y_{AlSi9MnMoZr} = 0.00222x-0.0171$ | $x \in (12, 22)$ | (3-8) | 0.9794 | 0.9591 | 0.9551 |
| $Y_{AlSi9MnMoZr} = 0.0311x-0.6684$ | $x \in (22, 25)$ | (3-9) | 0.9992 | 0.9985 | 0.9970 |

In Table 2, Reduced Chi-Sqr is equivalent to the residual mean square RSS/dof in Anova; $R^2$ is the coefficient of determination (COD); and "Adjusted $R^2$" is obtained through SPSSAU linear regression.

Based on the above empirical formulas, during the service process of die-cast aluminum alloy parts, the corresponding corrosion rate y can be calculated by the corresponding equivalent corrosion time x based on the corrosion medium and service time, providing guidance for product service life evaluation.

Taking Shanghai, China, as an example, 1d of die-cast aluminum alloy parts in a salt spray corrosion environment is equivalent to more than one year of corrosion in the actual environment (equivalent to establishing a third set of empirical relational expressions between the equivalent corrosion time under the selected corrosion test condition and the service time in the target service environment). Based on the first set of empirical relational expressions in formulas (3-1) to (3-9), a conservative corrosion rate prediction model for the four types of die-cast aluminum alloy parts over 25 years can be established.

4. Tensile test method

(1) Sample dimensions are shown in FIG. 23, in millimeters (mm), where R2.5 indicates a radius of 2.5 millimeters.
(2) Test instrument: Z20 TEW universal material testing machine.
(3) Test analysis method:

The tensile samples of the four die-cast aluminum alloys were wrapped with a blue film, leaving only the test surface exposed. The total test duration was 600 h, and during the test, a batch of samples was taken every 24 hours for tensile property testing, with at least three parallel samples set for each test.
Mechanical property tests were performed on tensile samples at different corrosion stages to obtain stress-strain curves, thereby obtaining the tensile strength at break (tensile strength, abbreviated as UTS, also referred to as tensile strength or ultimate tensile strength), elongation at break (elongation, abbreviated as El), and yield strength (yield strength, abbreviated as YS).

(4) Establishment of the second set of empirical relational expressions describing variation of the mechanical parameter with equivalent corrosion time

The total test duration was divided into three time periods, and fitting was performed using the following functions, to select the function with better fitting results.

$$\text{Optional function 1: } y2 = M \cdot x^N;$$

$$\text{Optional function 2: } y2 = m + n \cdot x;$$

where x is the equivalent corrosion time, y2 is the mechanical parameter, M is a positive number, N is a negative number, n is a negative number, and m is a positive number.
The fitting effect can be evaluated using at least one of Reduced Chi-Sqr, $R^2$, and the adjusted $R^2$.
(5) The first set of empirical relational expressions and the second set of empirical relational expressions form an empirical model for a relationship between corrosion degrees and mechanical properties.

(6) Test analysis results

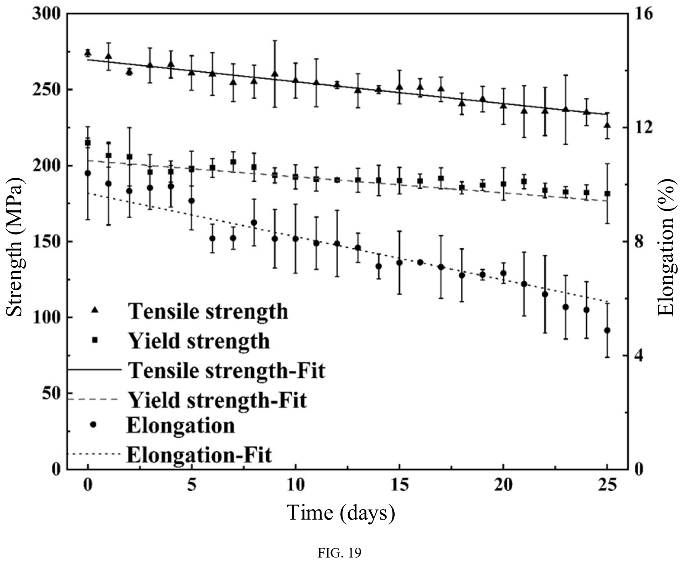

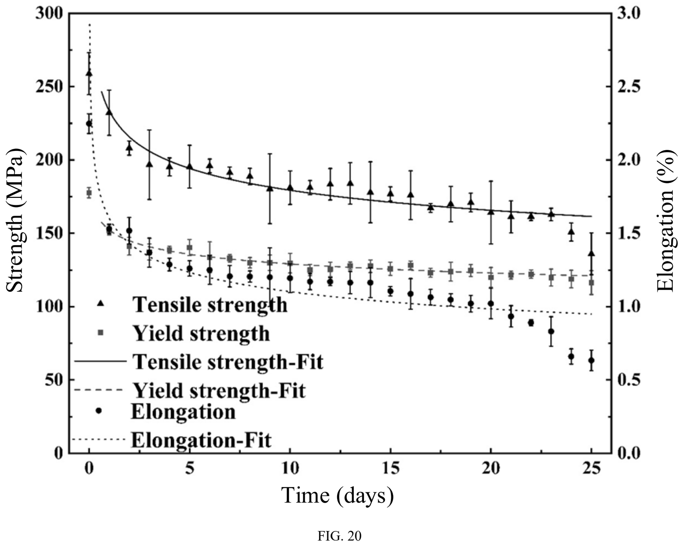

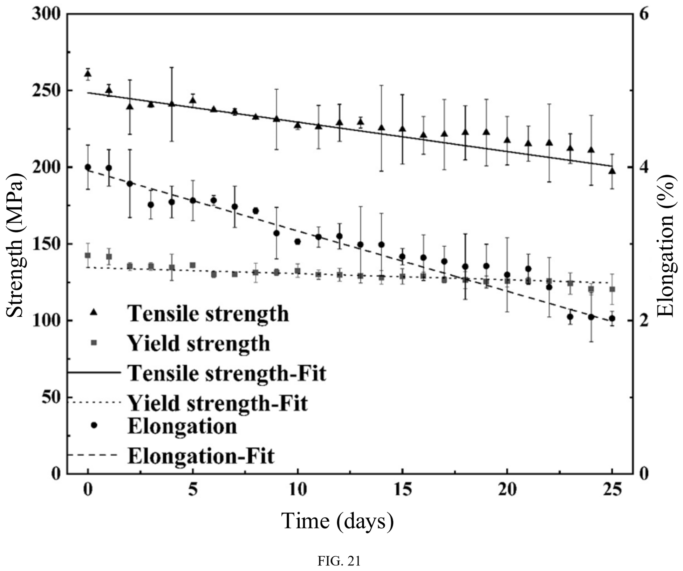

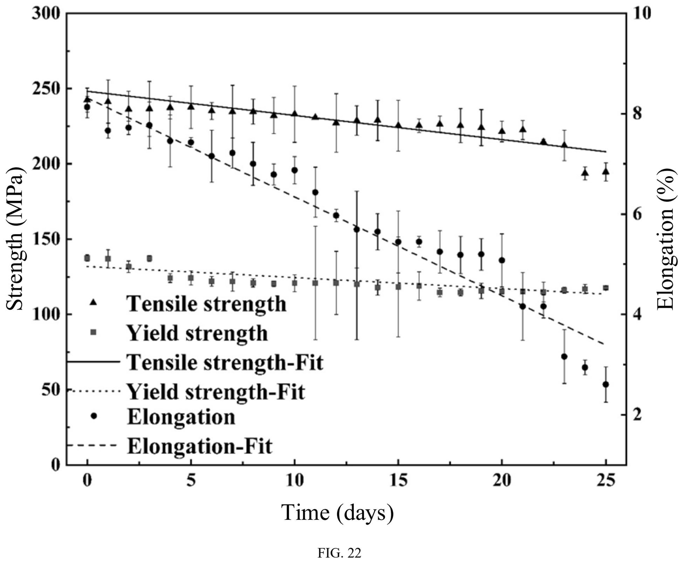

FIG. 19 to FIG. 22 show the experimental results and fitting results of mechanical parameter versus equivalent corrosion time for the four aluminum alloy materials A356.2, A380, AlSi10MgMn, and AlSi9MnMoZr at different equivalent corrosion time points in the salt spray corrosion test; where the mechanical parameters involve tensile strength at break (UTS), elongation at break (El), and yield strength (YS).
According to the test analysis results, the yield strength and tensile strength of A380 alloy decrease rapidly as corrosion time increases, particularly in the early corrosion stage (1d to 4d), with yield strength and tensile strength decreasing to 77.5% and 76% of the values before corrosion, respectively; subsequently, the performance degradation slows down, with final yield strength and tensile strength decreasing to 65.4% and 52.5% of the values before corrosion, respectively. The yield strength and tensile strength of A356.2, AlSi10MgMn, and AlSi9MnMoZr alloys decrease relatively steadily, with the yield strength and tensile strength of A356.2 alloy decreasing to 84.3% and 82.6% of the values before corrosion, respectively, the yield strength and tensile strength of AlSi10MgMn alloy decreasing to 84.4% and 75.6% of the values before corrosion, respectively, and the yield strength and tensile strength of AlSi9MnMoZr alloy decreasing to 85.8% and 80.3% of the values before corrosion, respectively. Additionally, at the corrosion time of 23 days, the tensile strength of AlSi9MnMoZr alloy only decreases to 87.5% of the value before corrosion, higher than the other three alloys (A356.2: 86.4%; A380: 62.9%; AlSi10MgMn: 81.4%). This indicates that when the corrosion time is short, AlSi9MnMoZr alloy has the best corrosion resistance, while A380 alloy consistently shows the worst corrosion resistance.
Compared with the yield strength and tensile strength, the elongation of all four die-cast aluminum alloys decreases rapidly as corrosion time increases. The elongation of A356.2, A380, AlSi10MgMn, and AlSi9MnMoZr alloys decreases to 38.6%, 25.6%, 41.5%, and 25.8% of the values before corrosion, respectively, indicating that corrosion causes a much greater loss in alloy elongation than in strength. This is because, after corrosion occurs, corrosion products are generated on the alloy surface, and $Cl^-$ ions continuously penetrate the alloy interior, creating weak zones that easily become stress concentration areas inside the alloys, promoting crack initiation and propagation during the tensile process. With prolonged corrosion time, more weak zones due to corrosion are formed within the alloys, greatly promoting crack initiation and propagation during the tensile process, leading to a significant decrease in the plastic deformation capacity of the alloys.
Fitting was performed for the three mechanical parameters UTS, El, and YS of the four die-cast aluminum alloys, as shown in FIGs. 19 to 22 and Table 3. The empirical relational expressions for the yield strength, ultimate tensile strength, and elongation with respect to equivalent corrosion time for the four alloys are shown in formulas (4-1) to (4-12), as shown in Table 3. It can be observed that the fitting curves show no significant deviation from the test results, with relatively high $R^2$ and adjusted $R^2$, generally above 0.9 and some even reaching 0.99. In this way, an empirical model for a relationship between corrosion degrees and mechanical properties has been established, where YS, UTS, and El represent yield strength, ultimate tensile strength, and elongation, respectively.

**Table 3. Second set of empirical relational expressions describing variation of mechanical parameter with equivalent corrosion time**

| Fitting result | Value range of equivalent corrosion time (davs) | Formula number |
|---|---|---|
| $YS_{A356.2} = -1.44x + 269.59$ | | (4-1) |
| $UTS_{A356.2} = -1.06x + 203.21$ | 0-25 | (4-2) |
| $El_{A356.2} = -0.15x + 9.69$ | | (4-3) |
| $YS_{A380} = 233.54x^{-0.11}$ | | (4-4) |
| $UTS_{A380} = 152.16x^{-0.07}$ | 0-25 | (4-5) |
| $El_{A380} = 1.60x^{-0.16}$ | | (4-6) |
| $YS_{AlSi10MgMn} = -1.91x + 248.48$ | | (4-7) |
| $UTS_{AlSi10MgMn} = -0.40x + 134.55$ | 0-25 | (4-8) |
| $El_{AlSi10MgMn} = -0.08x + 3.95$ | | (4-9) |
| $YS_{AlSi9MnMoZr} = -1.61x + 248.21$ | | (4-10) |
| $UTS_{AlSi9MnMoZr} = -0.73x + 131.83$ | 0-25 | (4-11) |
| $El_{AlSi9MnMoZr} = -0.19x + 8.31$ | | (4-12) |

5. Correlation analysis between corrosion degrees and mechanical parameters

As corrosion progresses, the mechanical properties of the material decrease. Generally, the factors affecting mechanical properties are complex. For battery casings, the special application environment of the battery casings has a high impact on corrosion resistance, allowing for mechanical property prediction and service life evaluation of metallic materials with the corrosion resistance performance as a link.

From comparison of the corrosion rate-equivalent corrosion time curves in FIGs. 15 to 18 with the mechanical parameter-equivalent corrosion time curves in FIGs. 19 to 22, it can be found that the response behaviors of the corrosion rate and mechanical parameter to equivalent corrosion time differ significantly, making it difficult to effectively reflect the corrosion failure behavior of samples using only the corrosion rate-equivalent corrosion time curve. Simply determining the corrosion rate cannot determine its impact on service life. In the above examples, a correspondence between corrosion resistance performance and service life is established by establishing a relationship between corrosion rates and mechanical properties.

Disadvantages of other analysis models: The decline in mechanical properties is caused by many factors. In addition to corrosion factors, environmental factors such as temperature, humidity, and electrolyte type also affect mechanical properties, making it difficult to construct a prediction model containing only mechanical property parameters.

According to the analysis of tensile fracture surfaces at different corrosion degrees, a strong correlation between corrosion degrees and mechanical parameters is found, validating the feasibility of using the established corrosion degree-mechanical parameter analysis model for predicting mechanical properties or service life.

Tensile fracture surface characterization method: Scanning electron microscopy (SEM) technology, NOVA NanoSEM 230 low-vacuum ultra-high-resolution field emission electron microscope.

The fracture morphology of the four die-cast aluminum alloy materials A356.2, A380, AlSil0MgMn, and AlSi9MnMoZr before salt spray corrosion can be observed using SEM technology. The fracture surface of A356.2 alloy shows large or small dimple morphology and cleavage steps, indicating a quasi-cleavage fracture mode. The fracture surface of A380 alloy has large areas of cleavage planes with almost no dimple morphology, showing obvious brittle fracture characteristics. The fracture surfaces of AlSi10MgMn and AlSi9MnMoZr alloys show cleavage facets roughly the size of the grain, because in polycrystalline materials, although the macroscopic fracture surface is nearly perpendicular to a direction of the maximum tensile stress, the cleavage planes of individual grains in the microstructure are not all perpendicular to the tensile stress. Additionally, a small number of terraced cleavage steps and river-like patterns are observed on the fracture surface, along with tear ridges caused by plastic deformation, indicating occurrence of plastic deformation.

The fracture morphology at the center (Core) and the edge (Edge) near the test surface of the four die-cast aluminum alloy materials at different corrosion times (5d, 15d, 25d) can be observed using SEM technology.

Fracture morphology of A356.2 alloy at different corrosion times during the salt spray corrosion test: As corrosion time increases, the morphology at the fracture center remains very similar, still exhibiting quasi-cleavage morphology with dimples, but the number and prominence of dimples decrease, and by 25 days, the fracture is essentially cleavage morphology. The fracture edge of A356.2 alloy differs, with many gray corrosion products appearing on the fracture surface, and the defect areas in the fracture gradually increase, with many small voids observable. As corrosion time increases, the volume and number of voids increase. These defect locations have significantly reduced strength and are prone to stress concentration, leading to crack initiation and propagation, thereby significantly reducing the elongation of A356.2 alloy.

Fracture morphology of A380 alloy at different corrosion times during the salt spray corrosion test: The fracture morphology of A380 alloy differs from that of A356.2 alloy. For A380 alloy, the fracture center shows cleavage fracture and some corrosion products, indicating a deeper corrosion depth. As corrosion time increases, the cleavage steps on the fracture surface gradually enlarge. Due to corrosion, the differences between different small facets gradually disappear, and multiple cleavage planes that are parallel or nearly parallel at different heights meet to form large cleavage steps. At the fracture edge, large cleavage planes appear initially, and as corrosion progresses, these cleavage planes crack into small planes with many slender microcracks between them, indicating that corrosion has extended into the grain interior of A380 alloy, resulting in numerous cracks from the surface to the grain interior, significantly reducing its elongation. Additionally, due to the presence of more corrosion products at the fracture center, indicating deeper corrosion, the increasing number of defects such as cracks within the alloy significantly affects strength.

Fracture morphology of AlSi10MgMn alloy at different corrosion times during the salt spray corrosion test: Many cleavage facets are present at the fracture center, and when corrosion time is relatively short (5d), some dimples are visible, indicating a quasi-cleavage fracture mode. Additionally, a small number of voids appear at the fracture center, particularly noticeable at relatively long corrosion times (15d and 25d). Gray corrosion products are also observed at the fracture edge, and as corrosion progresses, the fracture edge splits from large cleavage steps into small planes separated by cracks. Compared to A380 alloy, the number of corrosion products and small planes is lower, indicating better corrosion resistance.

Fracture morphology of AlSi9MnMoZr alloy at different corrosion times during the salt spray corrosion test: Compared to AlSi10MgMn alloy, many small dimples and obvious tear ridge morphology are present at the fracture center, indicating more plastic deformation. Dimples are a primary feature of plastic fracture, and a higher number of smaller dimples indicate stronger plastic deformation capacity, resulting in higher elongation than AlSi10MgMn alloy. Additionally, when corrosion time is relatively short (5d), many dimples and cleavage steps are still observable at the fracture edge, so the performance degradation of AlSi9MnMoZr alloy is not severe in relatively short corrosion time. As corrosion time increases, the fracture edge morphology gradually transitions to cleavage morphology, with a small amount of corrosion products appearing at the edge. When corrosion time is relatively long (25d), many small pores appear at the edge, but the number of microcracks is low, so macroscopically, the yield strength and tensile strength of AlSi9MnMoZr alloy do not decrease significantly, while the elongation decreases rapidly.

From the above analysis results, it can be seen that in a corrosive environment, for alloy samples with different mechanical failure behaviors and fracture morphologies, there is a strong correlation between mechanical properties and equivalent corrosion time. Therefore, the method for constructing a corrosion degree-mechanical parameter analysis model described above has a certain universality, and the constructed analysis model can effectively analyze mechanical properties or service life.

III. Electrochemical corrosion test

1. Test analysis method

Electrochemical corrosion includes: open circuit potential testing, alternating current impedance testing, and potentiodynamic polarization testing.

Before the test, the die-cast aluminum alloy was cut into samples of 10 mm $\times$ 10 mm $\times$ 2 mm, and the samples were cold-mounted with epoxy resin, with only a test surface area of 1 cm$^2$ exposed. The test surface was sequentially ground with abrasive paper and polished with a polishing agent until the surface was clean and shiny. Then the samples were cleaned with ethanol, and dried for later use.

During the test, the open circuit potential (OCP) of the sample was first measured. After the OCP became stable for 60 minutes, alternating current impedance (EIS) testing was performed to measure the electrochemical impedance spectrum of the sample at different AC frequencies. During the test, a 10 mV AC sine wave was used as the excitation voltage, and the test frequency was controlled within 0.01 Hz to $10^5$ Hz.

After the test, the EIS results were fitted using an equivalent circuit, and the parameters of the components in the equivalent circuit diagram were analyzed. After the electrochemical impedance spectroscopy test, potentiodynamic polarization testing was performed. Starting from an OCP of 300 mV, scanning was performed at a scanning speed of 0.167 mV/s until the current exceeded 1 mA. After the test was complete, Tafel fitting was performed for the polarization characteristic data of the sample through ZSimpWin v3.40 software.

The structure of the three-electrode system in the electrochemical corrosion test equipment is shown in FIG. 24, where R, mA, and V represent a resistance meter, an ammeter, and a voltmeter, respectively, and their combination can be understood as an electrochemical workstation which can collect sample signals as measurement data for corrosion parameters by adjusting the given voltage and current.

2. Test results

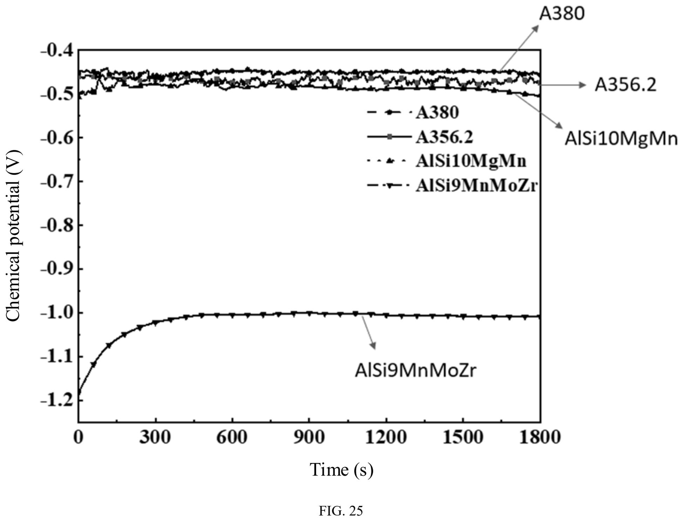

The variation curves of the open circuit potential (OCP) over time for the four aluminum alloy materials are shown in FIG. 25. The OCP values of the four die-cast aluminum alloy materials stabilize after 300s, and the potentials remain stable with minimal fluctuations after 1800s. The change in OCP values is related to the formation of the surface layer, and the surface layer can control subsequent electrochemical reactions. The OCP values of A356.2, A380, AlSi10MgMn, and AlSi9MnMoZr alloys are -0.474 V, -0.457 V, -0.603 V, and -1.009 V, respectively, indicating that the different microstructures of the four die-cast aluminum alloys alter the formation and surface reactions of their surface layers.

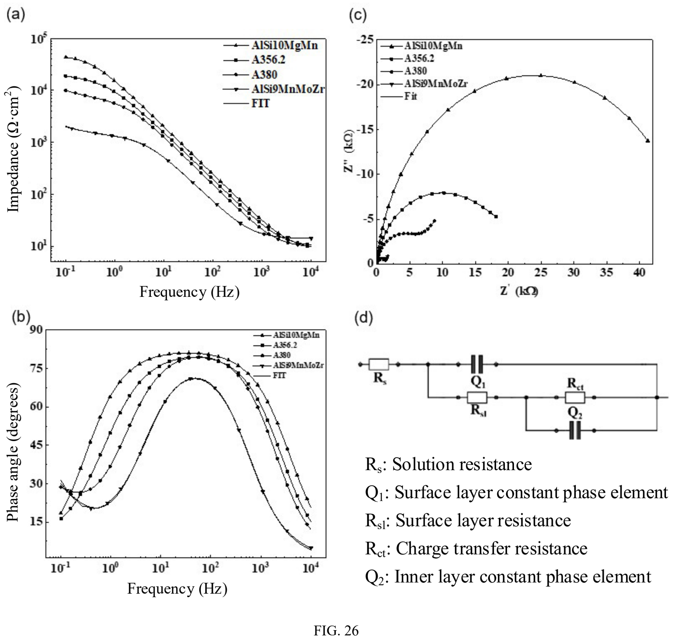

The electrochemical impedance test results for the four die-cast aluminum alloys are shown in FIG. 26 and Table 4. (a) is the Bode plot of the alternating-current impedance spectrum (EIS Bode plot), (b) is the phase plot (phase plot), (c) is the Nyquist plot (Nyquist plot), and (d) is the equivalent circuit diagram. From the Bode curve in FIG. 26(a) and the phase curve in FIG. 26(b), it can be seen that, whether at low or high frequencies, AlSi10MgMn alloy exhibits the highest impedance and phase angle, followed by A356.2 and A380 alloys, and finally AlSi9MnMoZr alloy. In the Nyquist plot (FIG. 26(c)), all four alloy materials show a suppressed capacitance loop, with AlSi10MgMn alloy having the largest capacitance loop diameter, indicating better capacitance performance and higher charge transfer resistance. The EIS test results are fitted with the equivalent circuit diagram in FIG. 26(d), and the specific parameters of the components are shown in Table 4.

In the equivalent circuit diagram, $R_s$ represents the solution resistance related to the corrosion solution used in the test, $R_{sl}$ and $R_{ct}$ represent the surface layer resistance and charge transfer resistance of each alloy as the working electrode, respectively. Additionally, the deviation of capacitance in EIS from ideal capacitance behavior is represented by a constant phase element (CPE), and n is used as an indicator to evaluate the closeness between actual test and theoretical calculations. $Q_1$ and $Q_2$ represent the capacitance of the surface layer and charge transfer layer during the establishment of the corrosion cell, corresponding to $n_1$ and $n_2$, respectively. The results show that since the corrosion solutions are all 3.5wt% NaCl solutions, the $R_s$ values of the four alloys are very close. Both $R_{sl}$ and $R_{ct}$ exhibit a trend of AlSi10MgMn > A356.2 > A380 > AlSi9MnMoZr, indicating that AlSi10MgMn demonstrates the best corrosion resistance among the four during the establishment of the corrosion cell. In the EIS test, the alloys are not corroded, so the EIS results are only analysis of the impending corrosion behavior from an electrochemical thermodynamics perspective. However, for some thermodynamically unstable metals, self-passivation may occur under appropriate conditions, transforming non-corrosion-resistant alloys into corrosion-resistant alloys, such as Al, Mg, and Cr. Therefore, studying corrosion behavior solely from a thermodynamic perspective is limited, and the thermodynamic stability of die-cast aluminum alloy materials in corrosive media also need to be considered. According to experimental data, AlSi9MnMoZr alloy undergoes self-passivation in a 3.5wt% NaCl solution, exhibiting excellent corrosion resistance.

**Table 4. Equivalent circuit parameters**

| Aluminum alloy material type | $R_s/\Omega$ | $Q_1/\mu F$ | $n_1$ | $R_{sl}/k\Omega$ | $R_{ct}/k\Omega$ | $Q_2/\mu F$ | $n_2$ |
|---|---|---|---|---|---|---|---|
| A356.2 | 12.64 | 116.0 | 0.901 | 14.3 | 7.79 | 14.7 | 0.923 |
| A380 | 12.37 | 91.4 | 0.985 | 4.67 | 5.59 | 12.5 | 0.965 |
| AlSi10MgMn | 14.26 | 131.2 | 0.918 | 35.1 | 12.8 | 17.5 | 0.969 |
| AlSi9MnMoZr | 11.28 | 35.9 | 0.928 | 1.83 | 1.33 | 8.1 | 0.935 |

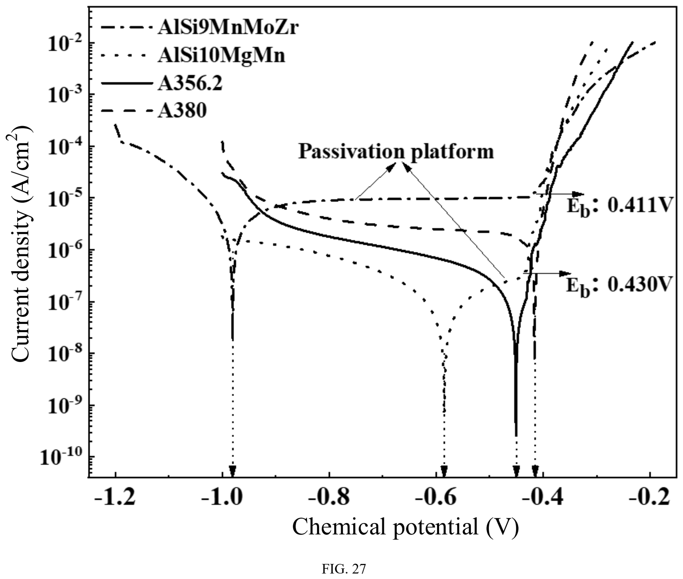

FIG. 27 shows the potentiodynamic polarization curves of the electrochemical corrosion test, and Table 5 lists the relevant potentiodynamic polarization parameters calculated based on the Tafel extrapolation method. The corrosion potentials ($E_{corr}$) of A356.2, A380, AlSi10MgMn, and AlSi9MnMoZr alloys are -0.450 V, -0.417 V, -0.585 V, and -0.981 V, respectively, closely matching the OCP values. Based on electrochemical corrosion behavior, the four die-cast aluminum alloy materials can be divided into two categories: one category consists of alloys without significant passivation, including A356.2 and A380 alloys; the other category consists of alloys with a distinct passivation region, including AlSi10MgMn and AlSi9MnMoZr alloys.

For alloys without significant passivation, corrosion resistance can be primarily evaluated by comparing the corrosion current density ($I_{corr}$) of the alloy. Alloys with a relatively low $I_{corr}$ value and a relatively high $R_p$ value have good corrosion resistance. Tafel fitting reveals that the $E_{corr}$ values of A356.2 and A380 alloys are very close, but the $I_{corr}$ value of A356.2 alloy is approximately one-fourth that of A380 alloy, and its polarization resistance $R_p$ is more than 20 times that of A380 alloy. Therefore, A356.2 alloy exhibits significantly better corrosion resistance than A380 alloy.

For alloys with a significant passivation region, the passivation region needs be analyzed during the corrosion resistance studies. The corrosion current density (Current Density) increases slowly as the potential in the passivation region shifts positively, indicating that the progression of corrosion behavior is significantly slowed. The passivation region inhibits the development of corrosion behavior until the potential reaches the breakdown potential ($E_b$), at which point the passivation film is fully destroyed, and corrosion begins to intensify. The results show that AlSi9MnMoZr alloy has a more obvious passivation region with an $E_b$ value of -0.411 V, higher than that of AlSi10MgMn alloy (-0.430 V). Even though AlSi10MgMn alloy has a lower $I_{corr}$ value than AlSi9MnMoZr alloy, the latter, due to its more stable passivation region, significantly slows the progression of corrosion behavior, exhibiting better corrosion resistance. Additionally, $B_c$ and $B_a$ represent the cathodic and anodic reaction slopes of the Tafel fitting, respectively, with $B_c$ being higher than $B_a$ for all four alloys, indicating that the electrochemical reaction is primarily controlled by the cathodic process.

**Table 5. Tafel fitting parameters**

| Aluminum Alloy material Type | A356.2 | A380 | AlSi10MgMn | AlSi9MnMoZr |
|---|---|---|---|---|
| $E_{corr}$ (V) | -0.45 | -0.417 | -0.585 | -0.981 |
| $I_{corr}$ ($\mu$A/cm$^2$) | 0.446 | 1.79 | 0.74 | 2.12 |
| $R_p$ (k$\Omega \cdot$ cm$^2$) | 168 | 7.08 | 372 | 12.5 |
| $B_a$ (mV/dec) | 127 | 24.6 | 171 | 98.8 |
| $B_c$ (mV/dec) | 166 | 355 | 226 | 158 |

Regarding the surface morphology of the alloy materials after dynamic polarization, A356.2 and A380 alloys primarily exhibit uniform corrosion after polarization, with A380 alloy having more corrosion products on its surface than A356.2 alloy, further confirming its poor corrosion resistance. After polarization, AlSi 10MgMn and AlSi9MnMoZr alloys, due to the presence of a passivation film on the surface, primarily exhibit pitting corrosion characteristics on the surface morphology. Additionally, compared to AlSi9MnMoZr alloy, AlSi10MgMn alloy has larger and deeper pitting corrosion sites, indicating that the passivation film of AlSi9MnMoZr alloy is more resistant to destruction, consistent with the results of the potentiodynamic polarization curves.

Therefore, parameters characterizing the corrosion degree in electrochemical corrosion tests can also be used to construct the corrosion resistance-mechanical property analysis model.

IV. Immersion corrosion test

1. Test analysis method

Staged immersion corrosion involves analyzing the macroscopic morphology, microscopic morphology, and corrosion rate of the battery casing material every 10 days in a total test duration of 30 days. In this application, 1d is equal to 1 day.

The samples for the immersion corrosion test can be blocks of 15 mm × 15 mm × 2 mm.

Before the test, the test surface was ground and polished, and then the sample was rinsed sequentially with deionized water and ethanol, dried, weighed, and stored in a desiccator for later use. During the test, the sample was placed into a container, and 500 mL of corrosion solution were added, where the composition of the corrosion solution was a 3.5wt% NaCl aqueous solution. After sealing, the entire container was placed into a constant-temperature water bath tank, with the temperature set to 25°C. Samples were taken every 10 days (that is, test durations were 10 days, 20 days, and 30 days), and the macroscopic morphology and microscopic morphology of the corroded sample surface were observed. Then, corrosion products on the sample surface are removed, and the sample masses before and after corrosion were compared to calculate the corresponding immersion corrosion rate. At least three parallel samples were set for each test.

2. Test result analysis

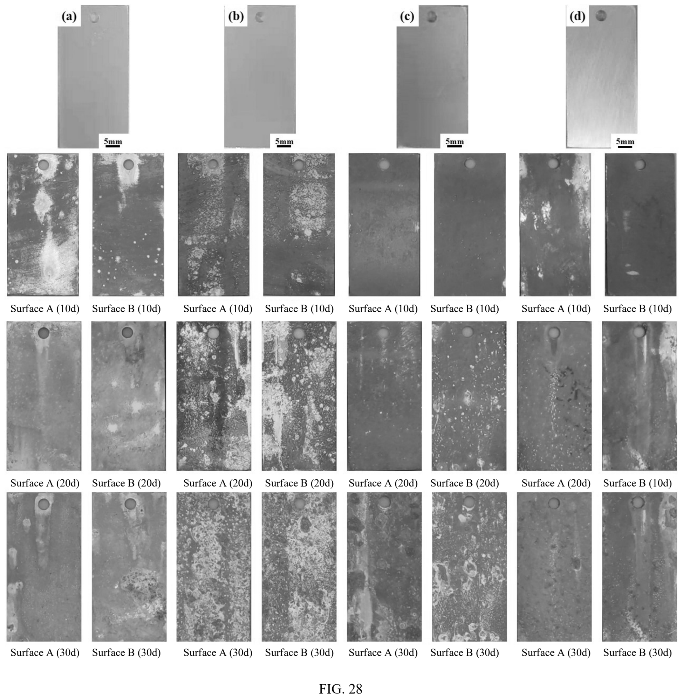

Immersion corrosion tests were conducted for 10 days, 20 days, and 30 days. The macroscopic morphologies of the sample surfaces at different immersion corrosion times are shown in FIG. 28. After immersion in a 3.5wt% NaCl solution, the surfaces of all four die-cast aluminum alloys are covered with obvious corrosion products. Compared to the appearance of the samples before corrosion, the surfaces of the corroded samples become rough, with a black corrosion layer and gray dotted corrosion products. Additionally, the corrosion products on A380 (FIG. 28(b)) and AlSi10MgMn alloys (FIG. 28(c)) are more obvious, indicating that their corrosion resistance is inferior to that of A356.2 (FIG. 28(a)) and AlSi9MnMoZr alloys (FIG. 28(d)). For samples at 10 days, 20 days, and 30 days, A380 alloy has the most corrosion products on its surface, indicating the worst corrosion resistance. At 10 days of immersion corrosion, the macroscopic corrosion morphologies of A356.2, AlSi10MgMn, and AlSi9MnMoZr alloys are similar, while at 20 days and 30 days of immersion corrosion, A356.2 alloy exhibits better corrosion resistance. Due to the destruction of the passivation film and the intensification of pitting corrosion, AlSi10MgMn and AlSi9MnMoZr alloys show a large amount of white corrosion products on the surface. AlSi10MgMn alloy has a relatively large area covered with spotted white corrosion products, indicating that AlSi9MnMoZr alloy has better corrosion resistance, consistent with the salt spray corrosion results.

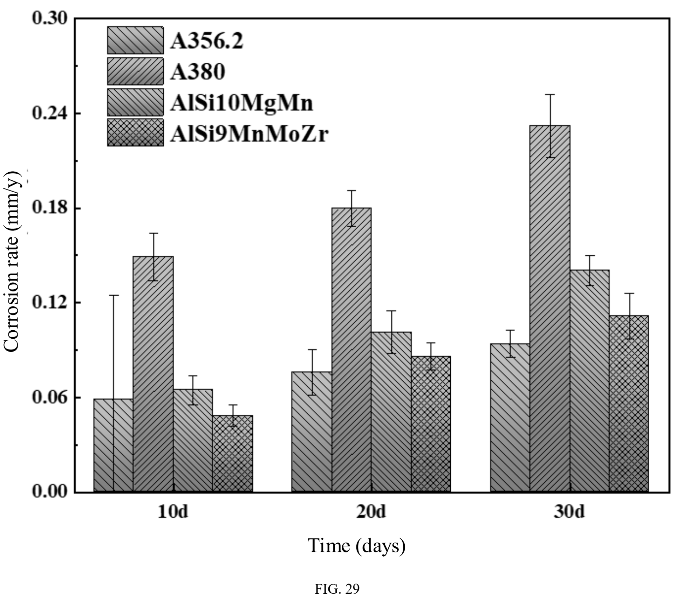

The immersion corrosion rates were calculated based on the weight loss results of the samples before and after immersion corrosion. The analysis results are shown in FIG. 29. As the immersion time increases from 10 days to 20 days and 30 days, the corrosion rates of all four alloy materials increase to varying degrees. This is because, as corrosion progresses, more defect sites appear on the alloy surface, and corrosive $Cl^-$ ions are more easily adsorbed at defect sites, thereby accelerating corrosion. After 10 days of immersion in a 3.5wt% NaCl aqueous solution, the corrosion rates of A356.2, A380, AlSi10MgMn, and AlSi9MnMoZr alloys are 0.058 mm/y, 0.149 mm/y, 0.064 mm/y, and 0.048 mm/y, respectively, indicating that AlSi9MnMoZr alloy has the best corrosion resistance in the early corrosion stage. After 30 days of immersion, the corrosion rates of A356.2, A380, AlSi10MgMn, and AlSi9MnMoZr alloys are 0.094 mm/y, 0.232 mm/y, 0.141 mm/y, and 0.111 mm/y, respectively, indicating that A356.2 alloy has better corrosion resistance in longer-term immersion corrosion tests. The destruction of the passivation film and the intensification of pitting corrosion are the main reasons for the decreased corrosion resistance of AlSi9MnMoZr alloy.

Therefore, parameters related to the corrosion degree in immersion corrosion tests can also be used to construct the corrosion resistance-mechanical property analysis model.

V. Application of the model construction method

(1) Test samples and test methods

An aluminum alloy sample (denoted as N1) was used. The composition of the alloy material is shown in Table 6.

**Table 6. Nominal composition and actual composition of aluminum alloy sample N1**

| Element type | Element content (wt%) | | | | | | | | |
|---|---|---|---|---|---|---|---|---|---|
| | Si | Cu | Ti | Mg | Zn | Mn | Sr | Fe | Al |
| Nominal composition (Nominal composition) | 8.5 | 0.7 | 0.3 | 0.4 | 0.5 | 1.0 | 0.04 | 0.4 | Base |
| Actual composition (Actual composition) | 8.01 | 0.69 | 0.47 | 0.35 | 0.45 | 0.98 | 0.02 | 0.23 | Base |

In Table 6, element content refers to a mass percentage in the aluminum alloy material, in wt%.

Taking the Mn element as an example, the corresponding component for the Mn element is referred to as "Mn component".

The components for each element are: $AlSi_{20}$ (Si component), $AlCu_{50}$ (Cu component), $AlTi_5$ (Ti component), Zn (Zn component), Mg (Mg component), $AlMn_{10}$ (Mn component), $AlSr_{10}$ (Sr component), and Al. Taking 1 kilogram (kg) as an example, the burn-off rate for Zn is 12%, and the burn-off rate for Mg is 15%.

The Fe element is introduced during the preparation process. It is an unavoidable impurity element and is not actively added.

The aluminum alloy material N1 was prepared using the following method:

(1) The pure aluminum ingot was first added to a smelting furnace, heated to 740°C, and held at that temperature until it was melted. Then the Mn component was added. After Mn was melted, the Cu component was added. When the temperature dropped to 710°C, the Si component, Ti component, Zn component, and Sr component were added for melting. After these elements were melted, the Mg component was added. After the Mg component was melted, the melt was left standing, the slag was removed, and a refining agent was added. After further slag removal, the Ti component and Sr component were added. Finally, the melt was cast into a mold to obtain an ingot of the preset dimensions. The preset dimensions are the sample dimensions required for the test.

(2) The ingot was held at 550°C for 2 hours, and then water-cooled to room temperature (20°C-30°C), to obtain the aluminum alloy material, which was a die-cast aluminum alloy sample. This aluminum alloy material can be used as the main material or constituent material for battery casings, including but not limited to being used as the main material or constituent material for lithium battery casings, fuel cell casings, and the like.

Elemental analysis was performed using an inductively coupled plasma emission spectrometer (ICP, Avio 5000) to accurately measure the actual composition of the aluminum alloy material. The ICP test results show that the actual composition of the aluminum alloy material is very close to its nominal composition, indicating good smelting results.

(2) Test methods:

The test conditions and sampling time points for the salt spray corrosion test, tensile test, electrochemical corrosion test, and immersion test are the same as those described in Sections II, III, and IV above.

(3) Test analysis results

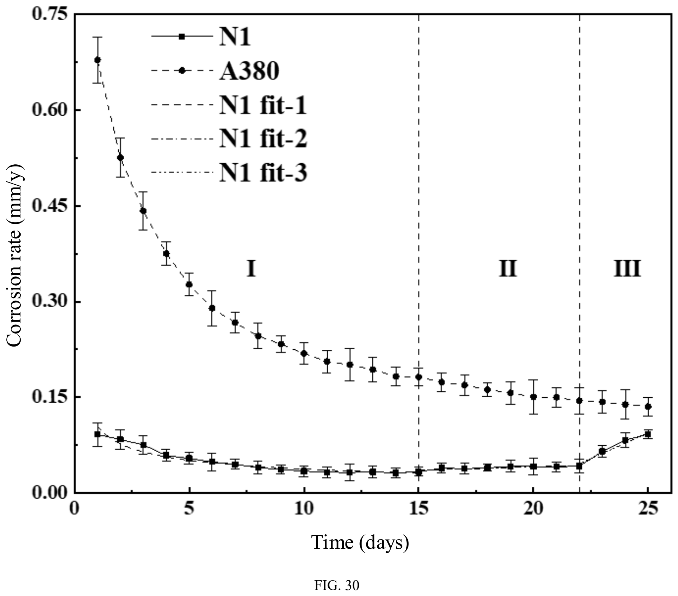

The empirical relational model for corrosion rate and equivalent corrosion time (first set of empirical relational expressions) is shown in formulas (5-1 to 5-3), as shown in Table 7 and FIG. 30. It can be found that after segmented fitting of the corrosion rate using the function $y = a \cdot b^x$ and the function $y = a + b \cdot x$, the fitting results are essentially consistent with the test results.

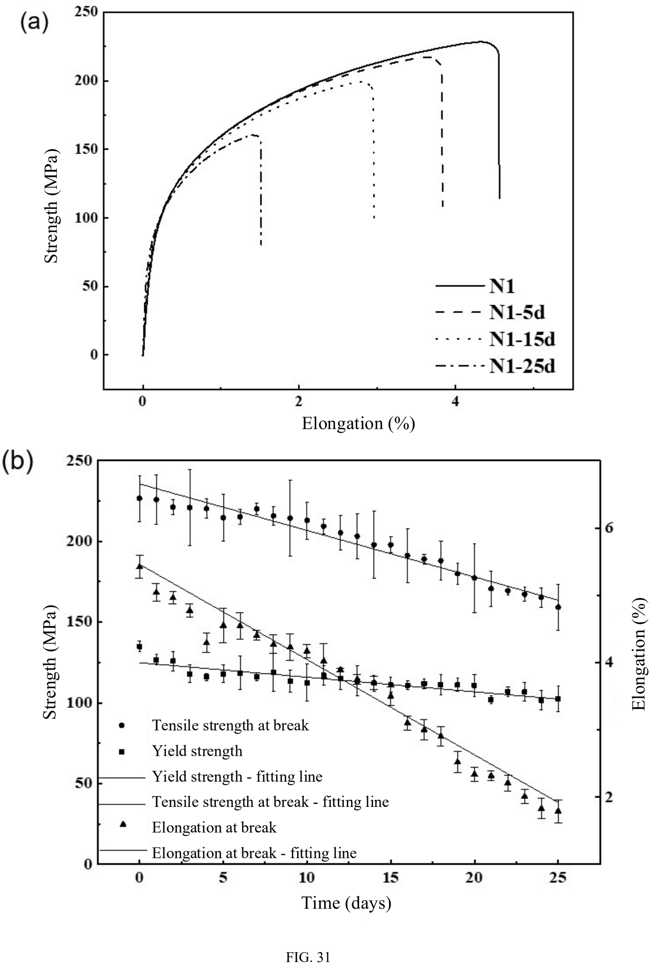

For the fitting results of the mechanical property-corrosion time curves, reference may be made to the second set of empirical relational expressions describing variation of the mechanical parameter with equivalent corrosion time in FIG. 31 and Table 8. The fitting results are essentially consistent with the test results.

**Table 7**

| Fitting result (first set of empirical relational expressions) | Value range of equivalent corrosion time (days) | Formula number |
|---|---|---|
| $y_{N1} = 0.105x^{-0.447}$ | $x \in (0, 15)$ | (5-1) |
| $y_{N1} = 0.002x + 0.019$ | $x \in (15, 22)$ | (5-2) |
| $y_{N1} = 0.016x - 0.307$ | $x \in (22, 25)$ | (5-3) |

**Table 8**

| Fitting result (second set of empirical relational expressions) | Formula number |
|---|---|
| $YS_{N1} = -0.902x + 124.889$ | (5-4) |
| $UTS_{N1} = -2.879x + 235.557$ | (5-5) |
| $EI_{N1} = -0.141x + 5.459$ | (5-6) |

The descriptions of the various embodiments and examples above tend to emphasize the differences between the embodiments and examples, and their similarities or commonalities can be referenced mutually, and for brevity, are not reiterated herein.

The technical features of the various embodiments and examples described above can be arbitrarily combined. To keep the description concise, not all possible combinations of the technical features in the above embodiments and examples are described. However, as long as there is no contradiction in the combination of these technical features, they should be considered within the scope described in this specification.

It should be noted that this application is not limited to the above embodiments and examples. The above embodiments and examples are merely exemplary, and embodiments with substantially the same configuration and effects as the technical idea within the scope of the technical solution of this application are included in the technical scope of this application. The embodiments and examples described above express only a few implementations of this application, and their descriptions are relatively detailed but should not be construed as limiting the scope of the patent. Additionally, within the scope not departing from the gist of this application, various modifications that those skilled in the art can conceive and other methods constructed by combining some constituent elements of the embodiments or examples are also included in the scope of this application.

**Claims**

1.  A method for constructing a corrosion resistance-mechanical property analysis model for a metallic material, comprising the following steps:

    using a metallic material as a test object, acquiring test values of a corrosion parameter at different equivalent corrosion time points under a corrosion test condition to obtain a corrosion performance test experimental dataset, and acquiring test values of a mechanical parameter at different corrosion degrees corresponding to the different equivalent corrosion time points to obtain a mechanical property test experimental dataset; wherein the corrosion test condition is used to simulate a target service environment of the metallic material; and the corrosion parameter comprises at least one of a corrosion degree and a corrosion rate; and

    establishing, based on the corrosion performance test experimental dataset, a first set of empirical relational expressions describing variation of the corrosion parameter with equivalent corrosion time, and establishing, based on the mechanical property test experimental dataset, a second set of empirical relational expressions describing variation of the mechanical parameter with equivalent corrosion time.

2.  The method for constructing a corrosion resistance-mechanical property analysis model for a metallic material according to claim 1, wherein the mechanical parameter comprises at least one of a tensile strength at break and an elongation at break.

3.  The method for constructing a corrosion resistance-mechanical property analysis model for a metallic material according to claim 1 or 2, wherein the mechanical parameter comprises a yield strength.

4.  The method for constructing a corrosion resistance-mechanical property analysis model for a metallic material according to any one of claims 1 to 3, wherein at least one of the following characteristics is met:

    the metallic material is an aluminum alloy material; or
    the metallic material is a metallic structural component, optionally an aluminum alloy structural component.

5.  The method for constructing a corrosion resistance-mechanical property analysis model for a metallic material according to any one of claims 1 to 4, wherein at least one of the following characteristics is met:

    the metallic material is any one of battery casing materials; optionally, the battery casing materials comprise a fuel cell casing material; optionally, the battery casing materials comprise a lithium battery casing material; or
    the metallic material comprises at least a portion of a structural component of a battery casing; optionally, the battery casing structural component comprises at least a portion of a structural component of a fuel cell casing; optionally, the battery casing structural component comprises at least a portion of a structural component of a lithium battery casing.

6.  The method for constructing a corrosion resistance-mechanical property analysis model for a metallic material according to any one of claims 1 to 5, wherein the corrosion performance test experimental dataset comprises at least a corrosion performance test experimental dataset under a salt spray corrosion test condition;

    optionally, the salt spray corrosion test condition comprises at least one of the following salt spray conditions: one or more of NaCl aqueous solution salt spray condition, acetic acid salt spray condition, copper-accelerated acetic acid salt spray condition, and alternating salt spray corrosion condition;
    optionally, the salt spray corrosion test condition comprises a NaCl aqueous solution salt spray condition;
    optionally, the NaCl aqueous solution salt spray condition comprises the following parameter: a simulated salt spray condition with a 3wt% to 6wt% NaCl aqueous solution at 34°C-36°C.

7.  The method for constructing a corrosion resistance-mechanical property analysis model for a metallic material according to any one of claims 1 to 6, wherein the establishing, based on the corrosion performance test experimental dataset, a first set of empirical relational expressions describing variation of the corrosion parameter with equivalent corrosion time comprises:
    for each type of corrosion parameter in the corrosion parameter, performing segmented fitting based on the corresponding corrosion performance test experimental dataset, and for each fitting interval, with the corresponding corrosion parameter as a dependent variable and the equivalent corrosion time as an independent variable, performing fitting in the form of a power function or a linear function to construct an empirical relational expression between each type of corrosion parameter and equivalent corrosion time for each fitting interval, thereby obtaining the first set of empirical relational expressions.

8. The method for constructing a corrosion resistance-mechanical property analysis model for a metallic material according to claim 7, wherein in the first set of empirical relational expressions, a fitting form of the power function is $y1 = A \cdot x^B$, and a fitting form of the linear function is $y1 = a + b \cdot x$; wherein x is the equivalent corrosion time, y1 is the corrosion parameter, A is a positive number, B is a negative number, b is a positive number, and a is a real number; optionally, a is a negative number.

9. The method for constructing a corrosion resistance-mechanical property analysis model for a metallic material according to claim 8, wherein A is a real number selected from 0.01 to 1.00, B is a real number selected from -0.3 to -0.8, b is a real number selected from 0.001 to 0.05, and a is a real number selected from 0.02 to -0.8;

   optionally, a is a real number selected from -0.001 to -0.8, further optionally, a is a real number selected from -0.001 to -0.5;
   optionally, b is a real number selected from 0.001 to 0.02, further optionally, b is a real number selected from 0.001 to 0.01.

10. The method for constructing a corrosion resistance-mechanical property analysis model for a metallic material according to any one of claims 1 to 9, wherein the corrosion performance test experimental dataset comprises at least one of a corrosion performance test experimental dataset under an electrochemical corrosion test condition and a corrosion performance test experimental dataset under an immersion corrosion test condition;

   optionally, a corrosion parameter in the corrosion performance test experimental dataset under the electrochemical corrosion test condition comprises at least one of a self-corrosion potential or a self-corrosion current;
   optionally, a corrosion parameter in the corrosion performance test experimental dataset under the immersion corrosion test condition comprises at least one of a corrosion degree and a corrosion rate.

11. The method for constructing a corrosion resistance-mechanical property analysis model for a metallic material according to any one of claims 1 to 10, wherein the establishing, based on the mechanical property test experimental dataset, a second set of empirical relational expressions describing variation of the mechanical parameter with equivalent corrosion time comprises:
   for each type of mechanical parameter in the mechanical parameter, performing segmented fitting based on the corresponding mechanical property test experimental dataset, and for each fitting interval, with the corresponding mechanical parameter as a dependent variable and the equivalent corrosion time as an independent variable, performing fitting in the form of a power function or a linear function to construct an empirical relational expression between each type of mechanical parameter and the equivalent corrosion time, thereby obtaining the second set of empirical relational expressions.

12. The method for constructing a corrosion resistance-mechanical property analysis model for a metallic material according to claim 11, wherein in the second set of empirical relational expressions, a fitting form of the power function is $y2 = M \cdot x^N$, and a fitting form of the linear function is $y2 = m + n \cdot x$; wherein x is the equivalent corrosion time, y2 is the mechanical parameter, M is a positive number, N is a negative number, n is a negative number, and m is a positive number.

13. The method for constructing a corrosion resistance-mechanical property analysis model for a metallic material according to claim 12, wherein M is a real number selected from 1 to 250, N is a real number selected from -0.01 to -1, n is a real number selected from -0.05 to -5, and m is a real number selected from 1 to 300;

   optionally, N is a real number selected from -0.01 to -0.5, further optionally, N is a real number selected from -0.01 to -0.2;
   optionally, n is a real number selected from -1 to -3; optionally, n is a real number selected from - 0.5 to -1.5;
   optionally, n is a real number selected from -0.05 to -0.5.

14. The method for constructing a corrosion resistance-mechanical property analysis model for a metallic material according to any one of claims 1 to 13, further comprising the following step: establishing a third set of empirical relational expressions between equivalent corrosion time under the corrosion test condition and service time in the target service environment.

15. The method for constructing a corrosion resistance-mechanical property analysis model for a metallic material according to any one of claims 1 to 14, comprising the following step: establishing a fourth set of empirical relational expressions between a corrosion rate and equivalent corrosion time under the corrosion test condition, wherein the first set of empirical relational expressions comprises the fourth set of empirical relational expressions.

16. A method for analyzing service life of a metallic material, wherein the metallic material is as defined in any one of claims 1 to 3; and
the method for analyzing service life of a metallic material comprises:

determining a mechanical parameter related to a failure behavior of the metallic material based on a target service environment of the metallic material, determining a corrosion test condition capable of simulating the target service environment, and acquiring a test value of a corrosion degree of the metallic material under the corrosion test condition; and
obtaining a service life parameter of the metallic material under the corrosion test condition based on the test value of the corrosion degree, an effective state threshold of the mechanical parameter, and a corrosion resistance-mechanical property analysis model for the metallic material; wherein the corrosion resistance-mechanical property analysis model for the metallic material comprises at least the following relational expressions: a first set of empirical relational expressions describing variation of a corrosion parameter characterizing the corrosion degree with equivalent corrosion time, and a second set of empirical relational expressions describing variation of the mechanical parameter with equivalent corrosion time.

17. The method for analyzing service life of a metallic material according to claim 16, wherein the service life parameter comprises at least the equivalent corrosion time.

18. The method for analyzing service life of a metallic material according to claim 16 or 17, wherein the obtaining a service life parameter of the metallic material under the corrosion test condition based on the test value of the corrosion degree, an effective state threshold of the mechanical parameter, and a corrosion resistance-mechanical property analysis model for the metallic material comprises:
obtaining remaining service time of the metallic material based on the test value of the corrosion degree, the effective state threshold of the mechanical parameter, the corrosion resistance-mechanical property analysis model for the metallic material, and a third set of empirical relational expressions between the equivalent corrosion time under the corrosion test condition and the service time in the target service environment.

19. The method for analyzing service life of a metallic material according to any one of claims 16 to 18, wherein the mechanical parameter comprise at least two types, and the mechanical parameters are sorted in descending order by degrees of response to failure of the metallic material, and the corrosion resistance-mechanical property analysis model for the metallic material is obtained based on the mechanical parameter ranked first.

20. The method for analyzing service life of a metallic material according to any one of claims 16 to 19, wherein the corrosion resistance-mechanical property analysis model for a metallic material is constructed according to the method for constructing a corrosion resistance-mechanical property analysis model for a metallic material according to any one of claims 1 to 15.

21. An apparatus for analyzing service life of a metallic material, wherein the metallic material is as defined in any one of claims 1 to 3; and
the apparatus for analyzing service life of a metallic material comprises:

a corrosion performance data acquisition module configured to determine a mechanical parameter related to a failure behavior of the metallic material based on a target service environment of the metallic material, determine a corrosion test condition capable of simulating the target service environment, and acquire a test value of a corrosion degree of the metallic material under the corrosion test condition; and
a corrosion performance data processing module configured to obtain a service life parameter of the metallic material under the corrosion test condition based on the test value of the corrosion degree, an effective state threshold of the mechanical parameter, and a corrosion resistance-mechanical property analysis model for the metallic material; wherein the corrosion resistance-mechanical property analysis model for the metallic material is as defined in any one of claims 1 to 20.

22. A method for analyzing a mechanical property of a metallic material, wherein the metallic material is as defined in any one of claims 1 to 3; and
the method for analyzing a mechanical property of a metallic material comprises the following steps:

determining a mechanical parameter related to a failure behavior of the metallic material based on a target service environment of the metallic material, determining a corrosion test condition capable of simulating the target service environment, and acquiring a test value of a corrosion degree of the metallic material under the corrosion test condition;
obtaining, based on the test value of the corrosion degree, a target service time of the metallic material, a third set of empirical relational expressions between equivalent corrosion time under the corrosion test condition and service time in the target service environment, and a corrosion resistance-mechanical property analysis model for the metallic material, a preliminary predicted value of the mechanical parameter of the metallic material having been used for the target service time under the corrosion test condition; wherein the corrosion resistance-mechanical property analysis model for the metallic material is as defined in any one of claims 1 to 20; and
comparing the preliminary predicted value of the mechanical parameter with an effective state threshold of the mechanical parameter to obtain a predicted result of the mechanical parameter of the metallic material having been used for the target service time under the corrosion test condition; wherein under a condition that the preliminary predicted value of the mechanical parameter is greater than or equal to the effective state threshold of the mechanical parameter, the preliminary predicted value is output as an effective predicted value of the mechanical parameter of the metallic material having been used for the target service time under the corrosion test condition; or under a condition that the preliminary predicted value of the mechanical parameter is less than the effective state threshold of the mechanical parameter, a prediction result indicating that the metallic material fails before the target service time is reached is output.

23. An apparatus for analyzing a mechanical property of a metallic material, wherein the metallic material is as defined in any one of claims 1 to 3; and
the apparatus for analyzing a mechanical property of a metallic material comprises:

a test data acquisition module configured to determine a mechanical parameter related to a failure behavior of the metallic material based on a target service environment of the metallic material, determine a corrosion test condition capable of simulating the target service environment, and acquire a test value of a corrosion degree of the metallic material under the corrosion test condition;
a test data processing module configured to obtain, based on the test value of the corrosion degree, a target service time of the metallic material, a third set of empirical relational expressions between equivalent corrosion time under the corrosion test condition and service time in the target service environment, and a corrosion resistance-mechanical property analysis model for the metallic material, a preliminary predicted value of the mechanical parameter of the metallic material having been used for the target service time under the corrosion test condition; wherein the corrosion resistance-mechanical property analysis model for the metallic material is as defined in any one of claims 1 to 20; and
a mechanical property classification and identification module configured to compare the preliminary predicted value of the mechanical parameter with an effective state threshold of the mechanical parameter to obtain a predicted result of the mechanical parameter of the metallic material having been used for the target service time under the corrosion test condition; wherein under a condition that the preliminary predicted value of the mechanical parameter is greater than or equal to the effective state threshold of the mechanical parameter, an effective predicted value of the mechanical parameter of the metallic material having been used for the target service time under the corrosion test condition is equal to the preliminary predicted value.

24. A computer device, comprising a memory and a processor, wherein the memory stores a computer program, and when the processor executes the computer program, the steps of the method for constructing a corrosion resistance-mechanical property analysis model for a metallic material according to any one of claims 1 to 15, or the method for analyzing service life of a metallic material according to any one of claims 16 to 20, or the method for analyzing a mechanical property of a metallic material according to claim 22 are implemented.

25. A computer-readable storage medium, storing a computer program, wherein when the computer program is executed by a processor, the steps of the method for constructing a corrosion resistance-mechanical property analysis model for a metallic material according to any one of claims 1 to 15, or the method for analyzing service life of a metallic material according to any one of claims 16 to 20, or the method for analyzing a mechanical property of a metallic material according to claim 22 are implemented.

26. An electric apparatus, comprising:

a battery system comprising a battery casing and a battery cell located inside the battery casing, wherein the battery casing comprises a metallic material as defined in any one of claims 1 to 3; and
at least one of the apparatus for analyzing service life of a metallic material according to claim 21, the apparatus for analyzing a mechanical property of a metallic material according to claim 23, the computer device according to claim 24, and the computer-readable storage medium according to claim 25.

27. The electric apparatus according to claim 26, wherein the battery cell comprises a fuel cell.

28. The electric apparatus according to claim 26, wherein the battery cell comprises a lithium battery cell.

Using a metallic material as a test object, acquire test values of a corrosion parameter at different equivalent corrosion time points under a corrosion test condition to obtain a corrosion performance test experimental dataset, and acquire test values of a mechanical parameter at different corrosion degrees corresponding to the different equivalent corrosion time points to obtain a mechanical property test experimental dataset; where the corrosion test condition is used to simulate a target service environment of the metallic material

S110

Establish, based on the corrosion performance test experimental dataset, a first set of empirical relational expressions describing variation of the corrosion parameter with equivalent corrosion time, and establishing, based on the mechanical property test experimental dataset, a second set of empirical relational expressions describing variation of the mechanical parameter with equivalent corrosion time

S120

FIG. 1

Using a metallic material as a test object, acquire test values of a corrosion parameter at different equivalent corrosion time points under a corrosion test conditions to obtain a corrosion performance test experimental dataset, and acquire test values of a mechanical parameter at different corrosion degrees corresponding to the different equivalent corrosion time points to obtain a mechanical property test experimental dataset; where the corrosion test condition is used to simulate a target service environment of the metallic material; the corrosion parameter includes at least one of a corrosion degree and a corrosion rate; and the mechanical parameter includes at least one of a tensile strength at break and an elongation at break

S110

Establish, based on the corrosion performance test experimental dataset, a first set of empirical relational expressions describing variation of the corrosion parameter with equivalent corrosion time, and establish, based on the mechanical property test experimental dataset, a second set of empirical relational expressions describing variation of the mechanical parameter with equivalent corrosion time

S120

FIG. 2

Using a metallic material as a test object, acquire test values of a corrosion parameter at different equivalent corrosion time points under a corrosion test condition to obtain a corrosion performance test experimental dataset, and acquire test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate

S110

Establish, based on the corrosion performance test experimental dataset, a first set of empirical relational expressions describing variation of the corrosion parameter with equivalent corrosion time, establish, based on the mechanical property test experimental dataset, a second set of empirical relational expressions describing variation of the mechanical parameter with equivalent corrosion time, and establish a third set of empirical relational expressions between equivalent corrosion time under the corrosion test condition and service time in the target service environment

S120

FIG. 3

Using a metallic material as a test object, acquire test values of a corrosion parameter at different equivalent corrosion time points under a corrosion test condition to obtain a corrosion performance test experimental dataset, and acquire test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate

S110

Establish, based on the corrosion performance test experimental dataset, a first set of empirical relational expressions describing variation of the corrosion parameter with equivalent corrosion time, and establish, based on the mechanical property test experimental dataset, a second set of empirical relational expressions describing variation of the mechanical parameter with equivalent corrosion time; where the first set of empirical relational expressions includes a fourth set of empirical relational expressions between the corrosion rate under the corrosion test condition and the equivalent corrosion time

S120

FIG. 4

Using a metallic material as a test object, acquire test values of a corrosion parameter at different equivalent corrosion time points under a corrosion test condition to obtain a corrosion performance test experimental dataset, and acquire test values of a mechanical parameter at different corrosion degrees corresponding to the different equivalent corrosion time points to obtain a mechanical property test experimental dataset; where the corrosion test condition is used to simulate a target service environment of the metallic material; and the corrosion parameter includes at least one of a corrosion degree and a corrosion rate

S110

Establish, based on the corrosion performance test experimental dataset, a first set of empirical relational expressions describing variation of the corrosion parameter with equivalent corrosion time, establish, based on the mechanical property test experimental dataset, a second set of empirical relational expressions describing variation of the mechanical parameter with equivalent corrosion time, and establish a third set of empirical relational expressions between equivalent corrosion time under the corrosion test condition and service time in the target service environment; where the first set of empirical relational expressions includes a fourth set of empirical relational expressions between the corrosion rate under the corrosion test condition and the equivalent corrosion time

S120

FIG. 5

Determine a mechanical parameter related to a failure behavior of a metallic material based on a target service environment of the metallic material, determine a corrosion test condition capable of simulating the target service environment, and acquire a test value of a corrosion degree of the metallic material under the corrosion test condition ⟶ S210

Obtain a service life parameter of the metallic material under the corrosion test condition based on the test value of the corrosion degree, an effective state threshold of the mechanical parameter, and a corrosion resistance-mechanical property analysis model for the metallic material; where the corrosion resistance-mechanical property analysis model for the metallic material includes at least the following relational expressions: a first set of empirical relational expressions describing variation of a corrosion parameter characterizing the corrosion degree with equivalent corrosion time, and a second set of empirical relational expressions describing variation of the mechanical parameter with equivalent corrosion time ⟶ S220

FIG. 6

Apparatus for analyzing service life of a metallic material

Corrosion performance data acquisition module ⟶ 310

Corrosion performance data processing module ⟶ 320

FIG. 7

Determine a mechanical parameter related to a failure behavior of a metallic material based on a target service environment of the metallic material, determine a corrosion test condition capable of simulating the target service environment, and acquire a test value of a corrosion degree of the metallic material under the corrosion test condition — S410

Obtain, based on the test value of the corrosion degree, a target service time of the metallic material, a third set of empirical relational expressions between equivalent corrosion time under the corrosion test condition and service time in the target service environment, and a corrosion resistance-mechanical property analysis model for the metallic material, a preliminary predicted value of the mechanical parameter of the metallic material having been used for the target service time under the corrosion test condition — S420

Compare the preliminary predicted value of the mechanical parameter with an effective state threshold of the mechanical parameter to obtain a predicted result of the mechanical parameter of the metallic material having been used for the target service time under the corrosion test condition; where under a condition that the preliminary predicted value of the mechanical parameter is greater than or equal to the effective state threshold of the mechanical parameter, the preliminary predicted value is output as an effective predicted value of the mechanical parameter of the metallic material having been used for the target service time under the corrosion test condition; or under a condition that the preliminary predicted value of the mechanical parameter is less than the effective state threshold of the mechanical parameter, a prediction result indicating that the metallic material fails before the target service time is reached is output — S430

FIG. 8

Acquire test values of a corrosion degree of a metallic material under a corrosion test condition

Obtain a preliminary predicted value of a mechanical parameter of the metallic material having been used for target service time under the corrosion test condition

Is the preliminary predicted value greater than or equal to an effective state threshold of the mechanical parameter?

No → The metallic material fails before the target service time is reached

Yes

The preliminary predicted value is used as the effective predicted value of the mechanical parameter

FIG. 9

Apparatus for analyzing a mechanical property of a metallic material

Test data acquisition module — 510

Test data processing module — 520

Mechanical property classification and identification module — 520

FIG. 10

FIG. 11

FIG. 12

FIG. 13

FIG. 14

| Fit-1 | $y1=A\cdot x^B$ | Fit-2 | $y1=a+b\cdot x$ |
|---|---|---|---|
| A | 0.14531±0.00477 | Intercept a | -0.12448±0.02864 |
| B | -0.53339±0.01429 | Slope b | 0.00713±0.00123 |
| Reduced Chi-Sqr | 0.45371 | Person's r | 0.95794 |
| $R^2$ (ROD) | 0.97801 | $R^2$ (ROD) | 0.91766 |
| Adjusted $R^2$ | 0.97685 | Adjusted $R^2$ | 0.89021 |

FIG. 15

| Model | Allometric 1 |
|---|---|
| Fit | $y1 = A \cdot x^B$ |
| A | 0.75575±0.01281 |
| B | -0.53176±0.00697 |
| $R^2$ (ROD) | 0.99349 |
| Adjusted $R^2$ | 0.9932 |

FIG. 16

| Fit-1 | y1=A·x$^B$ | Fit-2 | y1=a+b·x | Fit-3 | y1=a+b·x |
|---|---|---|---|---|---|
| A | 0.1259±0.00433 | Intercept a | -0.01322±0.00416 | Intercept a | -0.36659±0.08511 |
| B | -0.68014±0.01739 | Slope b | 0.00334±(2.58734E-4) | Slope b | 0.01974±0.00386 |
| Reduced Chi-Sqr | 0.03087 | Person's r | 0.97401 | Person's r | 0.94706 |
| R$^2$ (ROD) | 0.99215 | R$^2$ (ROD) | 0.9487 | R$^2$ (ROD) | 0.89692 |
| Adjusted R$^2$ | 0.99117 | Adjusted R$^2$ | 0.94299 | Adjusted R$^2$ | 0.86256 |

FIG. 17

| Fit-1 | y1=A·x$^B$ | Fit-2 | y1=a+b·x | Fit-3 | y1=a+b·x |
|---|---|---|---|---|---|
| A | 0.13406±0.00347 | Intercept a | -0.00171±0.00232 | Intercept a | -0.66839±0. 0.02883 |
| B | -0.68838±0.01312 | Slope b | 0.00222±(1.44608 E-4) | Slope b | 0.03111±0.00121 |
| Reduced Chi-Sqr | 0.19457 | Person's r | 0.97936 | Person's r | 0.99924 |
| R$^2$ (ROD) | 0.99549 | R$^2$ (ROD) | 0.95914 | R$^2$ (ROD) | 0.99848 |
| Adjusted R$^2$ | 0.99499 | Adjusted R$^2$ | 0.95505 | Adjusted R$^2$ | 0.99697 |

FIG. 18

FIG. 19

FIG. 20

FIG. 21

FIG. 22

**EP 4 738 372 A1**

FIG. 23

FIG. 24

61

FIG. 25

FIG. 26

FIG. 27

FIG. 28

FIG. 29

FIG. 30

FIG. 31

## INTERNATIONAL SEARCH REPORT

| International application No. |
|---|
| **PCT/CN2024/116845** |

| A. | CLASSIFICATION OF SUBJECT MATTER |
|---|---|

G16C60/00(2019.01)i; G16C20/70(2019.01)i

According to International Patent Classification (IPC) or to both national classification and IPC

| B. | FIELDS SEARCHED |
|---|---|

Minimum documentation searched (classification system followed by classification symbols)

IPC: G16C

Documentation searched other than minimum documentation to the extent that such documents are included in the fields searched

Electronic data base consulted during the international search (name of data base and, where practicable, search terms used)

CNABS; CNTXT; CNKI; WPABS; DWPI; USTXT; WOTXT; EPTXT: 腐蚀, 金属, 测试, 力学, 性能, 速率, 时间, 周期, 关系式, 等式, corrosion, metal, test, mechanics, performance, speed, time, period, equation

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 106896052 A (BEIHANG UNIVERSITY) 27 June 2017 (2017-06-27) description, paragraphs [0054]-[0081] | 1-28 |
| A | CN 116884509 A (BEIHANG UNIVERSITY) 13 October 2023 (2023-10-13) entire document | 1-28 |
| A | CN 113283137 A (NO.59 INSTITUTE OF CHINA ORDNANCE INDUSTRY) 20 August 2021 (2021-08-20) entire document | 1-28 |
| A | CN 116678814 A (FAW JIEFANG AUTOMOTIVE CO., LTD.) 01 September 2023 (2023-09-01) entire document | 1-28 |
| A | CN 116343969 A (CENTRAL RESEARCH INSTITUTE OF BUILDING AND CONSTRUCTION CO., LTD. MCC GROUP) 27 June 2023 (2023-06-27) entire document | 1-28 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **22 November 2024** | **27 November 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** |  |
|  | Telephone No. |

Form PCT/ISA/210 (second sheet) (July 2022)

**INTERNATIONAL SEARCH REPORT**
Information on patent family members

| International application No. |
| --- |
| **PCT/CN2024/116845** |

| Patent document cited in search report | | | Publication date (day/month/year) | Patent family member(s) | | | Publication date (day/month/year) |
| --- | --- | --- | --- | --- | --- | --- | --- |
| CN | 106896052 | A | 27 June 2017 | CN | 106896052 | B | 16 August 2019 |
| CN | 116884509 | A | 13 October 2023 | None | | | |
| CN | 113283137 | A | 20 August 2021 | CN | 113283137 | B | 03 March 2023 |
| CN | 116678814 | A | 01 September 2023 | None | | | |
| CN | 116343969 | A | 27 June 2023 | None | | | |

Form PCT/ISA/210 (patent family annex) (July 2022)

**REFERENCES CITED IN THE DESCRIPTION**

**Patent documents cited in the description**

- CN 2023116080448